# EUROPEAN PATENT APPLICATION

(11) **EP 4 647 042 A2**
(43) Date of publication of application: **12.11.2025**
(21) Application number: 25205346.7
(22) Date of filing: 19.03.2021
(51) Int. Cl.: A61F 2/24

(54) **DELIVERY SYSTEM CONFIGURATIONS**

(30) Priority: 24.03.2020 US 202062993885 P
(62) Divisional of application: 21718385.4
(71) Applicant: Edwards Lifesciences Corporation, Irvine, CA 92614 (US)
(72) Inventor: OKABE, Hiroshi, Irvine, CA, 92614 (US); LUONG, Hieu Minh, Westminster, CA, 92683 (US); SANCHEZ, Julio Cesar, Irvine, CA, 92614 (US); EDWARDS, Jesse Robert, Irvine, CA, 92614 (US); COOPER, Alexander H., Irvine, CA, 92614 (US); LANDON, David Robert, Irvine, CA, 92614 (US)
(74) Representative: Venner, Julia Ann

(57) **Abstract**

Disclosed are embodiments of delivery systems for delivery of replacement heart valves. This can include mitral, aortic, tricuspid, and pulmonary valves. The delivery systems can include one or more different components and configurations that advantageously improve placement of the replacement heart valves during the operation of the delivery system.

## Description

### RELATED APPLICATIONS

This application claims priority benefit to US Provisional Application No. 62/993,885 filed March 24, 2020, the entire content of which is hereby incorporated by reference herein.

### FIELD

Certain embodiments disclosed herein relate generally to prostheses for implantation within a lumen or body cavity and delivery systems for a prosthesis. In particular, the prostheses and delivery systems relate in some embodiments to replacement heart valves, such as replacement mitral heart valves or replacement tricuspid heart valves.

### BACKGROUND

Human heart valves, which include the aortic, pulmonary, mitral and tricuspid valves, function essentially as one-way valves operating in synchronization with the pumping heart. The valves allow blood to flow downstream, but block blood from flowing upstream. Diseased heart valves exhibit impairments such as narrowing of the valve or regurgitation, which inhibit the valves' ability to control blood flow. Such impairments reduce the heart's blood-pumping efficiency and can be a debilitating and life-threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open heart surgery. Replacement valves are being designed to be delivered through minimally invasive procedures and even percutaneous procedures. Such replacement valves often include a tissue-based valve body that is connected to an expandable frame that is then delivered to the native valve's annulus.

Development of prostheses including but not limited to replacement heart valves that can be compacted for delivery and then controllably expanded for controlled placement has proven to be particularly challenging. An additional challenge relates to the ability of such prostheses to be secured relative to intralumenal tissue, e.g., tissue within any body lumen or cavity, in an atraumatic manner.

Delivering a prosthesis to a desired location in the human body, for example delivering a replacement heart valve to the mitral valve, can also be challenging. Obtaining access to perform procedures in the heart or in other anatomical locations may require delivery of devices percutaneously through tortuous vasculature or through open or semi-open surgical procedures. The ability to control the deployment of the prosthesis at the desired location can also be challenging.

### SUMMARY

Embodiments of the present disclosure are directed to a delivery system, such as but not limited to a delivery system for a replacement heart valve. Further embodiments are directed to methods of use to deliver and/or controllably deploy a prosthesis, such as but not limited to a replacement heart valve, to a desired location within the body. In some embodiments, a replacement heart valve and methods for delivering a replacement heart valve to a native heart valve, such as a mitral valve, an aortic valve, or a tricuspid valve, are provided.

In some embodiments, a delivery system and method are provided for delivering a replacement heart valve to a native mitral valve location. The delivery system and method may utilize a transseptal approach. In some embodiments, components of the delivery system facilitate bending of the delivery system to steer a prosthesis from the septum to a location within the native mitral valve. In some embodiments, a capsule is provided for containing the prosthesis for delivery to the native mitral valve location. In other embodiments, the delivery system and method may be adapted for delivery of implants to locations other than the native mitral valve.

The present disclosure includes, but is not limited to, the following embodiments.

Embodiment 1: A delivery system for a replacement heart valve, the delivery system comprising:
an inner shaft having a proximal end and a distal end,
wherein the distal end of the inner shaft comprises a manifold,
wherein the manifold comprises a plurality of circumferentially-spaced apart apertures, each of the apertures having a tooth extending from a distal edge of the aperture toward a proximal edge of the aperture;
an outer shaft member configured to cover the apertures of the manifold when in a distal position to prevent release of the looped end of the attachment tether.
wherein the outer shaft member comprises at least one aperture configured to be aligned with the plurality of circumferentially-spaced apart apertures of the manifold;
at least one attachment tether configured to releasably connect to at least one eyelet of the replacement heart valve,
wherein a first looped end of the at least one attachment tether is configured to be attached to the distal end of the inner shaft,
wherein a second looped end of the at least one attachment tether is configured to extend through the at least one eyelet of the replacement heart valve, through the at least one aperture of the outer sleeve into a respective one of the apertures of the manifold, and then looped over a free proximal end of the tooth,
wherein at least one of the outer shaft member and the inner manifold is configured to translate axially relative to the other to uncover the second looped end of the at least one attachment tether such that the second looped end can be removed from the tooth, thereby facilitating release of the replacement heart valve from the delivery system.

Embodiment 2: The delivery system of Embodiment 1, wherein the at least one aperture of the outer shaft member comprises a plurality of circumferentially-spaced apart apertures.

Embodiment 3: The delivery system of Embodiment 1 or 2, wherein the outer shaft member is configured to be moved proximally relative to the inner shaft to uncover the second looped end of the at least one attachment tether.

Embodiment 4: The delivery system of Embodiment 1 or 2, wherein the inner shaft is configured to be moved distally relative to the outer shaft member to uncover the second looped end of the at least one attachment tether.

Embodiment 5: The delivery system of any of Embodiments 1-4, wherein the outer shaft member comprises a sleeve.

Embodiment 6: The delivery system of any of Embodiments 1-5, wherein the at least one attachment member comprises a plurality of attachment tethers, wherein a first looped end of each of the plurality of attachment tethers is configured to be attached to the distal end of the inner shaft, and wherein a second looped end of each of the plurality of attachment tethers is configured to configured to extend through the at least one eyelet of the replacement heart valve, through the at least one aperture of the outer sleeve into a respective one of the apertures of the manifold, and then looped over a free proximal end of the tooth of the respective one of the apertures of the manifold.

Embodiment 7: A method of facilitating delivery of a replacement heart valve within a body of a patient, the method comprising:
advancing a distal portion of a delivery system to a desired implantation location within a heart of the patient,
wherein the delivery system comprises an inner shaft and an outer shaft member,
wherein the distal end of the inner shaft comprises at least one radial aperture, the at least one radial aperture having a tooth extending from a distal edge of the at least one radial aperture toward a proximal edge of the at least one radial aperture,
wherein the outer shaft member comprises at least one radial aperture configured to be aligned with the at least one radial aperture of the inner shaft,
wherein a first looped end of an attachment tether is attached to a distal end of the inner shaft,
wherein a second looped end of the attachment tether is removably coupled to the tooth of the at least one radial aperture of the inner shaft after having been inserted through an eyelet of the replacement heart valve;
causing the outer shaft member and the inner shaft to transition from a locked configuration in which the second looped end of the attachment member cannot be removed from the tooth to an unlocked configuration in which the second looped end of the attachment member can be removed from the tooth, thereby decoupling the replacement heart valve from the delivery system and allowing the replacement heart valve to remain in the desired implantation location; and
removing the delivery system from the patient.

Embodiment 8: The method of Embodiment 7, wherein the desired implantation location is a native mitral valve.

Embodiment 9: The method of Embodiment 7. wherein the desired implantation location is a native tricuspid valve.

Embodiment 10: The method of any of Embodiments 7-9, wherein causing the outer shaft member and the inner shaft to transition from the locked configuration to the unlocked configuration comprises moving the inner shaft in a distal direction.

Embodiment 11: The method of any of embodiments 7-9, wherein causing the outer shaft member and the inner shaft to transition from the locked configuration to the unlocked configuration comprises moving the outer shaft member in a proximal direction.

Embodiment 12: A delivery system for a replacement heart valve, the delivery system comprising a shaft having a proximal end and a distal end, a manifold on a distal end of the shaft, wherein the manifold comprises a plurality of radially extending apertures, each aperture having a tooth, at least one attachment tether configured to releasably connect to the replacement heart valve, wherein looped portions of the at least one attachment tether extend through the radially extending apertures of the manifold to surround the tooth, and a sleeve configured to cover the radially extending apertures in a distal position to prevent release of the looped portions, wherein the sleeve is configured to be proximally translated to a proximal position to uncover the looped portions so that the replacement heart valve is released from the at least one attachment tether.

Embodiment 13: A delivery system for a replacement heart valve, the delivery system comprising:
a shaft having a proximal end and a distal end;
a manifold on a distal end of the shaft, wherein the manifold comprises a plurality of radially extending apertures;
at least one attachment tether configured to releasably connect to the replacement heart valve, wherein looped portions of the at least one attachment tether extend through the radially extending apertures of the manifold; and
a release tether configured to extend through the looped portions of the at least one attachment tether;
wherein when the release tether is withdrawn from the looped portions, the replacement heart valve is released from the at least one attachment tether.

Embodiment 14: The delivery system of Embodiment 13, wherein the manifold comprises an inner manifold and an outer manifold.

Embodiment 15: The delivery system of Embodiment 14, wherein the outer manifold comprises the radially extending apertures for receiving the looped portions of a distal end of the at least one attachment tether.

Embodiment 16: The delivery system of Embodiment 14 or 15, wherein a proximal end of the at least one attachment tether is attached to the inner manifold.

Embodiment 17: The delivery system of any one of Embodiments 13-15, wherein the at least one attachment tether comprises one and only one attachment tether.

Embodiment 18: The delivery system of any one of Embodiments 13-15, wherein the at least one attachment tether comprises a plurality of attachment tethers.

Embodiment 19: A handle for a replacement heart valve delivery system, the handle comprising, a housing, at least one knob located on the housing, at least one ring gear in communication with the at least one knob, wherein the at least one knob is configured to rotate the at least one ring gear, at least one planet gear located within the at least one ring gear and in communication with the at least one ring gear, wherein the at least one planet gear remains in the same circumferential position with relation to the at least one ring gear during rotation of the at least one ring gear, and a linear travel screw in communication with the at least one planet gear, wherein the linear travel screw is configured to move in an axial direction upon rotation of the at least one knob.

Embodiment 20: The handle of Embodiment 19, wherein the at least one ring gear comprises a plurality of planet gears, each planet gear of the plurality of planet gears in communication with one of a plurality of linear travel screws.

Embodiment 21: A delivery system for use with a replacement heart valve, the system comprising a bendable nose cone shaft having a proximal end and a distal end, a nose cone attached to a distal end of the nose cone shaft; and a rigid shaft at least partially covering the bendable nose cone shaft, wherein the rigid shaft is configured to axially translate with respect to the bendable nose cone shaft to cover or uncover the bendable nose cone shaft, wherein, when the bendable nose cone shaft is uncovered by the rigid shaft, the bendable nose cone shaft is configured to allow the replacement heart valve attached to the system to conform to a native anatomy of a native heart valve.

Embodiment 22: The delivery system of Embodiment 21, wherein the bendable nose cone shaft comprises a bendable polymer.

Embodiment 23: A delivery system for use with a replacement heart valve, the system comprising an inner retention member configured to releasably retain a replacement heart valve, and an outer retention member configured to at least partially cover a portion of the replacement heart valve and the inner retention member, wherein the outer retention member and the inner retention member are configured to have a locked and an unlocked configuration, wherein when in the unlocked configuration the outer retention member is configured to move axially with respect to the inner retention member, and when in the locked configuration the outer retention member is prevented from moving axially with respect to the inner retention member.

Embodiment 24: The delivery system of Embodiment 23, wherein the inner retention member comprises an outer threading and the outer retention member comprises an inner threading, and wherein the locked position occurs when the outer threading is threaded onto the inner threading.

Embodiment 25: The delivery system of Embodiment 23 or 24, wherein the inner retention member and outer retention member comprise locking features that an operator can unlock when the operator wants to move the outer retention member axially with respect to the inner retention member.

Embodiment 26: A delivery system for use with a replacement heart valve, the system comprising an inner shaft having a proximal end and a distal end, the inner shaft having a cut pattern to allow bending of the inner shaft, and a spine, an outer shaft surrounding the inner shaft and having a proximal end and a distal end, the outer shaft having a cut pattern to allow bending of the outer shaft, and a spine, and at least one pull wire configured to bend one or more of the inner shaft and the outer shaft, wherein one of the inner shaft and the outer shaft are configured to rotate with respect to one another between a flexing configuration and an unflexing configuration, wherein, in the flexing configuration, the cut pattern of the inner shaft is aligned with the cut pattern of the outer shaft, and wherein, in the unflexing configuration, the spine of the inner shaft is aligned with the cut pattern of the outer shaft.

Embodiment 27: The delivery system of Embodiment 26, wherein the cut pattern of the inner shaft is the same as the cut pattern of the outer shaft.

Embodiment 28: The delivery system of Embodiment 26, wherein the cut pattern of the inner shaft is different than the cut pattern of the outer shaft.

Embodiment 29: The delivery system of any of Embodiments 26-28, wherein the at least one pull wire comprises a plurality of pull wires, wherein a first pull wire is configured to cause bending of the inner shaft and wherein a second pull wire is configured to cause bending of the outer shaft.

Embodiment 30: A method of facilitating controlled bending of bendable coaxial shafts of a delivery system, the method comprising:
providing a delivery system comprising:
   an inner shaft having a proximal end and a distal end, the inner shaft having a cut pattern to allow bending of the inner shaft, and a spine;
   an outer shaft surrounding the inner shaft and having a proximal end and a distal end, the outer shaft having a cut pattern to allow bending of the outer shaft, and a spine; and
   at least one pull wire configured to bend one or more of the inner shaft and the outer shaft;
rotating one of the inner shaft and the outer shaft with respect to the other between a flexing configuration and an unflexing configuration;
wherein, in the flexing configuration, the cut pattern of the inner shaft is aligned with the cut pattern of the outer shaft to allow bending of the outer shaft upon actuation of the at least one pull wire; and
wherein, in the unflexing configuration, the spine of the inner shaft is aligned with the cut pattern of the outer shaft to prevent bending of the outer shaft upon actuation of the at least one pull wire.

Embodiment 31: The method of Embodiment 30, wherein the cut pattern of the inner shaft is the same as the cut pattern of the outer shaft.

Embodiment 32: The method of Embodiment 31, wherein the cut pattern of the inner shaft is different than the cut pattern of the outer shaft.

Embodiment 33: The method of any of Embodiments 30-32, wherein the at least one pull wire comprises a plurality of pull wires, wherein a first pull wire is configured to cause bending of the inner shaft and wherein a second pull wire is configured to cause bending of the outer shaft.

Embodiment 34: A delivery system for use with a replacement heart valve, the system comprising an inner shaft having a proximal end and a distal end, and an outer shaft surrounding the inner shaft and having a proximal end and a distal end, wherein the inner shaft and the outer shaft are keyed together at the distal end of the outer shaft and the distal end of the inner shaft to prevent rotation of the inner shaft with respect to the outer shaft.

Embodiment 35: The delivery system of Embodiment 34, wherein the inner shaft and the outer shaft each have an ovaloid cross-section.

Embodiment 36: The delivery system of Embodiment 34 or 35, wherein at least the distal end of the inner shaft comprises a locking tab and at least the distal end of the outer shaft comprises a notch or slot configured to receive the locking tab so as to prevent rotation of the inner shaft with respect to the outer shaft.

Embodiment 37: The delivery system of Embodiment 34 or 35, wherein at least the distal end of the outer shaft comprises a locking tab and at least the distal end of the inner shaft comprises a notch or slot configured to receive the locking tab so as to prevent rotation of the inner shaft with respect to the outer shaft.

Embodiment 38: The delivery system of any of Embodiment 34-37, wherein the outer shaft comprises an outer pull wire lumen and wherein the inner shaft comprises an inner pull wire lumen.

Embodiment 39: A delivery system for a replacement heart valve, the delivery system comprising an inner shaft having a proximal end and a distal end, a mid shaft surrounding the inner shaft and having a proximal end and a distal end and a lumen, the mid shaft having a disc on the distal end, the diameter of the disc being greater than a diameter of the inner shaft, wherein the disc comprises a longitudinally extending aperture radially outward of the lumen, and an outer shaft surrounding the mid shaft, the outer shaft comprising a radially extending aperture having a tooth, and an attachment tether configured to releasably connect to the replacement heart valve, wherein the attachment tether has a first end connected to the inner shaft, wherein the attachment tether extends through the lumen of the mid shaft and out the distal end of the mid shaft, and wherein the attachment tether extends proximally through the longitudinally extending aperture and attaches to the tooth of the outer shaft, wherein, when the disc is proximally translated it prevents release of the attachment tether from the tooth, and wherein, when the disc is distally translated it releases the tether from the tooth so that the replacement heart valve is released.

Embodiment 40: A delivery system for a replacement heart valve, the delivery system comprising a capsule configured to surround the replacement heart valve and configured to radially compress the replacement heart valve, wherein the capsule comprises a distal portion, wherein the distal portion comprises a hypotube with an interrupted spiral cut pattern, an outer polymer jacket configured to at least partially cover a radially outwards surface of the hypotube, a fluoropolymer liner on a radially inner surface of the hypotube, and a porous fluoropolymer outer coating on one of the hypotube or the outer polymer jacket, and a proximal portion having a smaller diameter than the distal portion, wherein the proximal portion comprises a hypotube with a cut pattern, the hypotube surrounded by an outer polymer jacket.

Embodiment 41: The delivery system of Embodiment 40, wherein the outer polymer jacket of the distal portion covers greater than 90% of a length of the hypotube of the distal portion.

Embodiment 42: The delivery system of Embodiment 40 or 41, wherein the hypotube of the distal portion is configured to provide compression resistance and the liner and/or outer polymer jacket of the distal portion is configured to provide tension resistance.

Embodiment 43: The delivery system of any of Embodiments 40-42, wherein the liner of the distal portion is porous.

Embodiment 44: The delivery system of any of Embodiments 40-43, wherein the liner of the distal portion is bonded to a metal structure of the hypotube of the distal portion using a reflow process.

Embodiment 45: A handle for a replacement heart valve delivery system, the handle comprising:
a housing comprising a threaded portion;
at least one adapter; and
at least one knob located on the housing, the at least one knob comprising:
   a first portion configured to be coupled to the at least one adapter, and
   a second portion configured to be detachably coupled to the first portion, wherein the first and second portions are configured to detach when a threshold force is exerted on the at least one adapter.

Embodiment 46: The handle of Embodiment 45, wherein the first portion comprises a recess and an internal spring within the recess.

Embodiment 47: The handle of Embodiment 46, wherein the second portion comprises a projection configured to be received by the recess of the first portion.

Embodiment 48: The handle of Embodiment 47, wherein the internal spring of the first portion releasably retains the projection of the second portion when the first and second portions are coupled.

Embodiment 49: The handle of Embodiment 45, wherein the first portion comprises a hole and the second portion comprises a spring tab, the hole of the first portion being configured to receive the spring tab of the second portion when the first and second portions are coupled.

Embodiment 50: The handle of Embodiment 49, wherein the spring tab of the second portion is configured to deflect from the hole of the first portion when the threshold force is exerted on the at least one adapter to decouple the first and second portions.

Embodiment 51: The handle of Embodiment 45, wherein the first portion comprises a ramped recess and the second portion comprises a ramped projection, the ramped recess of the first portion being configured to receive the ramped projection of the second portion when the first and second portions are coupled.

Embodiment 52: The handle of Embodiment 51, wherein the ramped projection of the second portion is configured to deflect from the ramped recess of the first portion when the threshold force is exerted on the at least one adapter to decouple the first and second portions.

Embodiment 53: The handle of Embodiment 45, wherein the first portion comprises a recess and the second portion comprises a pin, the recess of the first portion being configured to receive the pin of the second portion when the first and second portions are coupled.

Embodiment 54: The handle of Embodiment 53, wherein the pin of the second portion is configured to break from the second portion when the threshold force is exerted on the at least one adapter to decouple the first and second portions.

Embodiment 55: The handle of Embodiment 45, wherein the first portion comprises a recess and the second portion comprises a spring plunger, the recess of the first portion being configured to receive a portion of the spring plunger of the second portion when the first and second portions are coupled.

Embodiment 56: The handle of Embodiment 55, wherein the portion of the spring plunger of the second portion is configured to decouple from the ramped recess of the first portion when the threshold force is exerted on the at least one adapter to decouple the first and second portions.

Embodiment 57: The handle of any of Embodiments 45-56, wherein the second portion is configured to engage with the threaded portion.

Embodiment 58: A handle for a replacement heart valve delivery system, the handle comprising:
a housing comprising a threaded portion;
at least one adapter; and
at least one knob located on the housing, the at least one knob comprising:
   a first portion configured to be coupled to the at least one adapter, and
   a second portion coupled to the first portion, wherein a distance between the first portion and the second portion is correlated with a force exerted on the at least one adapter.

Embodiment 59: The handle of Embodiment 58, wherein the at least one knob further comprises a connector configured to extend between and couple the first and second portions, the connector comprising a distal portion and a proximal portion.

Embodiment 60: The handle of Embodiment 59, wherein the first portion is configured to receive the distal portion of the connector and the second portion is configured to receive the proximal portion of the connector.

Embodiment 61: The handle of Embodiment 59 or 60, wherein the connector comprises one or more indicators.

Embodiment 62: The handle of Embodiment 61, wherein the one or more indicators indicate the force exerted on the adapter.

Embodiment 63: The handle of any of Embodiments 58-62, wherein the distance between the first and second portions increases as the force exerted on the at least one adapter increases.

Embodiment 64: The handle of any of Embodiments 58-63, wherein the distance between the first and second portions decreases as the force exerted on the at least one adapter decreases.

Embodiment 65: The handle of any of Embodiments 58-64, wherein the first and second portions are configured to detach when the force exerted on the adapter reaches a threshold force.

Embodiment 66: The handle of any of Embodiments 58-65, wherein the second portion is configured to engage with the threaded portion.

### BRIEF DESCRIPTION OF THE DRAWINGS

Figure **1** shows an embodiment of a delivery system.
Figure 2A shows a partial cross-sectional view of the distal end of the delivery system of Figure 1 loaded with the valve prosthesis of Figure 3A.
Figure 2B shows a partial cross-sectional view of the distal end of the delivery system of **Figure 1** without the valve prosthesis of Figure 3A.
Figure 2C shows a partial cross-sectional view of the distal end of the delivery system of **Figure 1** without with certain shaft assemblies translated along the rail assembly.
**Figure** 3A shows a side view of an embodiment of a valve prosthesis that may be delivered using the delivery systems described herein.
**Figure 3B** shows a side view of an embodiment of an aortic valve prosthesis that may be delivered using the delivery systems described herein.
**Figure 4** shows a perspective view of the distal end of the delivery system of **Figure 1****.**
**Figure 5** show components of the delivery system of **Figure 4** with the outer sheath assembly moved proximally and out of view.
**Figure 6A** show components of the delivery system of **Figure 5** with the mid shaft assembly moved proximally and out of view.
**Figure 6B** illustrates a cross-section of the rail assembly.
**Figure 7** show components of the delivery system of **Figure 6A** with the rail assembly moved proximally and out of view.
**Figure 8** show components of the delivery system of **Figure 7** with the inner assembly moved proximally and out of view.
**Figures 9A** and **9B** illustrate embodiments of a guide wire shield.
**Figure 10** illustrates an embodiment of an outer hypotube.
**Figure 11** illustrates an embodiment of a mid shaft hypotube.
**Figure 12A** illustrates an embodiment of the mid shaft hypotube of **Figure 11** as a flat pattern.
**Figure 12B** illustrates an embodiment of an outer retention ring.
**Figure 13** illustrates an embodiment of a rail assembly.
**Figure 14** illustrates an embodiment of an inner assembly.
**Figure 15** illustrates a cross-section of a capsule.
**Figure 16** illustrates an embodiment of a delivery system handle.
**Figure 17** illustrates a cross-section of the delivery system handle of **Figure 16****.**
**Figure 18** illustrates a schematic representation of a transseptal delivery approach.
**Figure 19** illustrates a schematic representation of a valve prosthesis positioned within a native mitral valve.
**Figure 20** shows the valve prosthesis frame located within a heart.
**Figures 21-23** show steps of a method for delivery of the valve prosthesis to an anatomical location.
**Figures 24A-B** illustrate the methodology of the rail delivery system.
**Figures 25A-25B** illustrate embodiments of a planetary gear design.
**Figures 26A-26B** illustrates embodiments of a flexible shaft and stiffened sheath.
**Figure 27** illustrates an embodiment of threaded components.
**Figures 28A-28C** illustrate bendable aligned shafts.
**Figures 29A-29C** illustrate embodiments of disclosed rotatable shafts.
**Figures 30, 31****,** **31A** and **31B** illustrate unkeyed **(****Figure 30****)** and keyed **(****Figures 31****,** **31A and 31B****)** shaft configurations.
**Figures 32A-36** illustrate embodiments of tethering release mechanisms.
**Figures 37-39B** illustrate an embodiment of a capsule construction.
**Figure 40** illustrates another embodiment of a delivery system handle with an embodiment of a failsafe knob.
**Figure 41A** illustrates a cross-section of the failsafe knob of **Figure 40** in a first configuration.
**Figures 41B** illustrates a cross-section of the failsafe knob of **Figure 40** in a second configuration.
**Figures 41C-41F** illustrate schematic illustrations of example connection mechanisms.
**Figure 42A** illustrates a cross-section of an embodiment of an indicator knob in a first configuration.
**Figures 42B** illustrates a cross-section of the indicator knob of **Figure 42A** in a second configuration.

### DETAILED DESCRIPTION

The present specification and drawings provide aspects and features of the disclosure in the context of several embodiments of replacement heart valves, delivery systems and methods that are configured for use in the vasculature of a patient, such as for replacement of natural heart valves in a patient. These embodiments may be discussed in connection with replacing specific valves such as the patient's aortic, tricuspid, or mitral valve. However, it is to be understood that the features and concepts discussed herein can be applied to products other than heart valve implants. For example, the controlled positioning, deployment, and securing features described herein can be applied to medical implants, for example other types of expandable prostheses, for use elsewhere in the body, such as within an artery, a vein, or other body cavities or locations. In addition, particular features of a valve, delivery system, etc. should not be taken as limiting, and features of any one embodiment discussed herein can be combined with features of other embodiments as desired and when appropriate. While certain of the embodiments described herein are described in connection with a transfemoral delivery approach, it should be understood that these embodiments can be used for other delivery approaches such as, for example, transapical or transjugular approaches. Moreover, it should be understood that certain of the features described in connection with some embodiments can be incorporated with other embodiments, including those which are described in connection with different delivery approaches.

### Delivery System

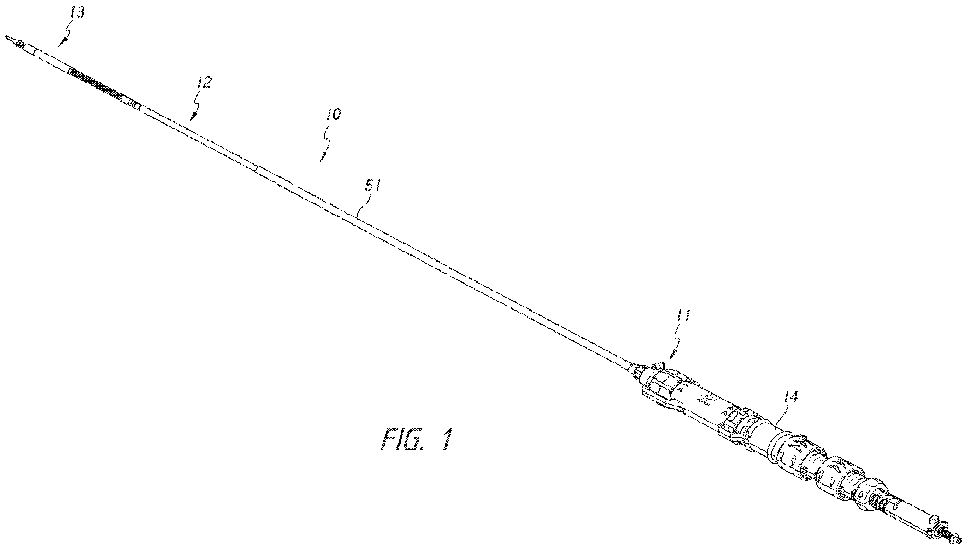

**Figure 1** illustrates an embodiment of a delivery device, system, or assembly **10.** The delivery system **10** can be used to deploy a prosthesis, such as a replacement heart valve, within the body. In some embodiments, the delivery system **10** can use a dual plane deflection approach to properly delivery the prosthesis. Replacement heart valves can be delivered to a patient's heart mitral or tricuspid valve annulus or other heart valve location in various manners, such as by open surgery, minimally-invasive surgery, and percutaneous or transcatheter delivery through the patient's vasculature. Example transfemoral approaches may be found in U.S. Pat. Pub. No. 2015/0238315, filed February 20, 2015, the entirety of which is hereby incorporated by reference in its entirety. While the delivery system **10** is described in connection with a percutaneous delivery approach, and more specifically a transfemoral delivery approach, it should be understood that features of delivery system **10** can be applied to other delivery system, including delivery systems for a transapical delivery approach.

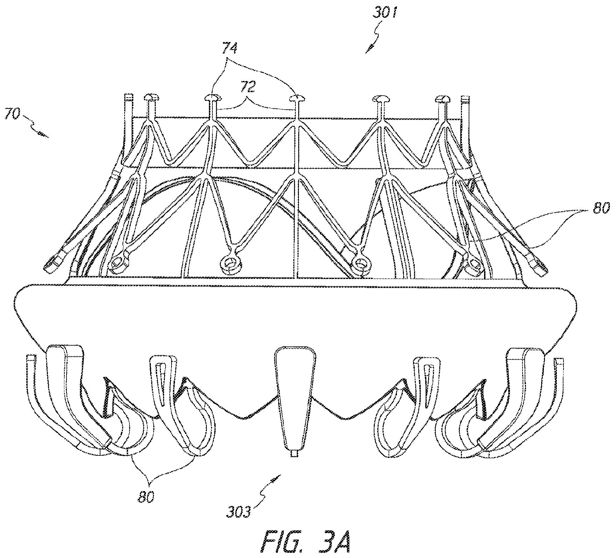

The delivery system **10** can be used to deploy a prosthesis, such as a replacement heart valve as described elsewhere in this specification, within the body. The delivery system **10** can receive and/or cover portions of the prosthesis such as a first end **301** and second end **303** of the prosthesis **70** illustrated in **Figure 3A** below. For example, the delivery system **10** may be used to deliver an expandable implant or prosthesis **70,** where the prosthesis **70** includes the first end **301** and the second end **303,** and wherein the second **303** end is configured to be deployed or expanded before the first end **301.**

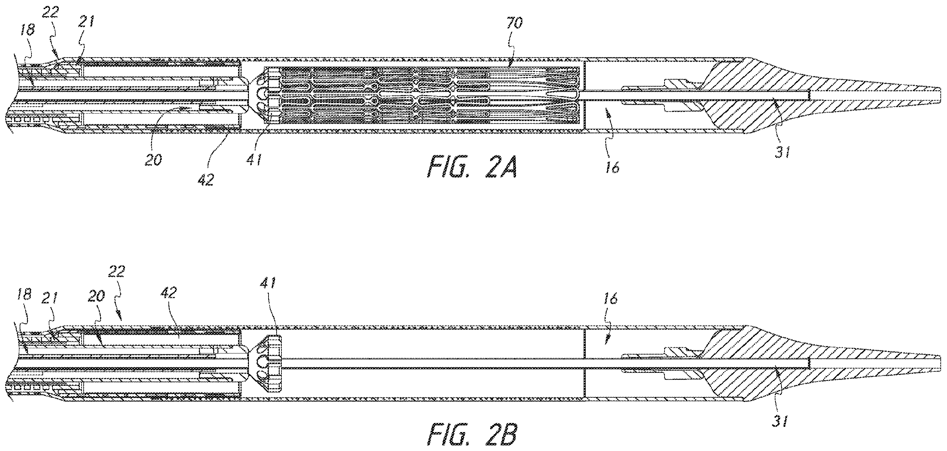

**Figure 2A** further shows an example of the prosthesis **70** that can be inserted into the delivery system **10,** specifically into the implant retention area **16.** For ease of understanding, in **Figure 2A****,** the prosthesis is shown with only the bare metal frame illustrated. The implant or prosthesis **70** can take any number of different forms. A particular example of frame for a prosthesis is shown in **Figure 3A****,** though it will be understood that other designs can also be used. The prosthesis **70** can include one or more sets of anchors, such as distal (or ventricular) anchors **80** extending proximally when the prosthesis frame is in an expanded configuration and proximal (or atrial) anchors **82** extending distally when the prosthesis frame is in an expanded configuration. The prosthesis can further include struts **72** which may end in mushroom-shaped tabs **74** at the first end **301.** Further discussion can be found in U.S. Publication No. 2015/0328000A1, published November 19, 2015, hereby incorporated by reference in its entirety.

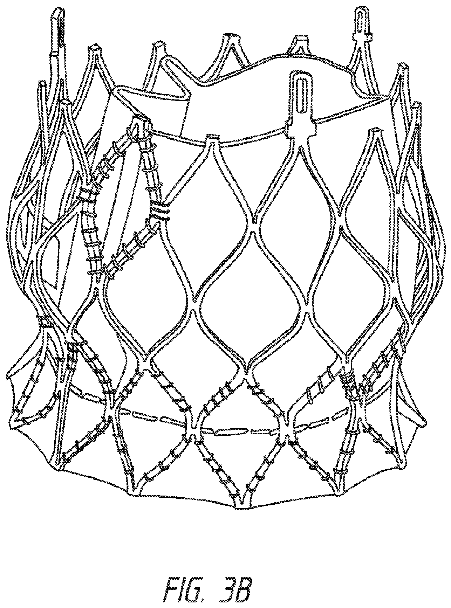

In some embodiments, the delivery system **10** can be used in conjunction with a replacement aortic valve, such as shown in **Figure 3B****.** In some embodiments the delivery system **10** can be modified to support and delivery the replacement aortic valve. However, the procedures and structures discussed below can similarly be used for a replacement mitral, tricuspid, or aortic valve.

Additional details and example designs for a prosthesis are described in U.S. Patent Nos. 8.403,983, 8,414,644, 8,652,203 and U.S. Patent Publication Nos. 2011/0313515, 2012/0215303, 2014/0277390, 2014/0277422, 2014/0277427, 2018/0021129, and 2018/0055629, the entirety of these patents and publications are hereby incorporated by reference and made a part of this specification. Further details and embodiments of a replacement heart valve or prosthesis and its method of implantation are described in U.S. Publication Nos. 2015/0328000 and 2016/0317301 the entirety of each of which is hereby incorporated by reference and made a part of this specification.

The delivery system **10** can be relatively flexible. In some embodiments, the delivery system **10** is particularly suitable for delivering a replacement heart valve to a mitral valve location through a transseptal approach (e.g., between the right atrium and left atrium via a transseptal puncture).

As shown in **Figure 1****,** the delivery system **10** can include a shaft assembly **12** comprising a proximal end **11** and a distal end **13,** wherein a handle **14** is coupled to the proximal end of the assembly **12.** The shaft assembly **12** can be used to hold the prosthesis for advancement of the same through the vasculature to a treatment location. The delivery system **10** can further comprise a relatively rigid live-on (or integrated) sheath **51** surrounding the shaft assembly **12** that can prevent unwanted motion of the shaft assembly **12.** The live-on sheath **51** can be attached at a proximal end of the shaft assembly **12** proximal to the handle **14,** for example at a sheath hub. The shaft assembly **12** can include an implant retention area **16** (shown in **Figures 2A-B** with **Figure 2A** showing the prosthesis **70** and **Figure 2B** with the prosthesis **70** removed) at its distal end that can be used for this purpose. In some embodiments, the shaft assembly **12** can hold an expandable prosthesis in a compressed state at implant retention area **16** for advancement of the prosthesis **70** within the body. The shaft assembly **12** may then be used to allow controlled expansion of the prosthesis **70** at the treatment location. In some embodiments, the shaft assembly **12** may be used to allow for sequential controlled expansion of the prosthesis **70** as discussed in detail below. The implant retention area **16** is shown in **Figures 2A-B** at the distal end of the delivery system **10,** but may also be at other locations. In some embodiments, the prosthesis **70** may be rotated in the implant retention area **16,** such as through the rotation of the inner shaft assembly **18** discussed herein.

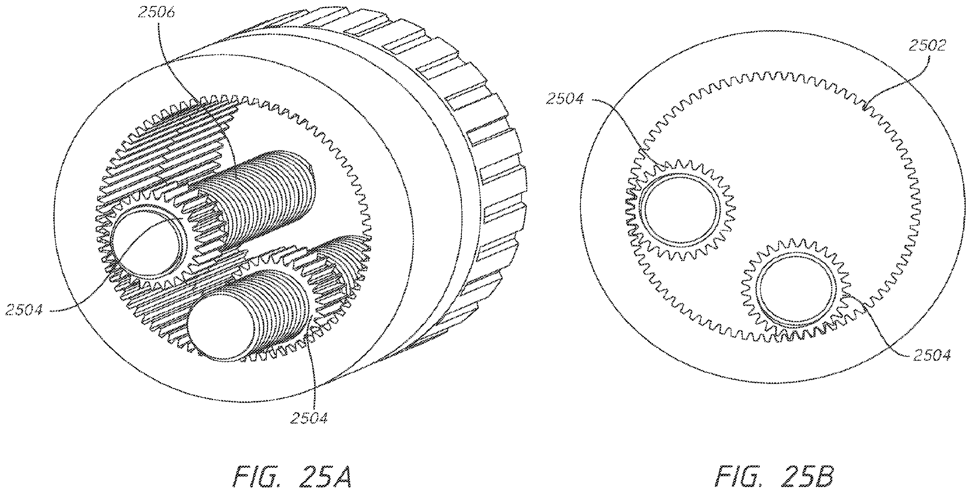

As shown in cross-sectional view of **Figures 2A-B****,** the distal end of the delivery system **10** can include one or more subassemblies such as an outer sheath assembly **22,** a mid shaft assembly **21,** a rail assembly **20,** an inner shaft assembly **18,** and a nose cone assembly **31** as will be described in more detail below. In some embodiments, the delivery system **10** may not have all of the assemblies disclosed herein. For example, in some embodiments a full mid shaft assembly may not be incorporated into the delivery system **10,** such as described in the embodiment of **Figures 25-36** below. In some embodiments, the assemblies disclosed below may be in a different radial order than is discussed.

In particular, embodiments of the disclosed delivery system **10** can utilize a steerable rail in the rail assembly **20** for steering the distal end of the delivery system **10,** allowing the implant to be properly located in a patient's body. As discussed in detail below, the steerable rail can be, for example, a rail shaft that extends through the delivery system **10** from the handle **14** generally to the distal end. In some embodiments, the steerable rail has a distal end that ends proximal to the implant retention area **16.** A user can manipulate the bending of the distal end of the rail, thereby bending the rail in a particular direction. In preferred embodiments, the rail has more than one bend along its length, thereby providing multiple directions of bending. As the rail is bent, it presses against the other assemblies to bend them as well, and thus the other assemblies of the delivery system **10** can be configured to steer along with the rail as a cooperating single unit, thus providing for full steerability of the distal end of the delivery system.

Once the rail is steered into a particular location in a patient's body, the prosthesis **70** can be advanced along or relative to the rail through the movement of the other sheaths/shafts relative to the rail and released into the body. For example, the rail can be bent into a desired position within the body, such as to direct the prosthesis **70** towards the native mitral valve. The other assemblies (e.g., the outer sheath assembly **22,** the mid shaft assembly **21,** the inner assembly **18,** and the nose cone assembly **31)** can passively follow the bends of the rail. Further, the other assemblies (e.g., the outer sheath assembly **22,** the mid shaft assembly **21,** the inner assembly **18,** and the nose cone assembly **31)** can be advanced together (e.g., relatively together. sequentially with one actuator, simultaneously, almost simultaneously, at the same time, closely at the same time) relative to the rail while maintaining the prosthesis **70** in the compressed position without releasing or expanding the prosthesis **70** (e.g., within the implant retention area **16).** The other assemblies (e.g., the outer sheath assembly **22,** the mid shaft assembly **21,** the inner assembly **18,** and the nose cone assembly **31)** can be advanced distally or proximally together relative to the rail. In some embodiments, only the outer sheath assembly **22,** mid shaft assembly **21,** and inner assembly **18** are advanced together over the rail. Thus, the nose cone assembly **31** may remain in the same position. The assemblies can be individually, sequentially, or simultaneously, translated relative to the inner assembly **18** in order to release the implant **70** from the implant retention area **16.**

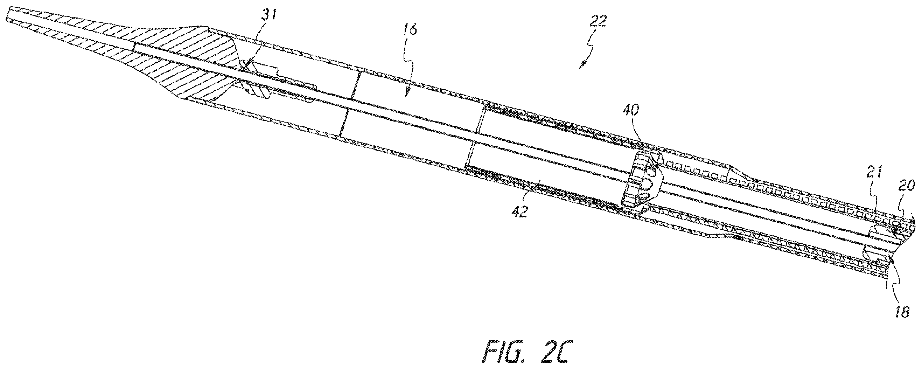

**Figure 2C** illustrates the sheath assemblies, specifically the outer sheath assembly **22,** the mid shaft assembly **21,** the inner shaft assembly **18,** and the nose cone assembly **31** having translated distally together along the rail assembly **20,** further details on the assemblies are below. In some embodiments, the outer sheath assembly **22,** the mid shaft assembly **21,** the inner shaft assembly **18,** and the nose cone assembly **31** translate together (e.g., relatively together, sequentially with one actuator, simultaneously, almost simultaneously, at the same time, closely at the same time). This distal translation can occur while the implant **70** remains in a compressed configuration within the implant retention area **16.**

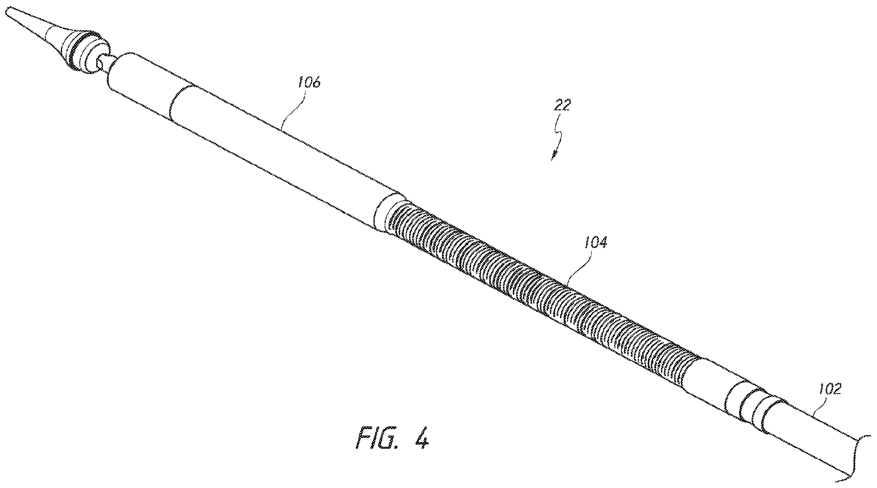

As shown in **Figures 2A-2C** and as further shown in **Figures 4-8****,** starting with the outermost assembly, the delivery system can include an outer sheath assembly **22** forming a radially outer covering, or sheath, to surround an implant retention area **16** and prevent the implant from radially expanding. Specifically, the outer sheath assembly **22** can prevent radial expansion of the distal end of the implant from radially expanding. Moving radially inward, the mid shaft assembly **21** can be composed of a mid shaft hypotube **43** with its distal end attached to an outer retention member or outer retention ring **42** for radially retaining a portion of the prosthesis in a compacted configuration, such as a proximal end of the prosthesis **70.** The mid shaft assembly **21** can be located within a lumen of the outer sheath assembly **22.** Moving further inwards, the rail assembly **20** can be configured for steerability, as mentioned above and further described below. The rail assembly **20** can be located within a lumen of the mid shaft assembly **21.** Moving further inwards, the inner shaft assembly **18** can be composed of an inner shaft with its distal end attached to inner retention member or inner retention ring **40** (such as a PEEK ring) for axially retaining the prosthesis, for example the proximal end of the prosthesis. The inner shaft assembly **18** can be located within a lumen of the rail assembly **20.** Further, the most radially-inward assembly is the nose cone assembly **31** which includes the nose cone shaft **27** having its distal end connected to the nose cone **28.** The nose cone **28** can have a tapered tip. The nose cone assembly **31** is preferably located within a lumen of the inner shaft assembly **18.** The nose cone assembly **31** can include a lumen for a guide wire to pass therethrough.

The shaft assembly **12,** and more specifically the nose cone assembly **31,** inner assembly **18,** rail assembly **20,** mid shaft assembly **21,** and outer sheath assembly **22,** can be collectively configured to deliver a prosthesis **70** positioned within the implant retention area **16** (shown in **Figure 2A****)** to a treatment location. One or more of the subassemblies can then be moved to allow the prosthesis **70** to be released at the treatment location. For example, one or more of the subassemblies may be movable with respect to one or more of the other subassemblies. The handle **14** can include various control mechanisms that can be used to control the movement of the various subassemblies as will also be described in more detail below. In this way, the prosthesis **70** can be controllably loaded onto the delivery system **10** and then later deployed within the body. Further, the handle **14** can provide steering to the rail assembly **20,** providing for bending/flexing/steering of the distal end of the delivery system **10.**

As will be discussed below, the inner retention member **40,** the outer retention ring **42,** and the outer sheath assembly **22** can cooperate to hold the prosthesis **70** in a compacted configuration. The inner retention member **40** is shown engaging struts **72** at the proximal end **301** of the prosthesis **70** in **Figure 2A****.** For example, slots located between radially extending teeth on the inner retention member **40** can receive and engage the struts **72** which may end in mushroom-shaped tabs **74** on the proximal end of the prosthesis **70.** The mid shaft assembly **21** can be positioned over the inner retention member **40** so that the first end **301** of the prosthesis **70** is trapped between the inner retention member **40** and the outer retention ring **42,** thereby securely attaching it to the delivery system **10** between the mid shaft assembly **21** and the inner retention member **40.** The outer sheath assembly **22** can be positioned to cover the second end **303** of the prosthesis **70.**

The outer retention member **42** may be attached to a distal end of the mid shaft hypotube **43** which can in turn be attached to a proximal tube **44** at a proximal end, which in turn can be attached at a proximal end to the handle **14.** The outer retention member **42** can provide further stability to the prosthesis **70** when in the compressed position. The outer retention member **42** can be positioned over the inner retention member **40** so that the proximal end of the prosthesis **70** is trapped therebetween, securely attaching it to the delivery system **10.** The outer retention member **42** can encircle a portion of the prosthesis **70,** in particular the first end **301,** thus preventing the prosthesis **70** from expanding. Further, the mid shaft assembly **21** can be translated proximally with respect to the inner assembly **18** into the outer sheath assembly **22,** thus exposing a first end **301** of the prosthesis **70** held within the outer retention member **42.** In this way the outer retention member **42** can be used to help secure a prosthesis **70** to or release it from the delivery system **10.** The outer retention member **42** can have a cylindrical or elongate tubular shape, and may be referred to as an outer retention ring, though the particular shape is not limiting.

The mid shaft hypotube **43** itself can be made of, for example, high density polyethylene (HDPE), as well as other appropriate materials as described herein. The mid shaft hypotube **43** can be formed of a longitudinally pre-compressed HDPE tube, which can provide certain benefits. For example, the pre-compressed HDPE tube can apply a force distally onto the outer retention member **42,** thus preventing accidental, inadvertent, and/or premature release of the prosthesis **70.** Specifically, the distal force by the mid shaft hypotube **43** keeps the distal end of the outer retention member **42** distal to the inner retention member **40,** thus preventing the outer retention member **42** from moving proximal to the inner retention member **40** before it is desired by a user to release the prosthesis **70.** This can remain true even when the delivery system **10** is bent/deflected at a sharp angle. Further disclosure for the outer retention member **42** and mid shaft hypotube **43** can be found in U.S. Pat. Pub. No. 2016/0317301, hereby incorporated by reference in its entirety.

As shown in **Figure 2A****,** the distal anchors **80** can be located in a delivered configuration where the distal anchors **80** point generally distally (as illustrated, axially away from the main body of the prosthesis frame and away from the handle of the delivery system). The distal anchors **80** can be restrained in this delivered configuration by the outer sheath assembly **22.** Accordingly, when the outer sheath **22** is withdrawn proximally, the distal anchors **80** can flip positions (e.g., bend approximately 180 degrees) to a deployed configuration (e.g., pointing generally proximally). **Figure 2A** also shows the proximal anchors **82** extending distally in their delivered configuration within the outer sheath assembly **22.** In other embodiments, the distal anchors **80** can be held to point generally proximally in the delivered configuration and compressed against the body of the prosthesis frame.

The delivery system **10** may be provided to users with a prosthesis **70** preinstalled. In other embodiments, the prosthesis **70** can be loaded onto the delivery system shortly before use, such as by a physician or nurse.

### Delivery System Assemblies

**Figures 4-8** illustrate further views of delivery system **10** with different assemblies translated proximally and described in detail.

Starting with the outermost assembly shown in **Figure 4****,** the outer sheath assembly **22** can include an outer proximal shaft **102** directly attached to the handle **14** at its proximal end and an outer hypotube **104** attached at its distal end. A capsule **106** can then be attached generally at the distal end of the outer hypotube **104.** In some embodiments, the capsule **106** can be 28 French or less in size. These components of the outer sheath assembly **22** can form a lumen for the other subassemblies to pass through.

The outer proximal shaft **102** may be a tube and is preferably formed of a plastic, but could also be a metal hypotube or other material. The outer hypotube **104** can be a metal hypotube which in some embodiments may be cut or have slots, as discussed in detail below. The outer hypotube **104** can be covered or encapsulated with a layer of ePTFE, PTFE, or other polymer/material so that the outer surface of the outer hypotube **104** is generally smooth.

A capsule **106** can be located at a distal end of the outer proximal shaft 102. The capsule **106** can be a tube formed of a plastic or metal material. In some embodiments, the capsule **106** is formed of ePTFE or PTFE. In some embodiments, this capsule **106** is relatively thick to prevent tearing and to help maintain a self-expanding implant in a compacted configuration. In some embodiments the material of the capsule **106** is the same material as the coating on the outer hypotube **104.** As shown, the capsule **106** can have a diameter larger than the outer hypotube **104,** though in some embodiments the capsule **106** may have a similar diameter as the hypotube **104.** In some embodiments, the capsule **106** may include a larger diameter distal portion and a smaller diameter proximal portion. In some embodiments, there may be a step or a taper between the two portions. The capsule **106** can be configured to retain the prosthesis **70** in the compressed position within the capsule **106.** Further construction details of the capsule **106** are discussed below.

The outer sheath assembly **22** is configured to be individually slidable with respect to the other assemblies. Further, the outer sheath assembly **22** can slide distally and proximally relative to the rail assembly **22** together with the mid shaft assembly **21,** inner assembly **18,** and nose cone assembly **31.**

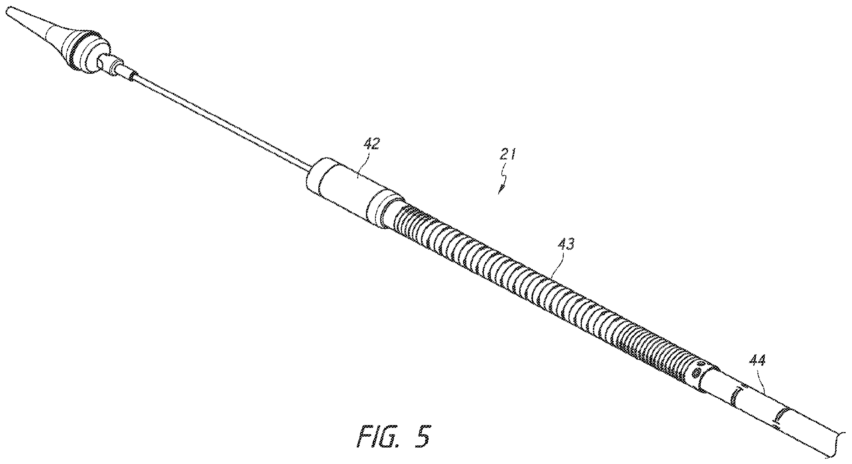

Moving radially inwardly, the next assembly is the mid shaft assembly **21.** **Figure 5** shows a similar view as **Figure 4****,** but with the outer sheath assembly **22** removed, thereby exposing the mid shaft assembly **21.**

The mid shaft assembly **21** can include a mid shaft hypotube **43** generally attached at its proximal end to a mid shaft proximal tube **44,** which in turn can be attached at its proximal end to the handle **14,** and an outer retention ring **42** located at the distal end of the mid shaft hypotube **43.** Thus, the outer retention ring **42** can be attached generally at the distal end of the mid shaft hypotube **43.** These components of the mid shaft assembly **21** can form a lumen for other subassemblies to pass through.

Similar to the other assemblies, the mid shaft hypotube **43** and/or mid shaft proximal tube **44** can comprise a tube, such as a hypodermic tube or hypotube (not shown). The tubes can be made from one of any number of different materials including Nitinol, stainless steel, and medical grade plastics. The tubes can be a single piece tube or multiple pieces connected together. Using a tube made of multiple pieces can allow the tube to provide different characteristics along different sections of the tube, such as rigidity and flexibility. The mid shaft hypotube **43** can be a metal hypotube which in some embodiments may be cut or have slots as discussed in detail below. The mid shaft hypotube **43** can be covered or encapsulated with a layer of ePTFE, PTFE, or other material so that the outer surface of the mid shaft hypotube **43** is generally smooth.

The outer retention ring **42** can be configured as a prosthesis retention mechanism that can be used to engage with the prosthesis **70,** as discussed with respect to **Figure 2A****.** For example, the outer retention ring **42** may be a ring or covering that is configured to radially cover the struts **72** on the prosthesis **70.** The outer retention ring **42** can also be considered to be part of the implant retention area **16,** and may be at the proximal end of the implant retention area **16.** With struts or other parts of a prosthesis **70** engaged with the inner retention member **40,** discussed below the outer retention ring **42** can cover both the prosthesis **70** and the inner retention member **40** to secure the prosthesis **70** on the delivery system **10.** Thus, the prosthesis **70** can be sandwiched between the inner retention member **40** of the inner shaft assembly **18** and the outer retention ring **42** of the mid shaft assembly **21.**

The mid shaft assembly **21** is disposed so as to be individually slidable with respect to the other assemblies. Further, mid shaft assembly **21** can slide distally and proximally relative to the rail assembly **22** together with the outer sheath assembly **22,** mid inner assembly **18,** and nose cone assembly **31.**

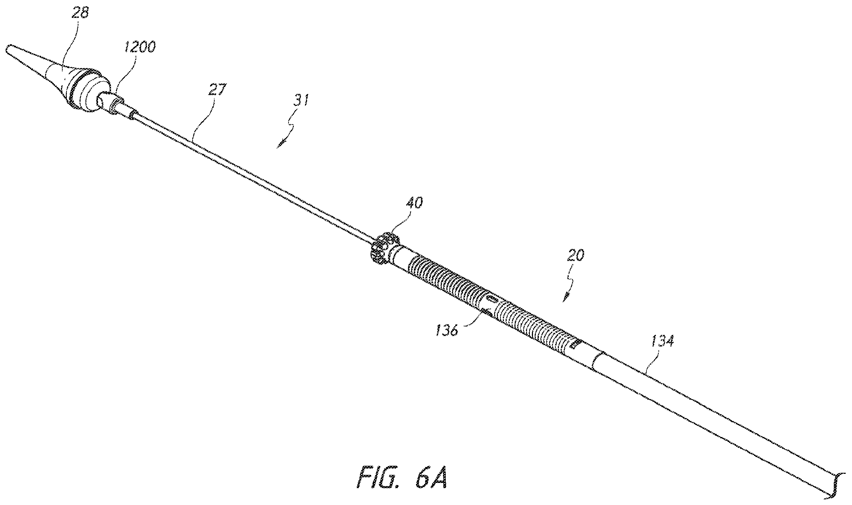

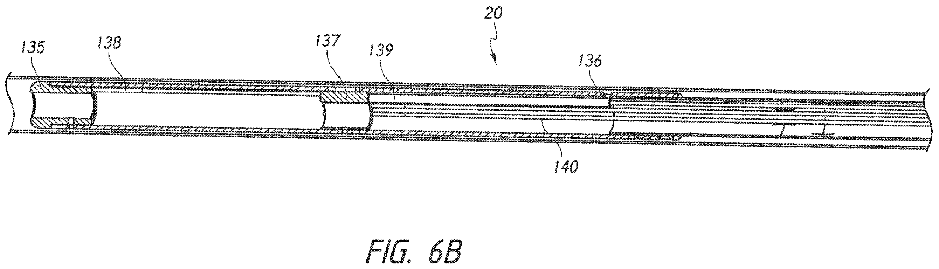

Next, radially inwardly of the mid shaft assembly **21** is the rail assembly **20.** **Figure 6A** shows approximately the same view as **Figure 5****,** but with the mid shaft assembly **21** removed, thereby exposing the rail assembly **20.** **Figure 6B** further shows a cross-section of the rail assembly **20** to view the pull wires. The rail assembly **20** can include a rail shaft **132** (or rail) generally attached at its proximal end to the handle **14.** The rail shaft **132** can be made up of a rail proximal shaft **134** directly attached to the handle at a proximal end and a rail hypotube **136** attached to the distal end of the rail proximal shaft **134.** The rail hypotube **136** can further include an atraumatic rail tip at its distal end. Furth, the distal end of the rail hypotube **136** can abut a proximal end of the inner retention member **40,** as shown in **Figure 6****.** In some embodiments, the distal end of the rail hypotube **136** can be spaced away from the inner retention member **40.** These components of the rail shaft assembly **20** can form a lumen for the other subassemblies to pass through.

As shown in **Figure 6B****,** attached to an inner surface of the rail hypotube **136** are one or more pull wires which can be used apply forces to the rail hypotube **136** and steer the rail assembly **20.** The pull wires can extend distally from the knobs in the handle **14,** discussed below, to the rail hypotube **136.** In some embodiments, pull wires can be attached at different longitudinal locations on the rail hypotube **136,** thus providing for multiple bending locations in the rail hypotube **136,** allowing for multidimensional steering.

In some embodiments, a distal pull wire **138** can extend to a distal section of the rail hypotube **136** and two proximal pull wires **140** can extend to a proximal section of the rail hypotube **136,** however, other numbers of pull wires can be used, and the particular amount of pull wires is not limiting. For example, a two pull wires can extend to a distal location and a single pull wire can extend to a proximal location. In some embodiments, ring-like structures attached inside the rail hypotube **136,** known as pull wire connectors, can be used as attachment locations for the pull wires, such as proximal ring **137** and distal ring **135.** In some embodiments, the rail assembly **20** can include a distal pull wire connector **135** and a proximal pull wire connector **139.** In some embodiments, the pull wires can directly connect to an inner surface of the rail hypotube **136.**

The distal pull wire **138** can be connected (either on its own or through a connector **135)** generally at the distal end of the rail hypotube **136.** The proximal pull wires **140** can connect (either on its own or through a connector **137)** at a location approximately one quarter, one third, or one half of the length up the rail hypotube **136** from the proximal end. In some embodiments, the distal pull wire **138** can pass through a small diameter pull wire lumen **139** (e.g., tube, hypotube, cylinder) attached on the inside of the rail hypotube **136.** This can prevent the wires **138** from pulling on the rail hypotube **136** at a location proximal to the distal connection. Further, the lumen **139** can act as compression coils to strengthen the proximal portion of the rail hypotube **136** and prevent unwanted bending. Thus, in some embodiments the lumen **139** is only located on the proximal half of the rail hypotube **136.** In some embodiments, multiple lumens **139,** such as spaced longitudinally apart or adjacent, can be used per distal wire **139.** In some embodiments, a single lumen **139** is used per distal wire **139.** In some embodiments, the lumen **139** can extend into the distal half of the rail hypotube **136.** In some embodiments, the lumen **139** is attached on an outer surface of the rail hypotube **136.** In some embodiments, the lumen **139** is not used.

For the pair of proximal pull wires **140,** the wires can be spaced approximately 180° from one another to allow for steering in both directions. Similarly, if a pair of distal pull wires **138** is used, the wires can be spaced approximately 180° from one another to allow for steering in both directions. In some embodiments, the pair of distal pull wires **138** and the pair of proximal pull wires **140** can be spaced approximately 90° from each other. In some embodiments, the pair of distal pull wires **138** and the pair of proximal pull wires **140** can be spaced approximately 0° from each other. However, other locations for the pull wires can be used as well, and the particular location of the pull wires is not limiting. In some embodiments, the distal pull wire **138** can pass through a lumen **139** attached within the lumen of the rail hypotube **136.** This can prevent an axial force on the distal pull wire **138** from creating a bend in a proximal section of the rail hypotube **136.**

The rail assembly **20** is disposed so as to be slidable over the inner shaft assembly **18** and the nose cone assembly **31.** In some embodiments, the outer sheath assembly **22,** the mid shaft assembly **21,** the inner shaft assembly **22,** and the nose cone assembly **31** can be configured to slide together along or relative to the rail assembly **20,** such as proximally and distally with or without any bending of the rail assembly **20.** In some embodiments, the outer sheath assembly **22,** the mid shaft assembly **21,** the inner shaft assembly **22,** and the nose cone assembly **31** can be configured to retain the implant **70** in a compressed position when they are simultaneously slid along or relative to the rail assembly **20.**

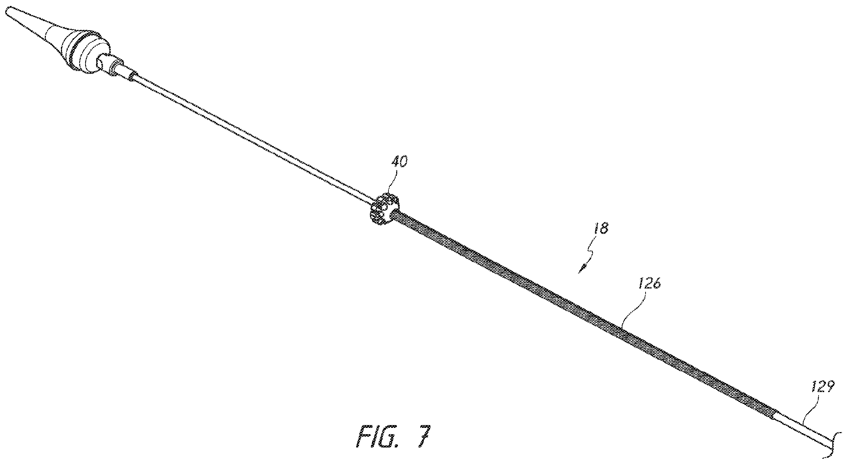

Moving radially inwards, the next assembly is the inner shaft assembly **18.** **Figure 7** shows approximately the same view as **Figure 6A****,** but with the rail assembly **20** removed, thereby exposing the inner shaft assembly **18.**

The inner shaft assembly **18** can include an inner shaft **122** generally attached at its proximal end to the handle **14,** and an inner retention ring **40** located at the distal end of the inner shaft **122.** The inner shaft **122** itself can be made up of an inner proximal shaft **124** directly attached to the handle **14** at a proximal end and a distal section **126** attached to the distal end of the inner proximal shaft **124.** Thus, the inner retention ring **40** can be attached generally at the distal end of the distal section **126.** These components of the inner shaft assembly **18** can form a lumen for the other subassemblies to pass through.

Similar to the other assemblies, the inner proximal shaft **124** can comprise a tube, such as a hypodermic tube or hypotube (not shown). The tube can be made from one of any number of different materials including Nitinol, cobalt chromium, stainless steel, and medical grade plastics. The tube can be a single piece tube or multiple pieces connected together. A tube comprising multiple pieces can provide different characteristics along different sections of the tube, such as rigidity and flexibility. The distal section **126** can be a metal hypotube which in some embodiments may be cut or have slots as discussed in detail below. The distal section **126** can be covered or encapsulated with a layer of ePTFE, PTFE, or other material so that the outer surface of the distal section **126** is generally smooth.

The inner retention member **40** can be configured as a prosthesis retention mechanism that can be used to engage with the prosthesis **70,** as discussed with respect to **Figure 2A****.** For example, the inner retention member **40** may be a ring and can include a plurality of slots configured to engage with struts **72** on the prosthesis **70.** The inner retention member **40** can also be considered to be part of the implant retention area **16,** and may be at the proximal end of the implant retention area **16.** With struts or other parts of a prosthesis **70** engaged with the inner retention member **40,** the outer retention ring **42** can cover both the prosthesis and the inner retention member **40** to secure the prosthesis on the delivery system **10.** Thus, the prosthesis **70** can be sandwiched between the inner retention member **40** of the inner shaft assembly **18** and the outer retention ring **42** of the mid shaft assembly **21.**

The inner shaft assembly **18** is disposed so as to be individually slidable with respect to the other assemblies. Further, the inner assembly **18** can slide distally and proximally relative to the rail assembly **22** together with the outer sheath assembly **22,** mid shaft assembly **21,** and nose cone assembly **31.**

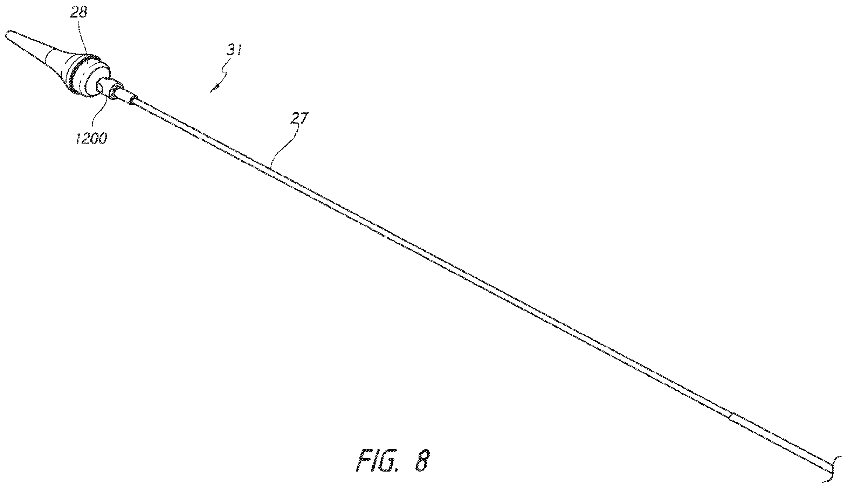

Moving further inwardly from the inner shaft assembly **18** is the nose cone assembly **31** also seen in **Figure 8****.** This may be a nose cone shaft **27**, and in some embodiments, may have a nose cone **28** on its distal end. The nose cone **28** can be made of polyurethane for atraumatic entry and to minimize injury to venous vasculature. The nose cone **28** can also be radiopaque to provide for visibility under fluoroscopy.

The nose cone shaft **27** may include a lumen sized and configured to slidably accommodate a guide wire so that the delivery system **10** can be advanced over the guide wire through the vasculature. However, embodiments of the system **10** discussed herein may not use a guide wire and thus the nose cone shaft **27** can be solid. The nose cone shaft **27** may be connected from the nose cone **28** to the handle, or may be formed of different segments such as the other assemblies. Further, the nose cone shaft **27** can be formed of different materials, such as plastic or metal, similar to those described in detail above.

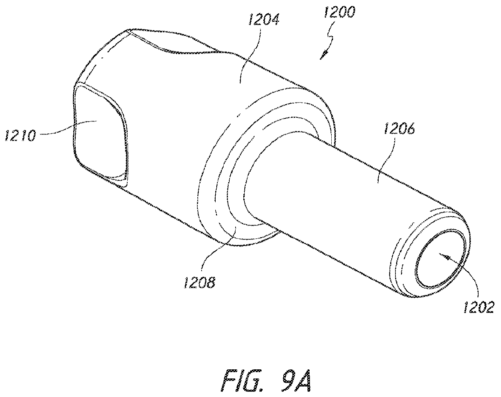

In some embodiments, the nose cone shaft **27** includes a guide wire shield **1200** located on a portion of the nose cone shaft **27.** Examples of such a guide wire shield can be found in **Figures 9A-B****.** In some embodiments, the guide wire shield **1200** can be proximal to the nose cone **28.** In some embodiments, the guide wire shield **1200** can be translatable along the nose cone shaft **27.** In some embodiments, the guide wire shield **1200** can be locked in place along the nose cone shaft **27.** In some embodiments, the guide wire shield **1200** can be at least partially located within the nose cone **28.**

Advantageously, the guide wire shield **1200** can allow for smooth tracking of the guide wire with the implant **70** loaded, and can provide a large axial diameter landing zone for a distal end of the implant so that the distal end of the implant **70** may spread out properly and be arranged in a uniform radial arrangement. This uniformity allows for proper expansion. Furthermore, the guide wire shield **1200** can prevent kinking or damaging of the nose cone shaft **27** during compression/crimping of the prosthesis **70,** which can exert a large compressive force on the nose cone shaft **27.** As the prosthesis **70** can be crimped onto the guide wire shield **1200** instead of directly on the nose cone shaft **27,** the guide wire shield **1200** can provide a protective surface.

As shown, the guide wire shield **1200** can include a lumen **1202** configured to surround the nose cone shaft **27.** The guide wire shield **1200** can include a larger diameter distal end **1204** and a smaller diameter proximal end **1206.** In some embodiments, the dimension change between the two ends can be tapered, or can be a step **1208** such as shown in **Figure 9A****.** The distal end **1204** can include a number of indents **1210** for easier gripping by a user, but may not be included in all embodiments. The proximal end **1206** and the distal end **1204** can both be generally cylindrical, but the particular shape of the guide wire shield **1200** is not limiting.

The distal end of the prosthesis **70** can be crimped so that it is radially in contact with the proximal end **1206** of the guide wire shield **1200.** This can allow the prosthesis **70** to be properly spread out around an outer circumference of the proximal end **1206** of the guide wire shield **1200.** In some embodiments, the distal end of the prosthesis **70** can longitudinally abut against the proximal end of the distal end **1204** (e.g., at the step **1208),** thus providing a longitudinal stop.

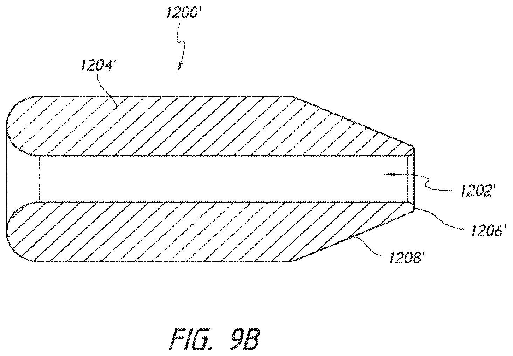

**Figure 9B** shows an alternate embodiment of a guide wire shield **1200'** having a more tapered configuration. As shown, the proximal end **1206'** of the guide wire shield **1200**' can be a single radially outward taper **1208'** to the distal end **1204'** of the guide wire shield **1200**', which can be generally cylindrical. The guide wire shield **1200'** can also include a lumen **1202'** for receiving the nose cone shaft **27.**

The nose cone assembly **31** is disposed so as to be individually slidable with respect to the other assemblies. Further, the nose cone assembly **31** can slide distally and proximally relative to the rail assembly **22** together with the outer sheath assembly **22,** mid shaft assembly **21,** and inner assembly **18.**

In some embodiments, one or more spacer sleeves (not shown) can be used between different assemblies of the delivery system **10.** For example, a spacer sleeve can be located concentrically between the mid shaft assembly and the rail assembly **20,** generally between the mid hypotube **43** and rail hypotube **136.** In some embodiments, the spacer sleeve can be generally embedded in the hypotube **43** of the mid shaft assembly **21,** such as on an inner surface of the mid shaft assembly **21.** In some embodiments, a spacer sleeve can be located concentrically between the rail assembly **20** and the inner assembly **18,** generally within the rail hypotube **136.** In some embodiments, a spacer sleeve can be used between the outer sheath assembly **22** and the mid shaft assembly **21.** In some embodiments, a spacer sleeve can be used between the inner assembly **18** and the nose cone assembly **31.** In some embodiments, 4, 3, 2, or 1 of the above-mentioned spacer sleeves can be used. The spacer sleeves can be used in any of the above positions.

The spacer sleeve can be made of a polymer material such as braided Pebax^{®} and can be lined, for example with PTFE, on the inner diameter, though the particular material is not limiting. The spacer sleeve can advantageously reduce friction between the steerable rail assembly **20** and its surrounding assemblies. Thus, the spacer sleeves can act as a buffer between the rail assembly **20** and the inner/nose cone assembly **18/30.** Further, the spacer sleeve can take up any gap in radius between the assemblies, preventing compressing or snaking of the assemblies during steering. In some embodiments, the spacer sleeve may include cuts or slots to facilitate bending of the spacer sleeve. In some embodiments, the spacer sleeve may not include any slots, and may be a smooth cylindrical feature.

The spacer sleeve can be mechanically contained by the other lumens and components, and is thus not physically attached to any of the other components, allowing the spacer sleeve to be "floating" in that area. The floating aspect of the spacer sleeve allows it to move where needed during deflection and provide a support and/or lubricious bear surface/surfaces. Accordingly, the floating aspect allows the delivery system **10** to maintain flex forces. However, in some embodiments, the spacer sleeve can be connected to other components.

### Hypotube/Shaft Construction

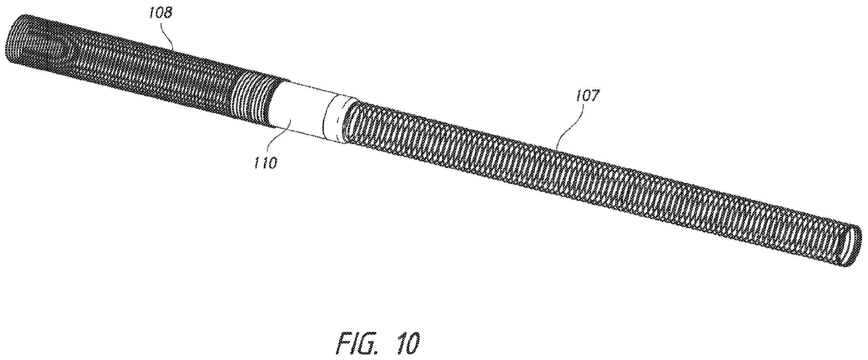

As discussed above, the outer sheath assembly **22,** the mid shaft assembly **21,** the inner assembly **18,** and the rail assembly **20** can contain an outer hypotube **104,** a mid shaft hypotube **43,** a distal section **126,** and a rail hypotube **136,** respectively. Each of these hypotubes/sections/shafts can be laser cut to include a number of slots, thereby creating a bending pathway for the delivery system to follow. While different slot assemblies are discussed below, it will be understood that any of the hypotubes can have any of the slot configurations discussed below. **Figures 10-14** show the different hypotubes in isolated format.

The outer hypotube **104,** shown in **Figure 10****,** can be generally formed of a metal coil or a plurality of metal coils. In some embodiments, the outer hypotube **104** can be formed of a proximal metal coil **107** and a distal metal coil **108.** The proximal metal coil **107** and the distal metal coil **108** can be longitudinally separated by a tube portion **110,** such as shown in **Figure 10****.** However, in some embodiments the proximal metal coil **107** and the distal metal coil **108** connect. The proximal metal coil **107** and the distal metal coil **108** can be connected to an outer surface of the tube portion **110,** for example at the distal end of the proximal metal coil **107** and a proximal end of the distal metal coil **108,** in order to form the full outer hypotube **104.** In some embodiments, the proximal metal coil **107** and the distal metal coil **108** are generally the same. In some embodiments, the proximal metal coil **107** and the distal metal coil **110** are different, for example in spacing between coils, curvature, diameter, etc. In some embodiments, the distal metal coil **108** has a larger diameter than the proximal metal coil **107,** such as when the distal metal coil **108** forms the large diameter of the capsule **106.** In some embodiments, they have the same diameter. In some embodiments, one or both of the metal coils **108/107** can form the capsule **106.** The coils can be coated by polymer layers, such as described in detail below regarding the capsule construction. The coil construction can allow the outer hypotube **104** to follow the rail in any desired direction.

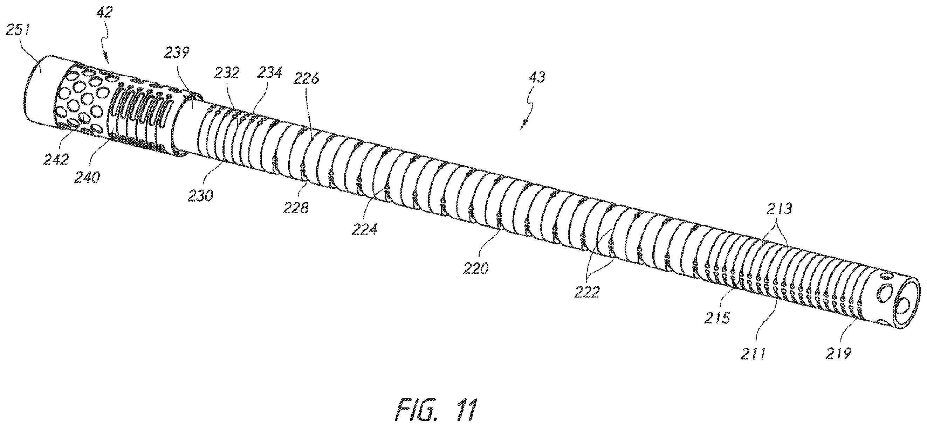

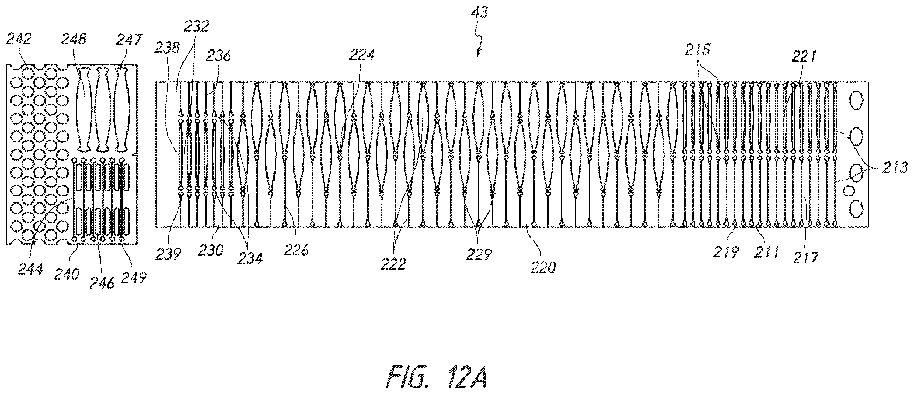

Moving radially inwardly, **Figures 11-12B** shows that the mid shaft hypotube **43** can be a metal laser cut hypotube, such as a lasercut Nitinol hypotube. **Figure 12A** illustrates a flat pattern of **Figure 11****.** As shown in the figures, the hypotube **43** can have a number of slots/apertures cut into the hypotube. In some embodiments, the cut pattern can be the same throughout. In some embodiments, the mid shaft hypotube **43** can have different sections having different cut patterns.

For example, the proximal end of the mid shaft hypotube **43** can be a first section **210** having a plurality circumferentially extending slot pairs **213** spaced longitudinally along the first section **211.** Generally, two slots are cut around each circumferential location forming almost half of the circumference. Accordingly, two backbones **215** are formed between the slots **213** extending up the length of the first section **211.** The slot pairs **213** can be composed of a first thin slot **217.** A second slot **221** of each of the slot pairs **213** can be thicker than the first slot **217,** such as 1, 2, 3, 4, or 5 times thicker. In some embodiments, the second slot **217** can be generally the same longitudinal thickness throughout the slot. Each of the slots of the slot pair **213** can end in a teardrop shape **219** in some embodiments to facilitate bending.

Moving distally, the mid shaft hypotube **43** can include a second section **220** having a number of slot pairs **222.** Similar to the first section **211,** the second section **220** can have a plurality of circumferentially extending slots spaced longitudinally along the second section **220.** Generally, two slots (e.g., one slot pair **222)** are cut around each circumferential location, forming almost half of a circumference. Accordingly, "backbones" **224** can be formed between the slots extending up the length of the second section **220.** Each slot pair **222** can include a first slot **226** that is generally thin and has no particular shape (e.g., it can look the same as the slots **213** in the first section **211),** and a second slot **228** that is significantly longitudinally thicker than the first slot **226.** The second slot **228** can be narrower at its ends and longitudinally thicker in its middle portion, thereby forming a curved slot. Moving longitudinally along the second section **220,** each slot pair **222** can be offset approximately 45 or 90 degrees as compared to longitudinally adjacent slot pairs **222.** In some embodiments, a second slot pair **222** is offset 90 degrees from an adjacent first slot pair **222,** and a third slot pair **222** adjacent the second slot pair **222** can have the same configuration of the first slot pair **222.** This repeating pattern can extend along a length of the second section **220,** thereby providing a particular bending direction induced by the second slot **228** of the slot pairs **222.** Accordingly, the "backbone" **224** shifts circumferential position due to the offsetting of adjacent shifting slot pairs **222.** Each of the slots of the slot pair **222** can end in a teardrop shape **229** in some embodiments to facilitate bending.

Moving distally, the mid shaft hypotube **43** can have a third section **230** having a number of slots. The outer retention ring **240** can be attached to a distal end of the third section **230.** The third section **230** can have circumferentially extending slot pairs **232,** each slot on the slot pair extending about half way along the circumference to form the two backbones **234.** The slot pairs **232** can be composed of a first thin slot **236,** similar to the slots **213** discussed in the first section **211.** A second slot **238** of each of the slot pairs **232** can be thicker than the first slot **236,** such as 1, 2, 3, 4, or 5 times thicker. In some embodiments, the second slot **238** can be generally the same longitudinal thickness throughout the slot, unlike the second slot **228** of the second section **220.** The first slots **236** and the second slots **238** can be circumferentially aligned along a length of the third section **230** so that all of the first slots **236** are in the same circumferential position and all of the second slots **238** are in the same circumferential position. The second slots **238** can be aligned with one of the circumferential positions of the second slots **228** of the second section **220.** Each of the slots of the slot pair **232** can end in a teardrop shape **239** in some embodiments to facilitate bending.

In some embodiments, an outer retention ring strengthener **240** which can partially or fully circumferentially surround the outer retention member **40** can have a number of slots/holes/apertures as well, such as shown in **Figures 11-12****.** This can allow it to bend over curves, especially tight curves. In some embodiments, the distal end of the strengthener **240** includes a number of generally circular/elliptical holes **242.** This can last for approximately half of the length of the strengthener **240.** On the proximal half, one circumferential half of the strengthener **240** can include repeating thin slots **244** spaced by elongate ovoid holes **246.** For example, two circumferentially spaced apart elongate ovoid holes **246** can be between each thin slot **244.** Each of the slots **244** can end in a teardrop shape **249** in some embodiments to facilitate bending. On the other circumferential half of the proximal section, the strengthener **240** can include a number of large slots **248,** for example 1, 2, 3, 4, or 5 large slots **248** spaced longitudinally apart. The large slots **248** can be larger in the middle and narrow towards each circumferential end. The large slots **248** may include ending expansions **247** to facilitate flexibility.

Additionally, the outer retention strengthener **240** can provide strength to lower deployment forces, protect the prosthesis **70** from any metal layers, and can add strength. In some embodiments, the liner can **240** be a polymer, such as PTFE, though the type of polymer or material is not limiting. In some embodiments, the strengthener **240** can be a metal. In some embodiments, the strengthener **240** can further include an outer polymer layer/jacket, such as a Pebax ^{®} jacket. This prevents the strengthener **240** from catching on the outer sheath assembly **22.**

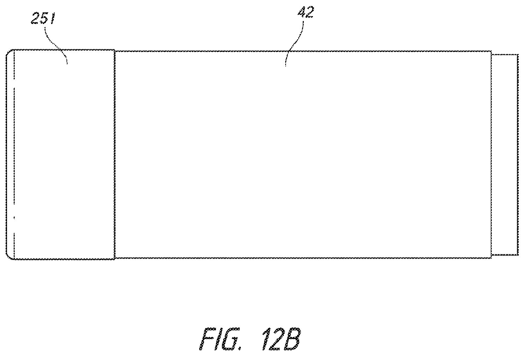

In certain embodiments, the outer retention ring **42** can further include an inner liner for smoothly transitioning over the prosthesis **70.** The inner liner can be PTFE or etched PTFE, though the particular material is not limiting and other reduced friction polymers can be used. As shown in **Figure 12B****,** to prevent delamination during loading of the implant **70,** the liner **251** may not be flush at the distal end of the outer retention ring **42.** Instead, the liner **251** can be extended and inverted at the distal end in order to cover the distal end of the outer retention ring **42.** In some embodiments, the liner **251** can cover an outer surface of the strengthener **240** as well. This can create a seamless rolled reinforced tip of the liner **251.** The liner **251** can fully or partially cover an outer surface of the outer retention ring **42,** for example 1/4, 1/3, 1/2, 2/3, 3/4 (or greater than 1/4, 1/3, 1/2, ¾), or all of the outer retention ring **42.** This solution is advantageous over previously known methods, such as disclosed in U.S. Pat. No. 6,622,367, incorporated by reference in its entirety, as PTFE lined applications do not adhere particularly well to reinforcements or the outer jacket. By inverting the liner **251** and fusing it to the outer retention ring **42** and/or the strengthener **240** and/or an outer polymer jacket on the strengthener **240/outer** retention ring **42,** this creates a seamless reinforced tip that can mitigate delamination. Delamination is a serious concern because the delaminated liner can tear and embolize during deployment, and the delaminated layer can cause extremely high loading and deployment forces. Delaminated layers can also cause lumen translation problems by locking up shafts thereby adding translational force requirements.

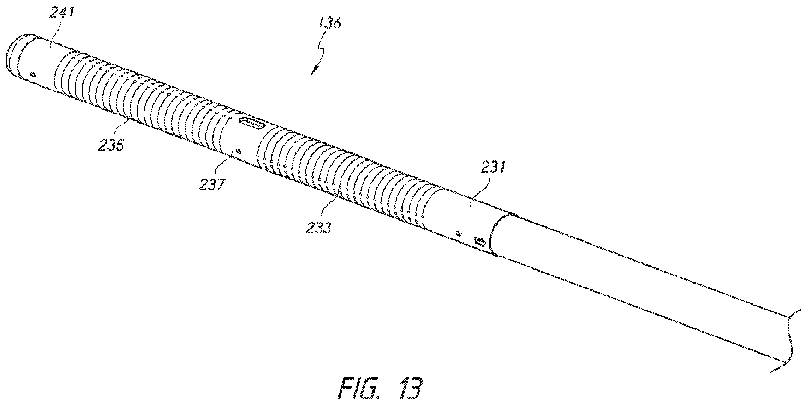

Next, again moving radially inward, **Figure 13** shows an embodiment of the rail hypotube **136** (distal end towards the right). The rail hypotube **136** can also contain a number of circumferential slots. The rail hypotube **136** can generally be broken into a number of different sections. At the most proximal end is an uncut (or unslotted) hypotube section **231.** Moving distally, the next section is the proximal slotted hypotube section **133.** This section includes a number of circumferential slots cut into the rail hypotube **136.** Generally, two slots are cut around each circumferential location forming almost half of the circumference. Accordingly, two backbones are formed between the slots extending up the length of the hypotube **136.** This is the section that can be guided by the proximal pull wires **140.** Moving further distally is the location **237** where the proximal pull wires **140** connect, and thus slots can be avoided. Thus section is just distal of the proximally slotted section.

Distally following the proximal pull wire connection area is the distal slotted hypotube section **235.** This section is similar to the proximal slotted hypotube section **233,** but has significantly more slots cut out in an equivalent length. Thus, the distally slotted hypotube section **235** provides easier bending than the proximally slotted hypotube section **233.** In some embodiments, the proximal slotted section **233** can be configured to experience a bend of approximately 90 degrees with a half inch radius whereas the distal slotted section **135** can bend at approximately 180 degrees within a half inch. Further, as shown in **Figure 13****,** the spines of the distally slotted hypotube section **235** are offset from the spines of the proximally slotted hypotube section **233.** Accordingly, the two sections will achieve different bend patterns, allowing for three-dimensional steering of the rail assembly **20.** In some embodiments, the spines can be offset 30, 45. or 90 degrees, though the particular offset is not limiting. In some embodiments, the proximally slotted hypotube section **233** can include compression coils. This allows for the proximally slotted hypotube section **233** to retain rigidity for specific bending of the distally slotted hypotube section **235.**

At the distalmost end of the distal slotted hypotube section **235** is the distal pull wire connection area **241** which is again a non-slotted section of the rail hypotube **136.**

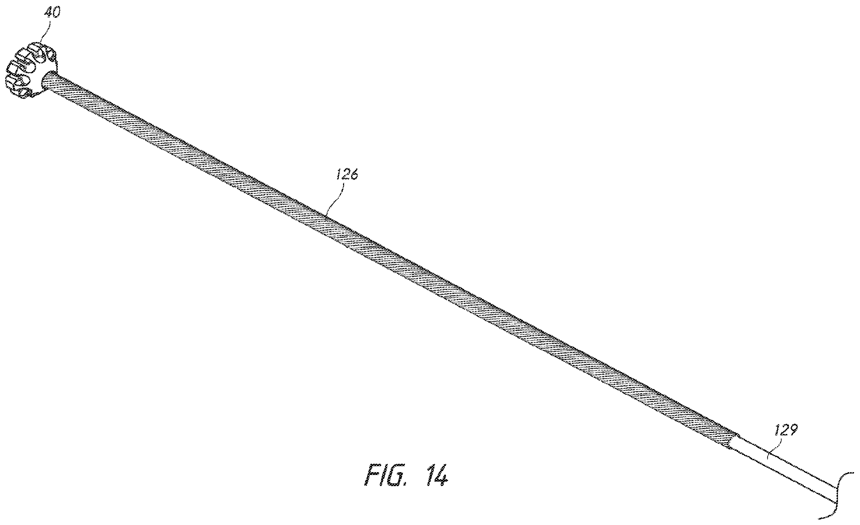

Moving radially inwardly in **Figure 14****,** the inner assembly **18** is composed generally of two sections. The proximal section is a hypotube **129,** either slotted or non-slotted. The distal section **126,** which at least partially overlaps an outer surface of the proximal hypotube **129,** can be designed to be particularly flexible. For example, the distal section **126** can be more flexible than any of the other shafts discussed herein. In some embodiments, the distal section **126** can be more flexible than any shaft discussed herein other than the nose cone shaft **27.** In some embodiments, the distal section **126** can be a flexible tube or hypotube. In some embodiments, the distal section **126** can be a cable, such as a flexible cable. For example, the cable can several strands of wire, such as metal, plastic, polymer, ceramic, etc., wound together to form a rope or cable. Because the cable is so flexible, it can more easily bend with the rail assembly **20.** Further, the cable can be smooth, which allows the rail assembly **20** to track over a smooth surface, eliminating the need for any inner liner on the rail assembly **20.**

### Capsule Construction

The capsule **106** can be formed from one or more materials, such as PTFE, ePTFE, polyether block amide (Pebax^{®}), polyetherimide (Ultem^{®}), PEEK, urethane, Nitinol, stainless steel, and/or any other biocompatible material. The capsule **106** is preferably compliant and flexible while still maintaining a sufficient degree of radial strength to maintain a replacement valve **70** within the capsule **106** without substantial radial deformation, which could increase friction between the capsule **106** and a replacement valve **70** contained therein. The capsule **106** also preferably has sufficient column strength to resist buckling of the capsule **106,** and sufficient tear resistance to reduce or eliminate the possibility of the replacement valve **70** tearing and/or damaging the capsule **106.** Flexibility of the capsule **106** can be advantageous, particularly for a transseptal approach. For example, while being retracted along a curved member, for example while tracking over a rail assembly as described herein, the capsule **106** can flex to follow the curved member without applying significant forces upon the curved member, which may cause the curved member to decrease in radius. More specifically, the capsule **106** can bend and/or kink as it is being retracted along such a curved member such that the radius of the curved member is substantially unaffected.

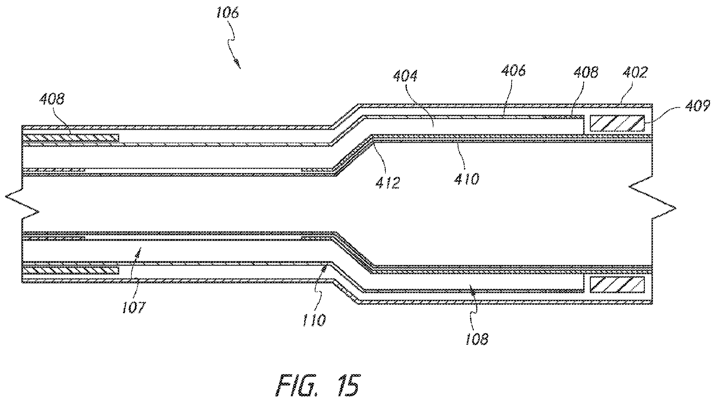

**Figure 15** shows embodiments of a capsule **106** that can be used with embodiments of the delivery system **10.** The capsule **106** may include any of the materials and properties discussed above. With many implant capsules, compression resistance and flexibility are typically balanced together, as improved flexibility can lead to worse compression resistance. Thus, there tends to be a choice made between compression resistance and flexibility. However, disclosed are embodiments of a capsule **106** that can achieve both high compression resistance as well as high flexibility. Specifically, the capsule **106** can bend in multiple directions.

In particular, a metal hypotube can provide radial strength and compression resistance, while specific slots/cuts in the hypotube can enable the flexibility of the capsule **106.** In some embodiments, a thin liner and a jacket can surround the capsule **106,** such as a polymer layer, to prevent any negative interactions between the implant **70** and the capsule **106.**

In some embodiments, the capsule **106** can have a particular construction to allow for it to achieve advantageous properties, as shown in **Figure 15****.** The capsule 106 can be made of several different layers to provide such properties.

In some embodiments, the capsule **106** can be formed of a metal layer **404,** which gives the capsule **106** its structure. This metal layer **404** can include the coils discussed with respect to **Figure 10****,** or could be one or more hypotubes. The capsule **106** is then covered on an outer surface by a polymer layer and on an inner surface by a liner. All of these features are discussed in detail below.

As mentioned, the metal layer **404** can be, for example, a metal hypotube or laser cut hypotube. In some embodiments, the metal layer **404** can be a metal coil or helix, as discussed in detail above with respect to **Figure 10****.** Though not limiting, the metal layer **404** can have a thickness of 0.007 inches (or about 0.007 inches).

If a metal coil, such as shown in **Figure 10****,** is used, the coil dimensions can stay the same throughout a length of the metal layer **404.** However, in some embodiments the coil dimensions can vary along a length of the metal layer **404.** For example, the coils can vary between coils having a 0.014-inch gap with a 0.021-inch pitch (e.g., small coils), coils having a 0.020 inch-gap with a 0.02-inch pitch (e.g., large coils), and coils having a 0.020-inch gap with a 0.027-inch pitch (e.g., spaced large coils). However, these particular dimensions are merely examples, and other designs can be used as well.

The distalmost end of the metal layer **404** can be formed out of the small coils. Moving proximally, the metal layer **404** may then transition to a section of large coils, followed again by a section of small coils, and then finally the proximalmost section can be the spaced large coils. As an example set of lengths, though not limiting, the distalmost small coil section may have a length of 10 mm (or about 10 mm). Moving proximally, the adjacent large coil section may extend 40 mm (or about 40 mm) to 60 mm (or about 60 mm) in length. These two sections would be found in the distal metal coil **108** shown in **Figure 10****.** Moving to the proximal metal coil **107** shown in **Figure 10****,** the small coil section can have a length of 10 mm (or about 10 mm). The remaining portion of the proximal metal coil **107** can be the spaced large coil section. The spaced large coil section can have a length of 40 mm (or about 40 mm) to 60 mm (or about 60 mm) or greater.

As mentioned, the metal layer **404** (either coil or hypotube) can be covered by an outer polymer layer or jacket **402.** In some embodiments, the outer polymer **402** layer is an elastomer, though the particular material is not limiting. In some embodiments, the outer polymer layer **402** can comprise polytetrafluoroethylene (PTFE) or expanded polytetrafluoroethylene (cPTFE). The ePTFE can have very different mechanical properties that PTFE. For example, ePTFE can be much more flexible while still maintaining good tensile/elongation properties. In some embodiments, the outer polymer layer **402** can comprise a thermoplastic elastomer, such as PEBAX^{®}. In some embodiments, the outer polymer layer **402** can be pre-axially stressed before applying to the capsule. The outer polymer layer **402** can be approximately 0.006 to 0.008 inches in thickness, but the particular thickness is not limiting.

The outer polymer layer **402** can be applied to the metal layer **404** to form an outer jacket, such as by reflowing the polymer. In some embodiments, the outer polymer layer **402** can be directly applied to the metal layer **404.** In some embodiments, an adhesive layer **406** can be disposed between the metal layer **404** and the outer polymer layer **402** to promote attachment of the outer polymer layer to the metal layer. For example, a fluoropolymer, or other soft durometer fluoroelastomer, can be applied between the metal layer **404** and the outer layer **402** in order to attach the two layers together and prevent delamination. In some embodiments, the adhesive layer **406** is not used.

In some implementations, other materials can be included between the metal layer **404** and the outer polymer layer **402** in order to improve properties. For example, fluorinated ethylene propylene (FEP) sections **408** can improve radial strength, in particular when the implant is under compression. While an FEP layer **408** is discussed as a particular material, other high strength polymers, metals, or ceramics can be used as well, and the particular material is not limiting. The FEP layer **408** can also act as an adhesive in some instances.

FEP sections **408** can be included at the distal and proximal ends of the capsule **106.** The FEP sections **408** can either overlap the adhesive layer **406.** Thus, FEP sections **408** can be located between the adhesive layer **406** and the metal layer **404** or between the adhesive layer **406** and the outer polymer layer **402.** In some embodiments, the FEP sections **408** may be located in sections of the capsule **106** that do not include an adhesive layer **406.**

The FEP section **408** located at the distal end of the capsule **106** can have a length of 10 mm (or about 10 mm), thought he particular length is not limiting. In some embodiments, the FEP section **408** is approximately 0.003 inches in thickness, but the thickness may vary and is not limited by this disclosure. In some embodiments, different FEP sections **408** (e.g., a proximal section and a distal section) can have different thicknesses. In some embodiments, all FEP **408** layers have the same thickness. Example thicknesses can be 0.006 inches or 0.003 inches.

Moving to the inside of the metal layer **404,** a liner **410** can be included on its radially inner surface. The liner **410** can be formed of a low friction and/or high lubricity material that allows for the capsule **106** to be translated over the prosthesis **70** without catching or damaging portions of the prosthesis **70.** In some embodiments, the liner **410** can be PTFE, which can resist radial expansion and decrease friction with the prosthesis **70.**

In some embodiments, the liner **410** is made from ePTFE. However, it can be difficult to reflow a standard ePTFE liner **410** on the inner layer of the capsule **106.** Accordingly, the ePTFE liner layer **410** can be pre-compressed before applying onto the inner layer of the capsule **106.** In some embodiments, portions of the outer polymer layer **402** and the liner **410** can be in contact with one another. Thus, prior to bonding the two layers together, the ePTFE liner **410** and/or outer polymer layer **402** can be axially compressed. Then, the layers can be bonded together with reflow techniques during manufacturing. For example, the ePTFE liner **410** can be axially compressed, such as over a mandrel, while the outer polymer layer **402** can be placed over it. These two layers can then be reflowed (e.g., melting under pressure) to connect. The combined layers can be slid into and/or around the metal layer **404** discussed herein, and can be melted under pressure again to form the final capsule **106.** This technique can allow for the capsule **106** to maintain flexibility and prevent breakage/tearing.

As mentioned above, the inner liner **410** can be ePTFE in some embodiments. The surface friction of ePTFE can be about 15% less than standard PTFE, and can be about 40% less than standard extruded thermoplastics that are used in the art.

In certain embodiments, the liner layer **410** can extend only along an inner surface of the capsule **106** and terminate at a distal end. However, to prevent delamination during loading of the implant **70,** the liner **410** may not be flush at the distal end of the capsule **106.** Instead, the liner **410** can be extended and inverted at the distal end in order to cover the distal end of the capsule **106** as well as an outer diameter of a portion of the outer polymer layer **402.** This can create a seamless rolled reinforced tip of the liner **410.** This solution is advantageous over previously known methods, such as disclosed in U.S. Pat. No. 6,622,367, incorporated by reference in its entirety, as PTFE lined applications do not adhere particularly well to reinforcements or the outer jacket. By inverting the liner **410** and fusing it with the outer polymer layer **402,** this creates a seamless reinforced capsule tip that can mitigate delamination. Delamination is a serious concern because the delaminated liner can tear and embolize during deployment, and the delaminated layer can cause extremely high loading and deployment forces. Delaminated layers can also cause lumen translation problems by locking up shafts thereby adding translational force requirements.

In some embodiments, another FEP section **412** can be included between the liner **410** and the metal layer **404.** The FEP section **412** can be located on distal metal coil **108,** as well as the tube **110** transitioning between the distal metal coil **108** and the proximal metal coil **107.** In some embodiments, the FEP section **412** may continue partially or fully into the proximal metal coil **107.**

In some embodiments, an FEP section **412** can be included in the proximalmost portion of the proximal metal coil **107.** This FEP section **412** be approximately 0.5 inches in length. In some embodiments, there is a longitudinal gap between the proximalmost FEP section **412** and the FEP section **412** that extends over the distal metal coil **108.** In some embodiments, the previously mentioned FEP sections **412** are continuous.

As shown in **Figure 15****,** the metal layer **404** may stop proximal to the edges of the outer polymer layer **402,** liner **410,** and FEP section **412.** If so, a thicker portion of an adhesive layer **409** can be applied at the distal end of the metal layer 404 to match the distal end of the other layers. However, this section can be removed during manufacture, so the distal end of the metal layer **404** is the distal end of the capsule **106,** which can then be covered by the liner **410.** In some embodiments, the extended sections distal to the metal layer **404** are not used.

### Handle

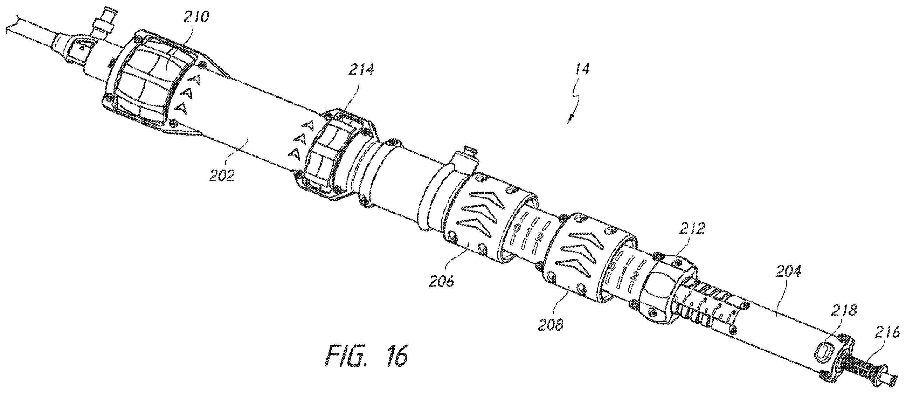

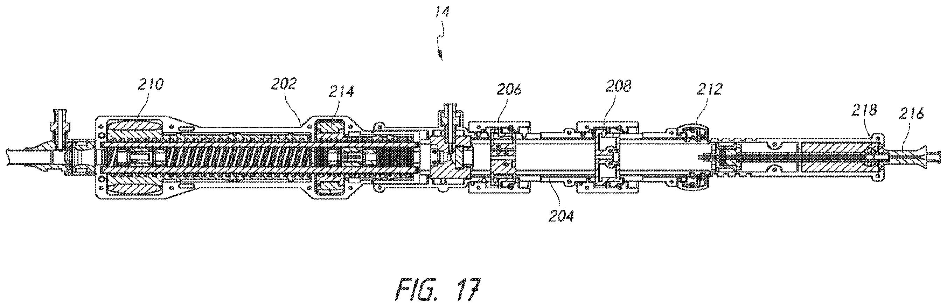

The handle **14** is located at the proximal end of the delivery system **10** and is shown in **Figure 16****.** A cross-section of the handle **14** is shown in **Figure 17****.** The handle **14** can include a number of actuators, such as rotatable knobs, that can manipulate different components of the delivery system **10.** The operation of the handle **10** is described with reference to delivery of a replacement mitral valve prosthesis **70,** though the handle **10** and delivery system **10** can be used to deliver other devices as well.

The handle **14** is generally composed of two housings, a rail housing **202** and a delivery housing **204,** the rail housing **202** being circumferentially disposed around the delivery housing **204.** The inner surface of the rail housing **202** can include a screwable section configured to mate with an outer surface of the delivery housing **204.** Thus, the delivery housing **204** is configured to slide (e.g., screw) within the rail housing **202,** as detailed below. The rail housing **202** generally surrounds about one half the length of the delivery housing **204,** and thus the delivery housing **204** extends both proximally and distally outside of the rail housing **202.**

The rail housing **202** can contain two rotatable knobs, a distal pull wire knob **206** and a proximal pull wire knob **208.** However, the number of rotatable knobs on the rail housing **202** can vary depending on the number of pull wires used. Rotation of the distal pull wire knob **206** can provide a proximal force, thereby providing axial tension on the distal pull wires **138** and causing the distal slotted section **135** of the rail hypotube **136** to bend. The distal pull wire knob **206** can be rotated in either direction, allowing for bending in either direction, which can control anterior-posterior angles. Rotation of the proximal pull wire knob **208** can provide a proximal force, and thus axial tension, on the proximal pull wires **140,** thereby causing the proximal slotted section **133** of the rail hypotube **136** to bend, which can control the medial-lateral angle. The proximal pull wire knob **108** can be rotated in either direction, allowing for bending in either direction. Thus, when both knobs are actuated, there can be two bends in the rail hypotube **136,** thereby allowing for three-dimensional steering of the rail shaft **132,** and thus the distal end of the delivery system **10.** Further, the proximal end of the rail shaft **132** is connected on an internal surface of the rail housing **202.**

The bending of the rail shaft **132** can be used to position the system, in particular the distal end, at the desired patient location, such as at the native mitral valve. In some embodiments, rotation of the pull wire knobs **206/208** can help steer the distal end of the delivery system **10** through the septum and left atrium and into the left ventricle so that the prosthesis **70** is located at the native mitral valve.

Moving to the delivery housing **204,** the proximal ends of the inner shaft assembly **19,** outer sheath assembly **22,** mid shaft assembly **21,** and nose cone shaft assembly **30** can be connected to an inner surface of the delivery housing **204** of the handle **14.** Thus, they can move axially relative to the rail assembly **20** and rail housing **202.**

A rotatable outer sheath knob **210** can be located on the distal end of the delivery housing **204,** being distal to the rail housing **202.** Rotation of the outer sheath knob **210** will pull the outer sheath assembly **22** in an axial direction proximally, thus pulling the capsule **106** away from the implant **70** and releasing the distal end **301** of implant **70.** Thus the outer sheath assembly **22** is individually translated with respect to the other shafts in the delivery system **10.** The distal end **303** of the implant **70** can be released first, while the proximal end **301** of the implant **70** can remain radially compressed between the inner retention member **40** and the outer retention member **42.**

A rotatable mid shaft knob **214** can be located on the delivery housing **204,** in some embodiments proximal to the rotatable outer sheath knob **210,** being distal to the rail housing **202.** Rotation of the mid shaft knob **214** will pull the mid shaft assembly **21** in an axial direction proximally, thus pulling the outer retention ring **42** away from the implant **70** and uncovering the inner retention member **40** and the proximal end **301** of the implant **70,** thereby releasing the implant **70.** Thus, the mid shaft assembly **21** is individually translated with respect to the other shafts in the delivery system **10.**

Located on the proximal end of the delivery housing **204,** and thus proximal to the rail housing **202,** can be a rotatable depth knob **212.** As the depth knob **212** is rotated, the entirety of the delivery housing **204** moves distally or proximally with respect to the rail housing **202** which will remain in the same location. Thus, at the distal end of the delivery system **10,** the inner shaft assembly **18,** outer sheath assembly **22,** mid shaft assembly **21,** and nose cone shaft assembly **31** together (e.g., simultaneously) move proximally or distally with respect to the rail assembly **20** while the implant **70** remains in the compressed configuration. In some embodiments, actuation of the depth knob **212** can sequentially move the inner shaft assembly **18,** outer sheath assembly **22,** mid shaft assembly **21,** and nose cone shaft assembly **31** relative to the rail assembly **20.** In some embodiments, actuation of the depth knob **212** can together move the inner shaft assembly **18,** outer sheath assembly **22,** and mid shaft assembly **21** relative to the rail assembly **20.** Accordingly, the rail shaft **132** can be aligned at a particular direction, and the other assemblies can move distally or proximally with respect to the rail shaft **132** for final positioning while not releasing the implant **70.** The components can be advanced approximately 1, 2, 3, 5, 6, 7, 8, 9, or 10 cm along the rail shaft **132.** The components can be advanced more than approximately 1, 2, 3, 5, 6, 7, 8, 9, or 10 cm along the rail shaft **132.** An example of this is shown in **Figure 2C****.** The capsule **106** and outer retention ring **42** can then be individually withdrawn with respect to the inner assembly **18** as discussed above, in some embodiments sequentially, releasing the implant **70.** The assemblies other than the rail assembly **20** can then be withdrawn back over the rail shaft **132** by rotating the depth knob **212** in the opposite direction.

The handle **14** can further include a mechanism (knob, button, handle) **216** for moving the nose cone shaft **27,** and thus the nose cone **28.** For example, a knob **216** can be a portion of the nose cone assembly **31** that extends from a proximal end of the handle **14.** Thus, a user can pull or push on the knob **216** to translate the nose cone shaft **27** distally or proximally individually with respect to the other shafts. This can be advantageous for proximally translating the nose cone **28** into the outer sheath assembly **22** /capsule **106,** thus facilitating withdraw of the delivery system **10** from the patient.

In some embodiments, the handle **14** can provide a lock **218,** such as a spring lock, for preventing translation of the nose cone shaft **27** by the knob **216** discussed above. In some embodiments, the lock **218** can be always active, and thus the nose cone shaft **27** will not move without a user disengaging the lock **218.** The lock can be, for example, a spring lock that is always engaged until a button **218** on the handle **14** is pressed, thereby releasing the spring lock and allowing the nose cone shaft **27** to translate proximally/distally. In some embodiments, the spring lock **218** allows one-way motion, either proximal or distal motion, of the nose cone shaft **27** but prevents motion in the opposite direction.

The handle **14** can further include a communicative flush port for flushing out different lumens of the delivery system **10.** In some embodiments, a single flush port on the handle **14** can provide fluid connection to multiple assemblies. In some embodiments, the flush port can provide fluid connection to the outer sheath assembly **22.** In some embodiments, the flush port can provide fluid connection to the outer sheath assembly **22** and the mid shaft assembly **21.** In some embodiments, the flush port can provide fluid connection to the outer sheath assembly **22,** the mid shaft assembly **21,** and the rail assembly **20.** In some embodiments, the flush port can provide fluid connection to the outer sheath assembly **22,** the mid shaft assembly **21,** the rail assembly **20,** and the inner assembly **18.** Thus, in some embodiments, the rail shaft **132,** the outer retention ring **42,** and the capsule **406** can all be flushed by a single flush port.

### Valve Delivery Positioning

Methods of using the delivery system **10** in connection with a replacement mitral valve will now be described. In particular, the delivery system **10** can be used in a method for percutaneous delivery of a replacement mitral valve to treat patients with moderate to severe mitral regurgitation. The below methods are merely examples of the how the delivery system may be used. It will be understood that the delivery systems described herein can be used as part of other methods as well.

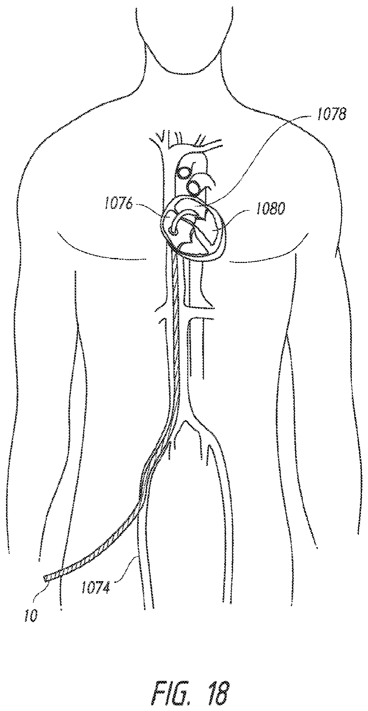

As shown in **Figure 18****,** in one embodiment the delivery system **10** can be placed in the ipsilateral femoral vein **1074** and advanced toward the right atrium **1076.** A transseptal puncture using known techniques can then be performed to obtain access to the left atrium **1078.** The delivery system **10** can then be advanced in to the left atrium **1078** and then to the left ventricle **1080.** **Figure 18** shows the delivery system **10** extending from the ipsilateral femoral vein **1074** to the left atrium **1078.** In embodiments of the disclosure, a guide wire is not necessary to position the delivery system **10** in the proper position, although in other embodiments, one or more guide wires may be used.

Accordingly, it can be advantageous for a user to be able to steer the delivery system **10** through the complex areas of the heart in order to position a replacement mitral valve in line with the native mitral valve. This task can be performed with or without the use of a guide wire with the above disclosed system. The distal end of the delivery system can be advanced into the left atrium 1078. A user can then manipulate the rail assembly **20** to target the distal end of the delivery system **10** to the appropriate area. A user can then continue to pass the bent delivery system **10** through the transseptal puncture and into the left atrium **1078.** A user can then further manipulate the delivery system **10** to create an even greater bend in the rail assembly **20.** Further, a user can torque the entire delivery system **10** to further manipulate and control the position of the delivery system **10.** In the fully bent configuration, a user can then place the replacement mitral valve in the proper location. This can advantageously allow delivery of a replacement valve to an in-situ implantation site, such as a native mitral valve, via a wider variety of approaches, such as a transseptal approach.

The rail assembly **20** can be particularly advantageous for entering into the native mitral valve. As discussed above, the rail assembly **20** can form two bends, both of which can be located in the left atrium **1078.** The bends in the rail assembly **20** can position the prosthesis **70,** located in the implant retention area **16,** so that it is coaxial with the native mitral valve. Once the prosthesis **70** is coaxial, the outer sheath assembly **22,** mid shaft assembly **21,** inner assembly **18,** and nose cone assembly **31** can together be advanced (e.g., using the depth knob **212** of the handle **14)** distally relative to the rail assembly **20.** These assemblies advance straight off of the rail assembly **20,** thus advancing them coaxial with the native mitral valve until the prosthesis **70** is to be released while maintain the prosthesis **70** in the compressed configuration, as discussed below. Thus, the rail assembly **20** provides the ability for a user to lock the angular position in place, so that the user then has to just longitudinally advance the other assemblies over the rail assembly **20** while not needed to make any angular changes, greatly simplifying the procedure. The rail assembly **20** acts as an independent steering assembly, where all the assembly does is provide steerability and no further prosthesis release functionality. Further, the construction of the rail assembly **20** as described above is sufficiently rigid so that when the rail assembly is actuated to its bent shape, movement of the other components, e.g., the outer sheath assembly **22,** mid shaft assembly **21,** inner assembly **18,** and/or nose cone assembly **31,** the rail assembly **20** maintains its shape. Thus, the rail assembly **20** can remain in the desired bent position during the sliding of the other assemblies relative to the rail assembly **20,** and the rail assembly **20** can help direct the other assemblies to the final position. The proximal/distal translation of the other assemblies over the rail assembly **20** allows for ventricular-atrial motion. In addition, once the distal anchors **80** of the prosthesis **70** have been released in the left ventricle **1080,** but prior to full release, the other assemblies can be proximally retracted over the rail assembly **20** to capture any leaflets or chordae.

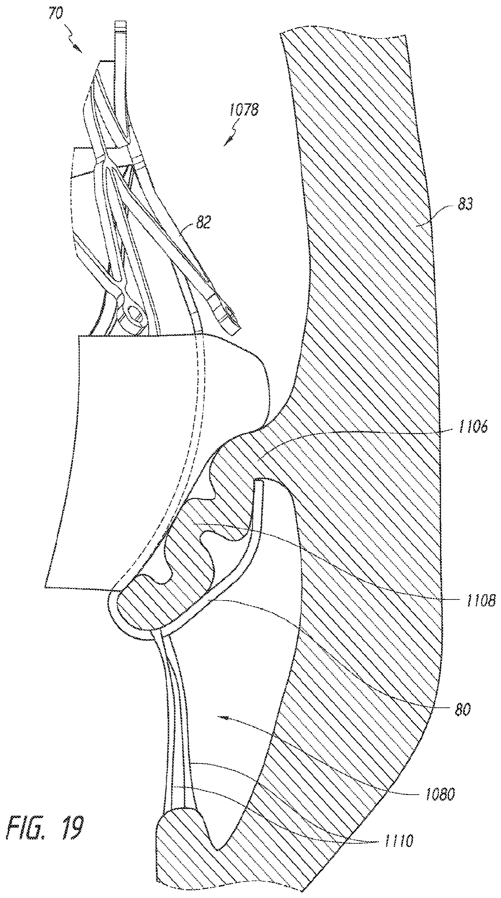

Reference is now made to **Figure 19** which illustrates a schematic representation of a portion of an embodiment of a replacement heart valve (prosthesis **70)** positioned within a native mitral valve of a heart **83.** Further details regarding how the prosthesis **70** may be positioned at the native mitral valve are described in U.S. Publication No. 2015/0328000A1, the entirety of which is hereby incorporated by reference, including but not limited to Figures 13A-15 and paragraphs [0036]-[0045]. A portion of the native mitral valve is shown schematically and represents typical anatomy, including a left atrium **1078** positioned above an annulus **1106** and a left ventricle **1080** positioned below the annulus **1106.** The left atrium **1078** and left ventricle **1080** communicate with one another through a mitral annulus **1106.** Also shown schematically in **Figure 19** is a native mitral leaflet **1108** having chordae tendineae **1110** that connect a downstream end of the mitral leaflet **1108** to the papillary muscle of the left ventricle **1080.** The portion of the prosthesis **70** disposed upstream of the annulus **1106** (toward the left atrium **1078)** can be referred to as being positioned supra-annularly. The portion generally within the annulus **1106** is referred to as positioned intra-annularly. The portion downstream of the annulus **1106** is referred to as being positioned sub-annularly (toward the left ventricle **1080).**

As shown in **Figure 19****,** the replacement heart valve (e.g., prosthesis **70)** can be positioned so that the mitral annulus **1106** is located the distal anchors **80** and the proximal anchors **82.** In some situations, the prosthesis **70** can be positioned such that ends or tips of the distal anchors **80** contact the annulus **1106** as shown, for example, in **Figure 19****. In** some situations, the prosthesis **70** can be positioned such that ends or tips of the distal anchors **80** do not contact the annulus **1106.** In some situations, the prosthesis **70** can be positioned such that the distal anchors **80** do not extend around the leaflet **1108.**

As illustrated in **Figure 19****,** the replacement heart valve **70** can be positioned so that the ends or tips of the distal anchors **80** are on a ventricular side of the mitral annulus **1106** and the ends or tips of the proximal anchors **82** are on an atrial side of the mitral annulus **1106.** The distal anchors **80** can be positioned such that the ends or tips of the distal anchors **80** are on a ventricular side of the native leaflets beyond a location where chordae tendineae **1110** connect to free ends of the native leaflets. The distal anchors **80** may extend between at least some of the chordae tendineae **1110** and, in some situations such as those shown in **Figure 19****,** can contact or engage a ventricular side of the annulus **1106.** It is also contemplated that in some situations, the distal anchors **80** may not contact the annulus **1106,** though the distal anchors **80** may still contact the native leaflet **1108.** In some situations, the distal anchors **80** can contact tissue of the left ventricle **104** beyond the annulus **1106** and/or a ventricular side of the leaflets.

During delivery, the distal anchors **80** (along with the frame) can be moved toward the ventricular side of the annulus **1106,** such as by translating the other assemblies (e.g., outer sheath assembly **22,** mid shaft assembly **21,** inner assembly **18,** and nose cone assembly **31)** proximally with respect to the rail assembly **20,** with the distal anchors **80** extending between at least some of the chordae tendineae **1110** to provide tension on the chordae tendineae **1110.** The degree of tension provided on the chordae tendineae **1110** can differ. For example, little to no tension may be present in the chordae tendineae **1110** where the leaflet **1108** is shorter than or similar in size to the distal anchors **80.** A greater degree of tension may be present in the chordae tendineae **1110** where the leaflet **1108** is longer than the distal anchors **80** and, as such, takes on a compacted form and is pulled proximally. An even greater degree of tension may be present in the chordae tendineae **1110** where the leaflets **1108** are even longer relative to the distal anchors **80.** The leaflet **1108** can be sufficiently long such that the distal anchors **80** do not contact the annulus **1106.**

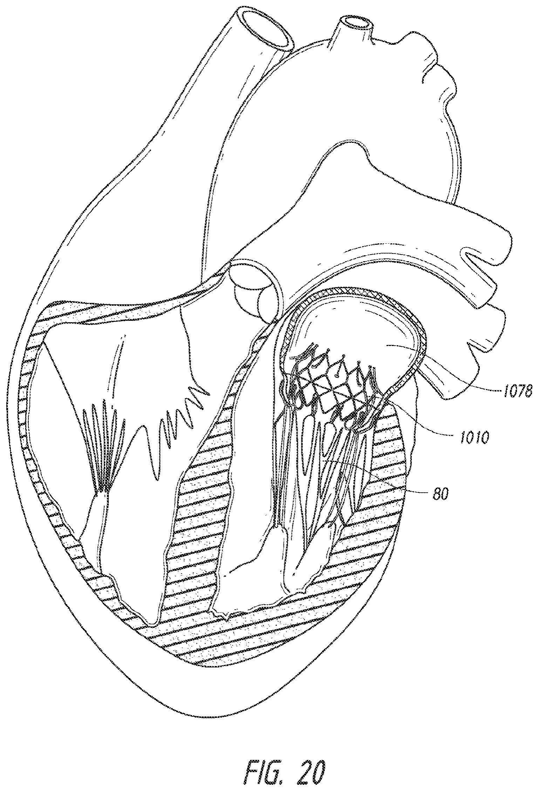

The proximal anchors **82,** if present, can be positioned such that the ends or tips of the proximal anchors **82** are adjacent the atrial side of the annulus **1106** and/or tissue of the left atrium **1078** beyond the annulus **1106.** In some situations, some or all of the proximal anchors **82** may only occasionally contact or engage atrial side of the annulus **1106** and/or tissue of the left atrium **1078** beyond the annulus **1106.** For example, as illustrate in **Figure 19****,** the proximal anchors **82** may be spaced from the atrial side of the annulus **1106** and/or tissue of the left atrium **1078** beyond the annulus **1106.** The proximal anchors **82** could provide axial stability for the prosthesis **70.** It is also contemplated that some or all of the proximal anchors **82** may contact the atrial side of the annulus **1106** and/or tissue of the left atrium **1078** beyond the annulus **1106.** **Figure 20** illustrates the prosthesis **70** implanted in the heart. Although the illustrated replacement heart valve includes both proximal and distal anchors, it will be appreciated that proximal and distal anchors are not required in all cases. For example, a replacement heart valve with only distal anchors may be capable of securely maintaining the replacement heart valve in the annulus. This is because the largest forces on the replacement heart valve are directed toward the left atrium during systole. As such, the distal anchors are most important for anchoring the replacement heart valve in the annulus and preventing migration.

### Delivery Method

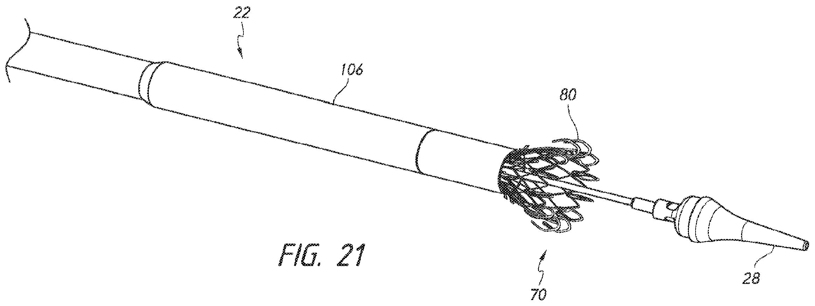

**Figures 21-23** illustrate the release mechanism of the delivery system **10.** During the initial insertion of the prosthesis **70** and the delivery system **10** into the body, the prosthesis **70** can be located within the system **10,** similar to as shown in **Figure 2A****.** The distal end **303** of the prosthesis **70,** and specifically the distal anchors **80,** are restrained within the capsule **106** of the outer sheath assembly **22,** thus preventing expansion of the prosthesis **70.** Similar to what is shown in **Figure 2A****,** the distal anchors **80** can extend distally when positioned in the capsule. The proximal end **301** of the prosthesis **70** is restrained within the capsule **106** and within a portion of the inner retention member **40** and thus is generally constrained between the capsule **106** and the inner retention member **40.**

The system **10** can first be positioned to a particular location in a patient's body, such as at the native mitral valve, through the use of the steering mechanisms discussed herein or other techniques.

Once the prosthesis **70** is loaded into the delivery system **10,** a user can thread a guide wire into a patient to the desired location. The guide wire passes through the lumen of the nose cone assembly **31,** and thus the delivery system **10** can be generally advanced through the patient's body following the guide wire. The delivery system **10** can be advanced by the user manually moving the handle **14** in an axial direction. In some embodiments, the delivery system **10** can be placed into a stand while operating the handle **14** controls.

Once generally in heart, the user can begin the steering operation of the rail assembly **20** using the distal pull wire knob **206** and/or the proximal pull wire knob **208.** By turning either of the knobs, the user can provide flexing/bending of the rail assembly **20** (either on the distal end or the proximal end), thus bending the distal end of the delivery system **10** in one, two, or more locations into the desired configuration. As discussed above, the user can provide multiple bends in the rail assembly **20** to direct the delivery system **10** towards the mitral valve. In particular, the bends of the rail assembly **20** can direct a distal end of the delivery system **10,** and thus the capsule **106,** along the center axis passing through the native mitral valve. Thus, when the outer sheath assembly **22,** mid shaft assembly **21,** inner assembly **18,** and nose cone assembly **31** are together advanced over the rail assembly **20** with the compressed prosthesis **70,** the capsule **106** proceed directly in line with the axis for proper release of the prosthesis **70.**

The user can also rotate and/or move the handle **14** itself in a stand for further fine tuning of the distal end of the delivery system **10.** The user can continually turn the proximal and/or distal pull wire knobs **208/206,** as well as moving the handle **14** itself, to orient the delivery system **10** for release of the prosthesis **70** in the body. The user can also further move the other assemblies relative to the rail assembly **20,** such as proximally or distally.

In a next step, the user can rotate the depth knob **212.** As discussed, rotation of this knob **212** together advances the inner shaft assembly **18,** mid shaft assembly **21,** outer sheath assembly **22,** and nose cone assembly **31** over/through the rail assembly **20** while the prosthesis **70** remains in the compressed configuration within the implant retention area **16.** Due to the rigidity of, for example, either the inner shaft assembly **18,** the mid shaft assembly **21,** and/or the outer sheath assembly **22,** these assemblies proceed straight forward in the direction aligned by the rail assembly **20.**

Once in the release position, the user can rotate the outer sheath knob **210,** which individually translates the outer sheath assembly **22** (and thus the capsule **106)** with respect to the other assemblies, in particular the inner assembly **18,** in a proximal direction towards the handle **14** as shown in **Figure 21****.** By doing so, the distal end **303** of prosthesis **70** is uncovered in the body, allowing for the beginning of expansion. At this point, the distal anchors **80** can flip proximally and the distal end **303** begins to expand radially outwardly. For example, if the system **10** has been delivered to a native mitral valve location through a transseptal approach, the nose cone is positioned in the left ventricle, preferably aligning the prosthesis **70** such that it is generally perpendicular to the plane of the mitral annulus. The distal anchors **80** expand radially outwardly within the left ventricle. The distal anchors **80** can be located above the papillary heads, but below the mitral annulus and mitral leaflets. In some embodiments, the distal anchors **80** may contact and/or extend between the chordac in the left ventricle, as well as contact the leaflets, as they expand radially. In some embodiments, the distal anchors **80** may not contact and/or extend between the chordae or contact the leaflets. Depending on the position of the prosthesis **70,** the distal ends of the distal anchors **80** may be at or below where the chordae connect to the free edge of the native leaflets.

As shown in the illustrated embodiment, the distal end **303** of the prosthesis **70** is expanded outwardly. It should be noted that the proximal end **301** of the prosthesis **70** can remain covered by the outer retention ring during this step such that the proximal end **301** remains in a radially compacted state. At this time, the system **10** may be withdrawn proximally so that the distal anchors **80** capture and engage the leaflets of the mitral valve, or may be moved proximally to reposition the prosthesis **70.** For example, the assemblies may be proximally moved relative to the rail assembly **20.** Further, the system **10** may be torqued, which may cause the distal anchors **80** to put tension on the chordae through which at least some of the distal anchors may extend between. However, in some embodiments the distal anchors **80** may not put tension on the chordae. In some embodiments, the distal anchors **80** may capture the native leaflet and be between the chordae without any further movement of the system **10** after withdrawing the outer sheath assembly **22.**

During this step, the system **10** may be moved proximally or distally to cause the distal or ventricular anchors **80** to properly capture the native mitral valve leaflets. This can be done by moving the outer sheath assembly **22,** mid shaft assembly **21,** inner assembly **18,** and nose cone assembly **31** with respect to the rail assembly **20.** In particular, the tips of the ventricular anchors **80** may be moved proximally to engage a ventricular side of the native annulus, so that the native leaflets are positioned between the anchors **80** and the body of the prosthesis **70.** When the prosthesis **70** is in its final position, there may or may not be tension on the chordae, though the distal anchors **80** can be located between at least some of the chordae.

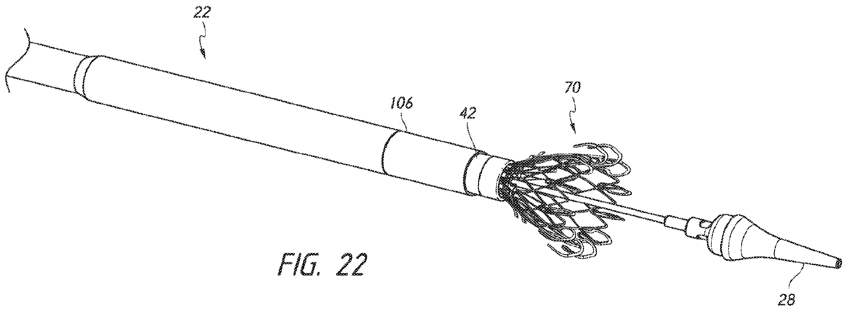

The proximal end **301** of the prosthesis **70** will remain in the outer retention ring **42** after retraction of the capsule **106.** As shown in **Figure 22****,** once the distal end **303** of the prosthesis **70** is fully expanded (or as fully expanded as possible at this point), the outer retention ring **42** can be individually withdrawn proximally with respect to the other assemblies, in particular relative to the inner assembly **18,** to expose the inner retention member **40,** thus beginning the expansion of the proximal end **301** of the prosthesis **70.** For example, in a mitral valve replacement procedure, after the distal or ventricular anchors **80** are positioned between at least some of the chordae tendincac and/or engage the native mitral valve annulus, the proximal end **301** of the prosthesis **70** may be expanded within the left atrium.

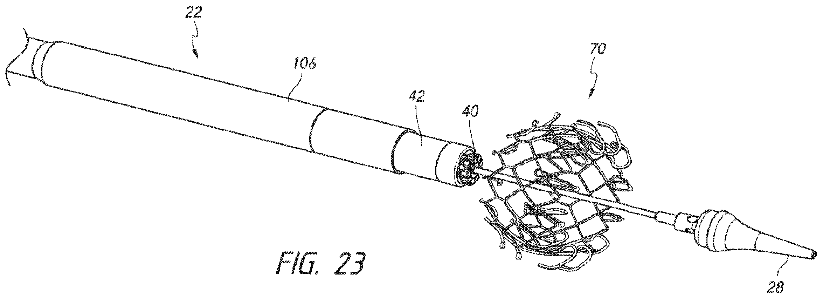

The outer retention ring **42** can be moved proximally such that the proximal end **310** of the prosthesis **70** can radially expand to its fully expanded configuration as shown in **Figure 23****.** After expansion and release of the prosthesis **70,** the inner assembly **18,** nose cone assembly **31,** mid shaft assembly **21,** and outer sheath assembly **22** can be simultaneously withdrawn proximally along or relative to the rail assembly **20** back to their original position. In some embodiments, they are not withdrawn relative to the rail assembly **20** and remain in the extended position. Further, the nose cone **28** can be withdrawn through the center of the expanded prosthesis **70** and into the outer sheath assembly **22,** such as by proximally translating the knob **216.** The system **10** can then be removed from the patient.

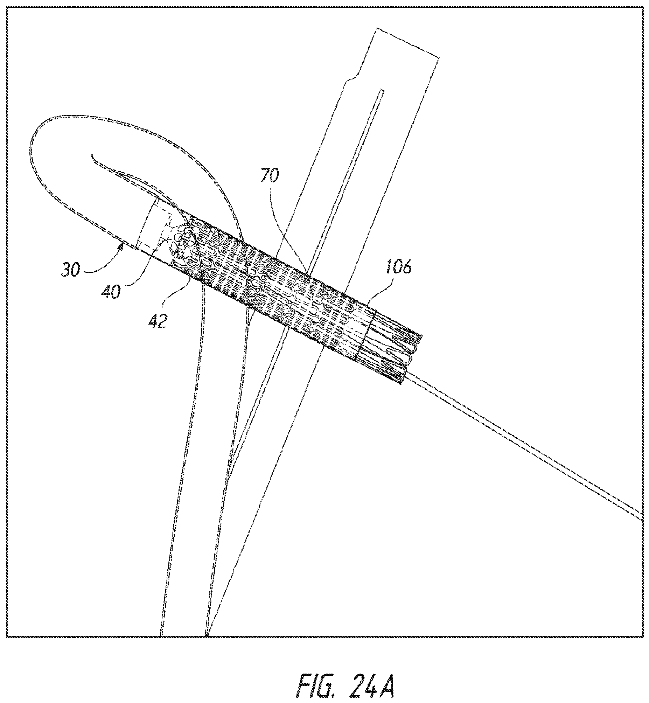

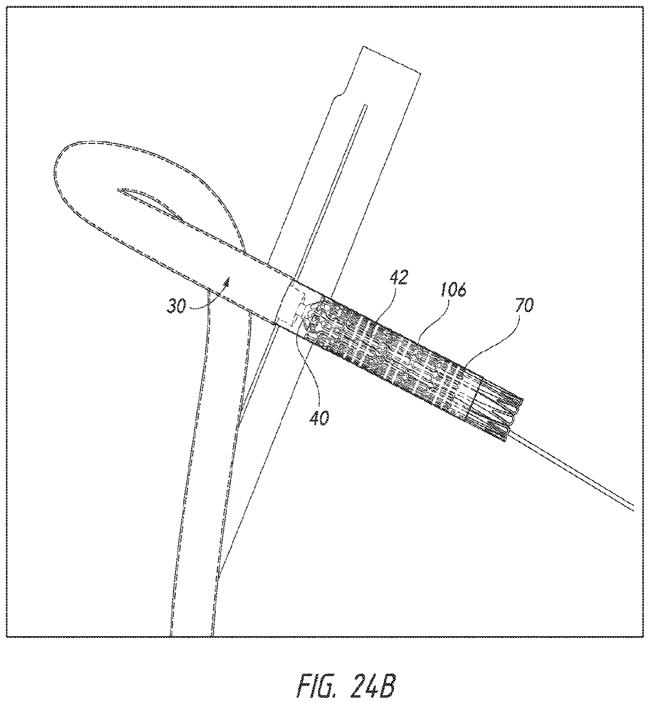

**Figures 24A-B** illustrate the advancement of the different assemblies over the rail assembly **20.** **Figure 24A** illustrates the assemblies in their proximalmost position over the rail assembly **20.** **Figure 24B** illustrates the assemblies in their distalmost position as compared to the rail assembly **20,** such as shown in **Figure 2C****.** Thus, the assemblies snake along the rail assembly **20** and extend distally away.

In some embodiments, the prosthesis **70** can be delivered under fluoroscopy so that a user can view certain reference points for proper positioning of the prosthesis **70.** Further, echocardiography can be used for proper positioning of the prosthesis **70.**

Following is a discussion of an alternative implantation method for delivering a replacement mitral valve to a mitral valve location. Elements of the below can be incorporated into the above discussion and vice versa. Prior to insertion of the delivery system **10,** the access site into the patient can be dilated. Further, a dilator can be flushed with, for example, heparinized saline prior to use. The delivery system **10** can then be inserted over a guide wire. In some embodiments, any flush ports on the delivery system **10** can be pointed vertically. Further, if an introducer tube is used, integrated or otherwise, this can be stabilized. The delivery system **10** can be advanced through the septum until a distal end of the delivery system **10** is positioned across the septum into the left atrium **1078.** Thus, the distal end of the delivery system **10** can be located in the left atrium **1078.** In some embodiments, the delivery system **10** can be rotated, such as under fluoroscopy, into a desired position. The rail can be flex so that direct a distal end of the delivery system **10** towards the septum and mitral valve. The position of the delivery system **10,** and the prosthesis **70** inside, can be verified using echocardiography and fluoroscopic guidance.

In some embodiments, the prosthesis **70** can be located, prior to release, above the mitral annulus **1106,** in line with the mitral annulus **1106,** or below the mitral annulus **1106.** In some embodiments, the prosthesis **70** can be located, prior to expansion, fully above the mitral annulus **1106,** in line with the mitral annulus **1106,** just below the mitral annulus **1106,** or fully below the mitral annulus **1106.** In some embodiments, the prosthesis **70** can be located, prior to expansion, partially above the mitral annulus **1106,** in line with the mitral annulus **1106,** or partially below the mitral annulus **1106.** In some embodiments, a pigtail catheter can be introduced into the heart to perform a ventriculogram for proper viewing.

In some embodiments, the position of the mitral plane and the height of any papillary muscles on the fluoroscopy monitor can be marked to indicate an example target landing zone. If needed, the delivery system **10** can be unflexed, reduced in rotation, and retracted to reduce tension on the delivery system **10** as well as reduce contact with the left ventricular wall, the left atrial wall, and/or the mitral annulus **1106.**

Further, the delivery system **10** can be positioned to be coaxial to the mitral annulus **1106,** or at least as much as possible, while still reducing contact with the left ventricular wall, the left atrial wall, and/or the mitral annulus **1106** and reducing delivery system tension. An echo probe can be positioned to view the anterior mitral leaflet (AML), the posterior mitral leaflet (PML) (leaflets **1108),** mitral annulus **1106,** and outflow tract. Using fluoroscopy and echo imaging, the prosthesis **1010** can be confirmed to be positioned at a particular depth and coaxiality with the mitral annulus **1106.**

Afterwards, the outer sheath assembly **22** can be retracted to expose the ventricular anchors **80,** thereby releasing them. In some embodiments, once exposed, the outer sheath assembly **22** can be reversed in direction to relieve tension on the outer sheath assembly **22.** In some embodiments, reversing the direction could also serve to partially or fully capture the prosthesis **70.**

The distal anchors **80** can be released in the left atrium **1078.** Further, the proximal anchors **82,** if included in the prosthesis **70,** are not yet exposed. Moreover, the body of the prosthesis **70** has not undergone any expansion at this point. However, in some embodiments, one or more of the distal anchors **80** can be released in either the left atrium **1078** (e.g., super-annular release) or generally aligned with the mitral valve annulus **1106** (e.g., intra-annular release), or just below the mitral valve annulus **1106** (e.g., sub-annular release). In some embodiments, all of the distal anchors **80** can be released together. In other embodiments, a subset of the distal anchors **80** can be released while at a first position and another subset of the distal anchors **80** can be released while at a second position. For example, some of the distal anchors **80** can be released in the left atrium **1078** and some of the distal anchors **80** can be released while generally aligned with the mitral valve annulus **1106** or just below the mitral valve annulus **1106.**

If the distal anchors **80** are released "just below" the mitral valve annulus **1106,** the may be released at 1 inch, 3/4 inch, 1/2 inch, 1/4 inch, 1/8 inch, 1/10 inch or 1/20 inch below the mitral valve annulus **1106.** In some embodiments, the distal anchors **80** the may be released at less than 1 inch, 3/4 inch, 1/2 inch, 1/4 inch, 1/8 inch, 1110 inch or 1/20 inch below the mitral valve annulus **1106.** This may allow the distal anchors **80** to snake through the chordae upon release. This can advantageously allow the prosthesis **70** to slightly contract when making the sharp turn down toward the mitral valve. In some embodiments, this may eliminate the need for a guide wire assisting to cross the mitral valve. In some embodiments, the guide wire may be withdrawn into the delivery system **10** before or following release of the distal anchors **80.**

In some embodiments, the distal anchors **80** can be released immediately after crossing the septum, and then the final trajectory of the delivery system **10** can be determined. Thus, the delivery system **10** can cross the septum, release the ventricular anchors **80,** establish a trajectory, and move into the left ventricle to capture the leaflets.

As discussed in detail above, upon release from the delivery system **10,** the distal anchors **80** can flip from extending distally to extending proximally. This flip can be approximately 180°. Accordingly, in some embodiments, the distal anchors **80** can be flipped in either the left atrium **1078** (e.g., super-annular flip), generally aligned with the mitral valve annulus **1106** (e.g., intra-annular flip), or just below the mitral valve annulus **1106** (e.g., sub-annular flip). The proximal anchors **82,** if any, can remain within the delivery system **10.** In some embodiments, all of the distal anchors **80** can be flipped together. In other embodiments, a subset of the distal anchors **80** can be flipped while at a first position and another subset of the distal anchors **80** can be released while at a second position. For example, some of the distal anchors **80** can be flipped in the left atrium **1078** and some of the distal anchors **80** can be flipped while generally aligned with the mitral valve annulus **1106** or just below the mitral valve annulus **1106.**

In some embodiments, the distal anchors **80** may be positioned in line with the annulus **1106** or just below the annulus **1106** in the non-flipped position. In some embodiments, the distal anchors **80** may be position in line with the annulus **1106** or just below the annulus **1106** in the flipped position. In some embodiments, prior to flipping the distalmost portion of the prosthesis **70** can be located within or below the mitral valve annulus **1106,** such as just below the mitral valve annulus **1106.** However, flipping the anchors can cause, without any other movement of the delivery system **10,** the distalmost portion of the prosthesis **7b**0/anchors **80** to move upwards, moving it into the left atrium **1078** or moving it in line with the mitral annulus **1106.** Thus, in some embodiments the distal anchors **80** can begin flipping at the annulus **1106** but be fully within the left atrium **1078** upon flipping. In some embodiments the distal anchors **80** can begin flipping below the annulus **1106** but be fully within the annulus **1106** upon flipping.

In some embodiments, the distal anchors **80** can be proximal (e.g., toward the left atrium **1078**) of a free edge of the mitral leaflets **1108** upon release and flipping. In some embodiments, the distal anchors **80** can be aligned with (e.g., toward the left atrium **1078**) a free edge of the mitral leaflets **1108** upon release and flipping. In some embodiments, the distal anchors **80** can be proximal (e.g., toward the left atrium **1078**) of a free edge of the mitral valve annulus **1106** upon release and flipping. In some embodiments, the distal anchors **80** can be aligned with (e.g., toward the left atrium **1078**) a free edge of the mitral valve annulus **1106** upon release and flipping.

Thus, in some embodiments the distal anchors **80** can be released/flipped above where the chordae **1110** attach to the free edge of the native leaflets **1108.** In some embodiments the distal anchors **80** can be released/flipped above where some the chordae **1110** attach to the free edge of the native leaflets **1108.** In some embodiments the distal anchors **80** can be released/flipped above where all the chordae **1110** attach to the free edge of the native leaflets **1108.** In some embodiments, the distal anchors **80** can be released/flipped above the mitral valve annulus **1106.** In some embodiments, the distal anchors **80** can be released/flipped above the mitral valve leaflets **1108.** In some embodiments, the distal anchors **80** can be released/flipped generally in line with the mitral valve annulus **1106.** In some embodiments, the distal anchors **80** can be released/flipped generally in line with the mitral valve leaflets **1108.** In some embodiments, the tips of the distal anchors **80** can be released/flipped generally in line with the mitral valve annulus **1106.** In some embodiments, the tips of the distal anchors **80** can be released/flipped generally in line with the mitral valve leaflets **1108.** In some embodiments the distal anchors **80** can be released/flipped below where some the chordac **1110** attach to the free edge of the native leaflets **1108.** In some embodiments the distal anchors **80** can be released/flipped below where all the chordae **1110** attach to the free edge of the native leaflets **1108.** In some embodiments, the distal anchors **80** can be released/flipped below the mitral valve annulus **1106.** In some embodiments, the distal anchors **1024** can be released/flipped below the mitral valve leaflets **1108.**

Once the distal anchors **80** are released and flipped, the delivery system **10** can be translated towards the left ventricle **1080** through the mitral valve annulus **1106** so that the distal anchors **80** enter the left ventricle **1080.** In some embodiments, the distal anchors **80** can compress when passing through the mitral valve annulus **1106.** In some embodiments, the prosthesis **70** can compress when passing through the mitral valve annulus **1106.** In some embodiments, the prosthesis **70** does not compress when it passes through the mitral annulus **1106.** The distal anchors **80** can be delivered anywhere in the left ventricle **1080** between the leaflets **1108** and the papillary heads.

In some embodiments, the distal anchors **80** are fully expanded prior to passing through the mitral valve annulus **1106.** In some embodiments, the distal anchors **80** are partially expanded prior to passing through the mitral valve annulus **1106** and continued operation of the delivery system **10** can fully expand the distal anchors **80** in the left ventricle **1080.**

When the distal anchors **80** enter the left ventricle **1080,** the distal anchors **80** can pass through the chordae **1110** and move behind the mitral valve leaflets **1108,** thereby capturing the leaflets **1108.** In some embodiments, the distal anchors **80** and/or other parts of the prosthesis **1010** can push the chordae **1110** and/or the mitral valve leaflets **1108** outwards.

Thus, after release of the distal anchors **80,** the delivery system **10** can then be repositioned as needed so that the ends of the left distal anchors **80** are at the same level of the free edge of the native mitral valve leaflets **1108.** The delivery system **10** can also be positioned to be coaxial to the mitral annulus **1106** if possible while still reducing contact with the left ventricular wall, the left atrial wall, and/or the annulus **1106.**

In some embodiments, only the distal anchors **80** are released in the left atrium **1078** before the prosthesis **70** is move to a position within, or below, the annulus. In some alternate embodiments, the distal end of the prosthesis **70** can be further expanded in the left atrium **1078.** Thus, instead of the distal anchors **80** flipping and no portion of the prosthesis **70** body expanding, a portion of the prosthesis **70** can be exposed and allowed to expand in the left atrium **1078.** This partially exposed prosthesis **1010** can then be passed through the annulus **1106** into the left ventricle **1080.** Further, the proximal anchors, if any, can be exposed. In some embodiments, the entirety of the prosthesis **70** can be expanded within the left atrium **1078.**

To facilitate passage through the annulus **1106,** the delivery system **10** can include a leader element (not shown) which passes through the annulus **1106** prior to the prosthesis **70** passing through the annulus **1106.** For example, the leader element can include an expandable member, such as an expandable balloon, which can help maintain the shape, or expand, the annulus **1106.** The leader element can have a tapered or rounded shape (e.g., conical, frustoconical, semispherical) to facilitate positioning through and expansion of the annulus **1106.** In some embodiments, the delivery system **10** can include an engagement element (not shown) which can apply a force on the prosthesis **70** to force the prosthesis **70** through the annulus **1106.** For example, the engagement element can include an expandable member, such as an expandable balloon, positioned within or above the prosthesis **70.**

In some embodiments, to facilitate passage through the annulus **1106,** a user can re-orient the prosthesis **70** prior to passing the prosthesis **70** through the annulus 1106. For example, a user can re-orient the prosthesis **70** such that it passes through the annulus **1106** sideways.

However, if only the distal anchors **80** are flipped, and no other expansion occurs, the prosthesis can be partially expanded in the ventricle **1080.** Thus, when the prosthesis **70** is in the proper location, the distal end can be allowed to expand to capture the leaflets **1108.** If the distal end is already expanded, no more expansion may take place or the distal end can be further expanded.

Further, the PML and AML **1106** can be captured, for example by adjusting the depth and angle of the prosthesis **70.** If a larger prosthesis diameter is needed to capture the leaflets **1106,** the outer sheath assembly **22** can be retracted until the desired diameter of the prosthesis **70**is achieved. Capture of the leaflets **1106** can be confirmed through echo imaging. In some embodiments, a user can confirm that the prosthesis **70**is still in the appropriate depth and has not advanced into the left ventricle **1080.** The position can be adjusted as needed.

In some embodiments, once the distal anchors **80** enter the left ventricle **1080** the system **10** can be pulled backwards (e.g., towards the left atrium **1078**) to fully capture the leaflets **1108.** In some embodiments, the system **10** does not need to be pulled backwards to capture the leaflets **1108.** In some embodiments, systolic pressure can push the leaflets **1108** upwards to be captured by the distal anchors **80.** In some embodiments, systolic pressure can push the entire prosthesis **70** up towards the mitral annulus **1106** after the leaflets **1108** are captured and the prosthesis **70** is fully or partially released. In some embodiments, a user can rotate the delivery system **10** and/or prosthesis **70** prior to and/or while pulling the delivery system **10** backwards. In some instances, this can beneficially engage a greater number of chordae tendineae.

The outer sheath assembly **22** can be further retracted to fully expand the prosthesis. Once the prosthesis **70** is fully exposed, the delivery system **10** can be maneuvered to be coaxial and height relative to the mitral annulus **1106,** such as by flexing, translating, or rotating the delivery system **10.** As needed, the prosthesis **70** can be repositioned to capture the free edge of the native mitral valve leaflets **1108.** Once full engagement of the leaflets **1108** is confirmed, the prosthesis **70** can be set perpendicular (or generally perpendicular) to the mitral annular plane.

Following, the mid shaft assembly **21** can be withdrawn. The mid shaft assembly **21** can then be reversed in direction to relieve any tension on the delivery system **10.**

Below is a discussion of proximal anchors **82,** though some embodiments of the prosthesis **70** may not include them. In some embodiments, proximal anchors **82** may not be released from the system **10** until the distal anchors **80** have captured the leaflets **1108.** In some embodiments, proximal anchors **82** may be released from the system **10** prior to the distal anchors **80** capturing the leaflets **1108.** In some embodiments, the proximal anchors **82** can be released when the distal anchors **80** are super or intra annular and the expanded prosthesis **70** (either partially or fully expanded) can be translated through the mitral annulus **1106.** In some embodiments, the proximal anchors **82** could be released when the distal anchors **80** are sub-annular and the entire prosthesis **70** can be pulled up into the left atrium **1078** such that the proximal anchors **82** are supra-annular prior to release. In some embodiments, the proximal anchors **82** could be intra-annular prior to release and the systolic pressure could push the prosthesis **70** atrially such that the proximal anchors **82** end up supra-annular.

After, the leaflet capture and positioning of the prosthesis **70** can be confirmed, along with the relatively perpendicular position with respect to the mitral annular plane. In some embodiments, the nosecone **28** can then be withdrawn until it is within the prosthesis **70.** The mid shaft assembly **21** can be further retracted until the prosthesis **70** is released from the delivery system **10.** Proper positioning of the prosthesis **70** can be confirmed using TEE and fluoroscopic imaging.

Following, the delivery system **10** can be centralized within the prosthesis **70.** The nosecone **28** and delivery system **10** can then be retracted into the left atrium **1078** and removed.

This intra-super annulus release can have a number of advantages. For example, this allows the distal anchors **82** to be properly aligned when contacting the chordae **1110.** If the distal anchors **82** were released in the left ventricle **1080,** this could cause misalignment or damage to heart tissue, such as the leaflets **1108** or chordae **1110.**

In an alternate delivery approach, the delivery system **10** can be translated into the left ventricle **1080** prior to release of the prosthesis **70.** Thus, the distal end of the prosthesis **70,** and thus the distal anchors **82,** can be released and flipped partially, or fully within the left ventricle **1080.** Accordingly, in some embodiments the anchors **70** can be released/flipped below the mitral annulus **1106,** just below the mitral annulus **1106,** and/or below the free edges of the leaflets **1108.** Further, the anchors **70** can be released above the papillary heads. Similar methodology as discussed above can then be used to properly position the prosthesis **70** and remove the delivery system **10** to deliver the prosthesis **1010.** Further, in some embodiments the distal anchors **82** can be released without expanding the prosthesis initially in the ventricle **1080.**

### Planetary Handle Gear

As discussed above, the delivery system **10** can include multiple shafts and one or more pull wires, some or all of which may be translated longitudinally/axially with respect to one another. However, having more movable components requires some sort of axial translation, which means that the handle **14** tends to become longer. This occurs because the different components within the handle **14** are generally moving in series, and thus there must be sufficient longitudinal space between handle components.

In order to prevent further lengthening of the handle **14,** or even to provide a reduction in size of the handle **14,** a planetary gear system can be used in the handle **14.** This planetary handle gear system can be used in conjunction with, or instead of, the current linear screws in the delivery system **10.** Embodiments of the planetary gears are shown in **Figures 25A and 25B****,** wherein the components can be located within a handle housing, such as handle **14.** By incorporating the disclosed planetary gears, rotary motion can be converted into linear motion in such a way that it allows room for multiple linear translation members to occupy the same area in the handle **14,** thereby shortening the handle design. Thus, instead of the different components being in series, they are in parallel. The disclosed planetary gear system can allow knobs or other rotatable actuating mechanisms on the handle **14** that control axial movement or bending of various shafts or lumens of the delivery system **10** to be placed much closer to one another.

In some embodiments, the handle **14** may include one or more outermost gears (e.g., ring gear **2502**), which can be connected to a rotatable knob on the handle **14** such as disclosed above. The ring gear **2502** can encompass an entire circumference or substantially an entire circumference of the handle **14** and thus be connected directly to a knob. Alternatively, the ring gear **2502** may be smaller than the handle **14** and connected to a knob by one or more intermediate components.

Further, the handle **14** can include one or more planet gears **2504** within the ring gear **2502.** The planet gears **2504** can be smaller in circumference/diameter than the ring gear **2502** and may be interlocked within an inner surface of the ring gear **2504.** The planet gear **2504** can be axially and circumferentially fixed in place, though in some embodiments it may not be. If fixed in place, the planet gear **2504** is only allowed to rotate in place by rotation of the ring gear **2504,** and thus does not translate around a circumference of the ring gear **2502.** This can prevent any components attached to the gear system from circumferentially moving around the ring gear **2504.**

Additionally, the planet gear **2504** can have an internal thread which can mate with a linear travel screw **2506,** which can be attached to any of the axially translating components (e.g., shaft assemblies such as the outer sheath assembly **22,** the mid shaft assembly **21,** the inner assembly **18,** and/or the nose cone assembly **31**) discussed above. Thus, as the ring gear **2502** is rotated by the handle **14,** the ring gear **2502** will rotate the planet gear **2504** in place, which will each in turn translate the linear travel screw **2506** in an axial direction. Thus, the rotational motion of the ring gear **2502** can be converted into linear translation of the travel screw **2506,** providing the linear translation discussed above.

As shown in Figures 25A and 25B, the planetary gear system could control multiple planet gears **2504** of the ring gear **2502.** In some embodiments, the handle **14** can contain multiple planetary gear systems, such as a 2, 3, 4, or 5 gear system. In some embodiments. each ring gear **2502** can include 1, 2, 3, 4, 5, or 6 planet gears **2504.**

### Valve Deployment Simulation Catheter

As discussed above, the delivery system **10** (or other delivery system) can include a rigid component attached to the prosthesis **70** prior to full implant deployment. For example, the prosthesis **70** generally surrounds nose cone shaft **27,** which can be a generally rigid shaft. When still partially or fully within the delivery system **10,** the prosthesis **70** may be forced into the proper position by the delivery system **10,** which may be against the natural anatomy of the valve area. However, the prosthesis **70** can jump or move inside the patient's anatomy once detached from the delivery system **10,** such as when uncovered by the outer retention ring **42** or capsule **106.** This can occur because when the prosthesis **70** is removed from the delivery system **10,** it loses the support of the delivery system **10** and moves to a non-resistant position inside the natural valve anatomy.

Thus, if the prosthesis **70** is not co-axial with the native anatomy, or perpendicular to the native annulus, when the prosthesis **70** is deployed, the prosthesis **70** loses the rigid support the delivery system **10** is exerting. If the prosthesis **70** does not capture the leaflets, then the valve can move into a position where a paravalvular leak forms around the prosthetic valve leaflets.

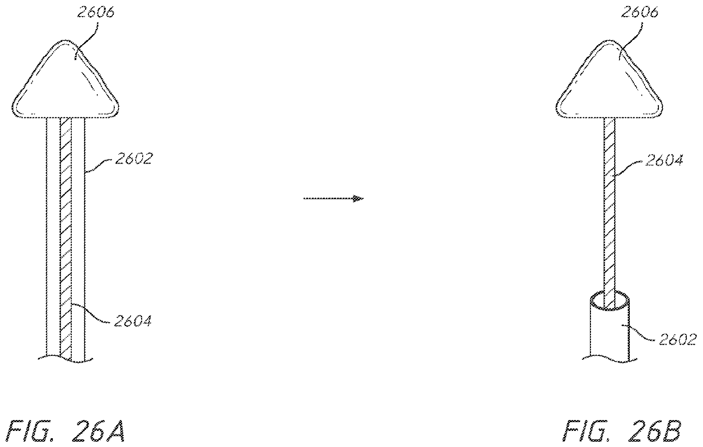

For example, as shown in **Figures 26A-26B****,** the delivery system **10** can include both a rigid shaft **2602** and a bendable (e.g., flexible) shaft **2604** for connection to the nosecone **2606.** These components can replace the nose cone shaft **27** discussed above. The rigid shaft **2602** can still be somewhat flexible but is just less bendable than the bendable shaft **2604.**

As shown, the rigid shaft **2602** can surround the bendable shaft **2604** so that the bendable shaft **2604** extends within a lumen of the rigid shaft **2602.** Thus, the rigid shaft **2602** prevents significant bending of the bendable shaft **2604.** The rigid shaft **2602** can extend all the way to the nosecone **2606,** or may extend only partially over the bendable shaft **2604.** The bendable shaft **2604** may attach to the nosecone **2606** as discussed in conjunction with the nose cone shaft **27.** The bendable shaft **2604** may be, for example, wires, cut shafts, a bendable polymer, or other materials and the particular material is not limiting.

As shown in **Figure 26A****,** while delivering the prosthesis **70,** the rigid shaft **2602** may be in a fully distal position covering the bendable shaft **2604.** Once in the desired position, the rigid shaft **2602** can be proximally withdrawn as shown in **Figure 26B** and the prosthesis **70** may be partially released from the delivery system **10.** Thus, the bendable shaft **2604** will allow the prosthesis **70** to conform with the natural patient anatomy while the prosthesis **70** is still attached to the delivery system **10.** If the positioning is not proper, an operator can cover the prosthesis **70** and/or distally translate the rigid shaft **2602** and try another position. The bendable shaft **2604** can eliminate most of the rigid support the delivery system exerts on the prosthesis **70** once uncovered by the rigid shaft **2602.**

In some embodiments, instead of using a rigid shaft **2602,** the bendable shaft **2604** can transition from a "rigid" structure to a less rigid "bendable" structure. In some embodiments, the rigid shaft **2602** may be located within a lumen of the bendable shaft **2604.**

### Locking Shaft Release

While the above-disclosed delivery system **10** includes a number of shafts and components that are fixed at their proximal end until movement of different actuators, such as at the handle **14,** it can be advantageous for the components to be locked (or fixed) instead at their distal end. In order to engage/disengage with one another, the layers can be rotated relative to one another.

Specifically, the inner retention member **40** and the outer retention member **42** can be locked together until any motion is desired. In some embodiments, the members **40/42** can be threaded together. In some embodiments, the components can have additional locking features, which an operator can unlock when the operator wants to move the outer retention member **42** axially with respect to the inner retention member **40.**

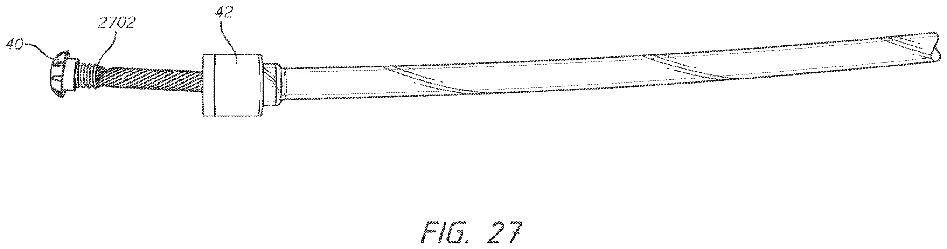

As shown in **Figure 27****,** the inner retention member **40** can include an outward (or outer or external) threading **2702** on its proximal end. Alternatively, or in conjunction with, the inner shaft **126** can include an outer threading. The outer retention member **42** can include an inner threading for attachment to the inner retention member **40.** Alternatively, or in conjunction with any of the above configurations, the mid shaft **43** may include an inner threading.

Thus, an operator can translate the prosthesis **70** within the delivery system **10** to a location while the inner retention member **40** and the outer retention member **42** are locked together (e.g., threaded together). The operator can torque or rotate either one of the inner retention member **40** and the outer retention member **42,** or both in some embodiments, in order to unlock the members **40/42** from one another. The operator can then axially withdraw the outer retention ring **42** to release the prosthesis **70.** By connecting the two components at their distal ends, the risk of unintentional release is mitigated as the members **40/42** cannot move with respect to one another when locked together.

While the inner retention member **40** and the outer retention member **42** are disclosed with the torqueing features discussed herein, other components such as the rail assembly **20,** nose cone assembly **31,** or outer sheath assembly **22** may additionally include such features to connect different shaft assemblies.

### Spine Alignment Deflection Stabilization

In some embodiments, it can be advantageous to further include additional structural elements that can be configured to reduce deflection about a flex plane of certain shafts in the delivery system **10.** This can improve the delivery system **10** under compressive loads by preventing uncontrolled deflection. For example, the delivery system **10** can inadvertently deflect in the direction of a flex plane during advancement of the delivery system **10** or when the delivery system **10** is under a compressive load, which can lead to procedural complications or accidental release of the prosthesis **70.**

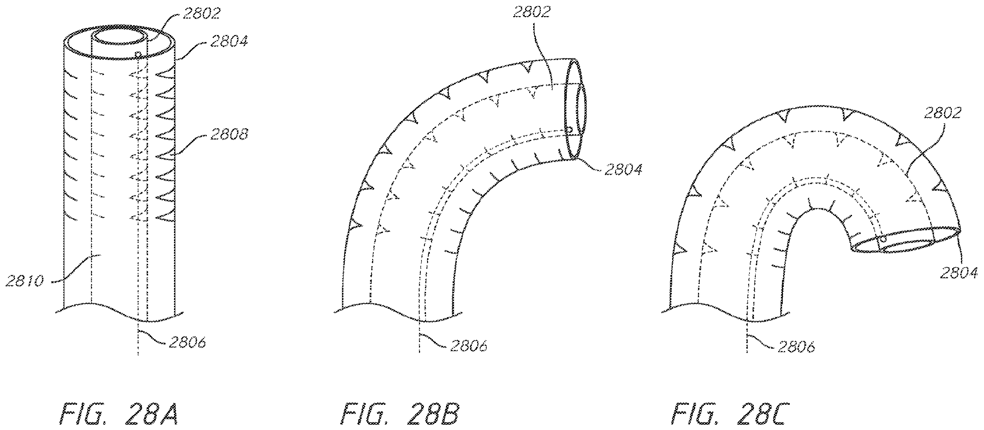

**Figures 28A-28C** illustrate a design which does not incorporate a stabilization feature as discussed herein. As shown, the spines **2810** of the different shafts **2802/2804** are aligned in **Figure 28A****,** so that when tension is applied to a pull wire **2806** the shafts **2802/2804** bend along the cut pattern **2808** as shown in **Figure 28B****.** However, if a compressive force is applied, the shafts **2802/2804** can transition into an uncontrolled bend as shown in **Figure 28C****,** which can lead to inadvertent release or movement of the prosthesis **70.**

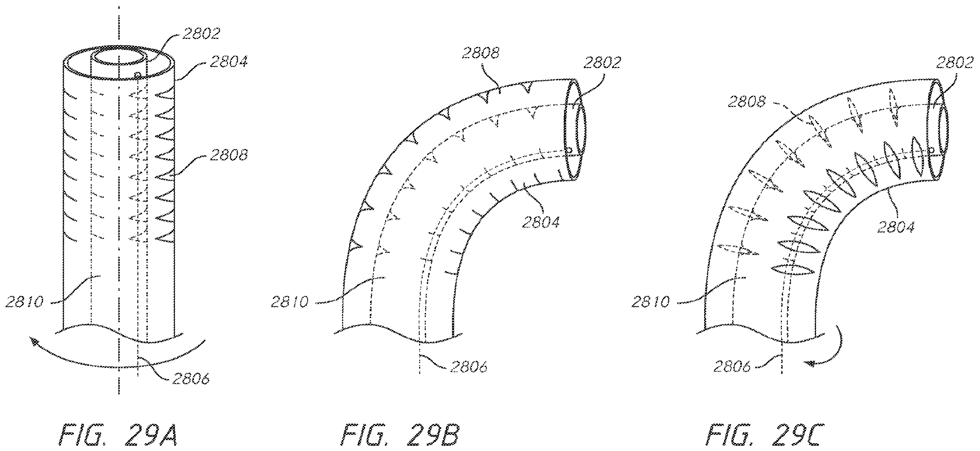

In order to avoid the uncontrolled bend shown in **Figure 28C****,** as shown in **Figures 29A-29C****,** the delivery system **10** can allow for rotation of the different shafts **2802/2804** with respect to one another. Either one of the shafts **2802/2804** can be configured to rotate, or both can rotate. During delivery, the shafts **2802/2804** can be rotated with respect to one another so that the spine **2810** of one shaft (e.g., shaft **2802** is aligned with the cut pattern **2808** of another shaft (e.g., shaft **2804**)**.** Thus, when a load is applied, the shafts **2802/2804** stabilize each other, preventing uncontrolled flexing as shown in **Figure 28C****,** and instead only partially bend such as shown in **Figure 29C****.** When a flex is desired, the shafts **2802/2804** can be rotated so that the cut patterns **2808** align, allowing a pull wire **2806** to bend the shafts **2802/2804** in the desired direction as shown in **Figure 29B****.**

The above disclosed shaft configuration can be used in the inner assembly **18,** mid shaft assembly **21,** the rail assembly **20,** nose cone assembly **31,** and/or outer sheath assembly **22** and may additionally include such features to connect different shaft assemblies of other delivery systems not specifically illustrated herein.

### Keyed Delivery Components

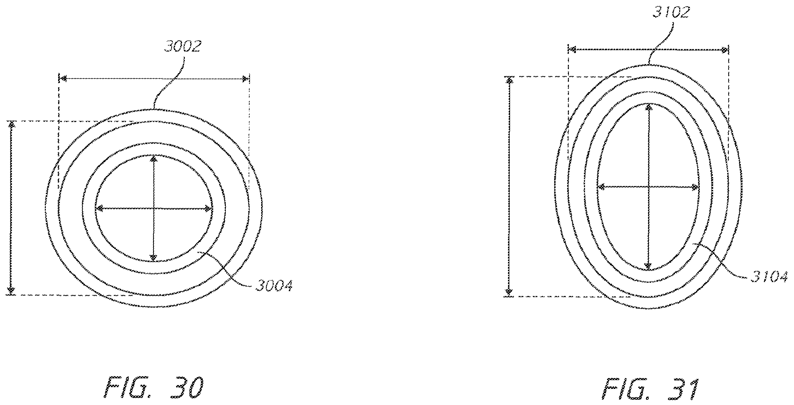

In some delivery systems, inner and outer shafts/lumens are not "keyed", meaning that the shafts or lumens can rotate with respect to one another, as shown in **Figure 30****.** This can cause incidental (e.g., undesired) rotation of the shafts while using the delivery system **10** as an inner shaft **3004** can freely rotate within an outer shaft **3002** without constraint. To avoid this rotation, the handle **14** may utilize different connectors to prevent rotation. However, as the handle is at a proximal end of the shafts or lumens, this type of delivery system does not ensure that the two shafts are clocked or keyed along the lengths of the shafts all the way to the distal ends, or at least at the distal ends.

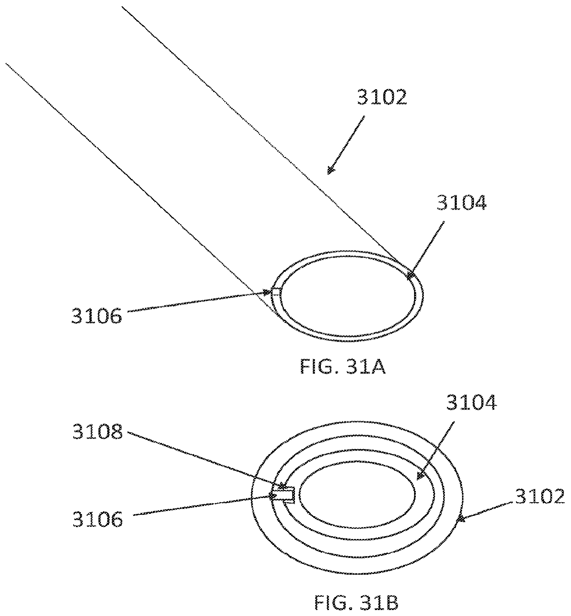

Accordingly, in some embodiments, the shafts or lumens (e.g., pull wire lumens **139** of the rail assembly **20**) disclosed herein may be "keyed" as shown in **Figures 31****,** **31A and 31B****.** The "keyed" configurations can prevent accidental (e.g., undesired, inadvertent, incidental) rotation of one shaft or lumen with respect to another shaft or lumen while still allowing translation (e.g., axial translation) with respect to each other. The keying can occur at a discrete portion of the shafts, such as at a distal end or a middle section, or may extend partially or fully along the length of the shafts.

As shown in **Figures 31****,** **31A and 31B****,** in order to key two concentrically-arranged shafts or lumens together, one axis of the shafts **3102/3014** may be larger (e.g., have a longer length) than an orthogonal axis. The illustrated arrangement can form generally ovaloid lumens (e.g., eccentric or elliptical lumens), rather than a circular lumen as shown in **Figure 30****.** Thus, the inner shaft **3104** is prevented from rotating within the outer shaft **3102** through the physical constraint of the shape of the shafts (e.g., outer shaft **3102** and/or inner shaft **3104**). While an ovaloid (e.g., eccentric or elliptical) configuration is disclosed, other configurations can be used as well, such as square or triangular shafts. In some embodiments, circular shafts can include internal components or external components that create an ovaloid shape, which can also prevent rotation while still allowing for relative translation.

Additionally, other keying embodiments can be used as well. For example, as illustrated in Figures 31A and 31B, one of the shafts (e.g., outer shaft **3102** as shown in the example of Figures 31A and 31B or inner shaft **3104**) can include a tab **3106** which fits into a slot **3108** on another shaft (e.g., inner shaft **3104** as shown in the example of Figures 31A and 31B or outer shaft **3102**), thereby preventing rotation. The particular keying structure is not limiting.

The above disclosed keying shaft configurations can be used in the inner assembly **18,** mid shaft assembly **21,** the rail assembly **20,** nose cone assembly **31**, and/or outer sheath assembly **22** or other shafts or lumens of a delivery system and may additionally include such features to connect different shaft assemblies or lumens. The keying shaft configurations can also be used in pull wire lumens (e.g., pull wire lumens **139** of the rail assembly **20**) of the delivery system (e.g., delivery system **10**).

### Valve Suture Locking and Release Mechanism

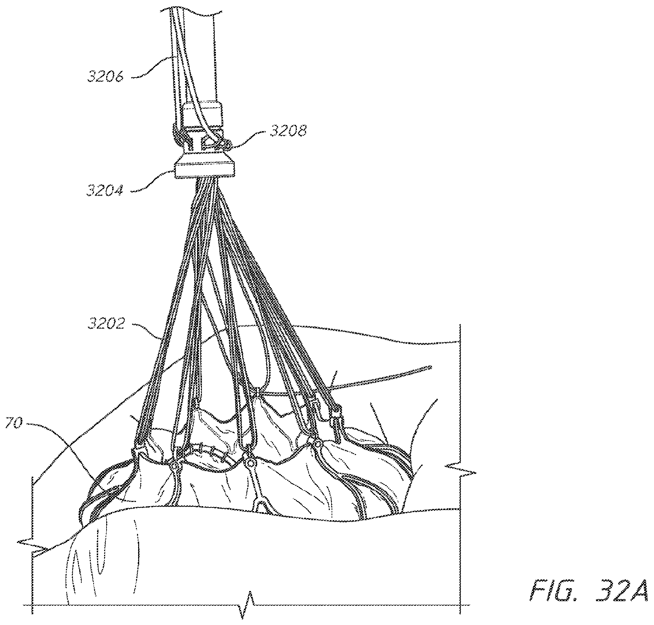

In some embodiments, tethers/sutures can be used as attachment mechanisms between a valve delivery system (e.g., delivery systems disclosed herein or delivery systems configured for delivery of replacement heart valves to replace an atrioventricular valve, a mitral valve, a tricuspid valve or other heart valve) instead of, or in conjunction with, the particular prosthesis **70** attachment mechanisms disclosed above. **Figures 32A-36B** illustrate embodiments of tether/suture attachment mechanisms.

As shown in **Figure 32A****,** tethers **3202** can extend from the prosthesis **70** (e.g., replacement heart valve) to a shaft having a manifold **3204** so that the prosthesis **70** can fully expand, such as using the methods disclosed above while still remaining connected to the delivery system **10** by the tethers **3202.** The manifold **3204** can be used instead of the inner retention member **40** disclosed above. In some embodiments, both components can be used so that when the prosthesis **70** is released from the inner retention member **40,** it stays attached to the delivery system **10** through the tethers **3202.**

The manifold **3204** can include a number of apertures **3201** extending radially for loops **3208** (such as looped ends) of the tethers **3202** to extend through. Thus, the tethers **3202** can extend into an inner lumen of the manifold **3204** (e.g., from a distal direction) and partially extend through the apertures **3201** to an outer surface (e.g., radial or side surface) of the manifold **3204.** A release (or locking) tether/suture **3206** can extend through the loops **3208,** thus preventing the prosthesis (e.g., valve) **70** from being released from the manifold **3204** until ready.

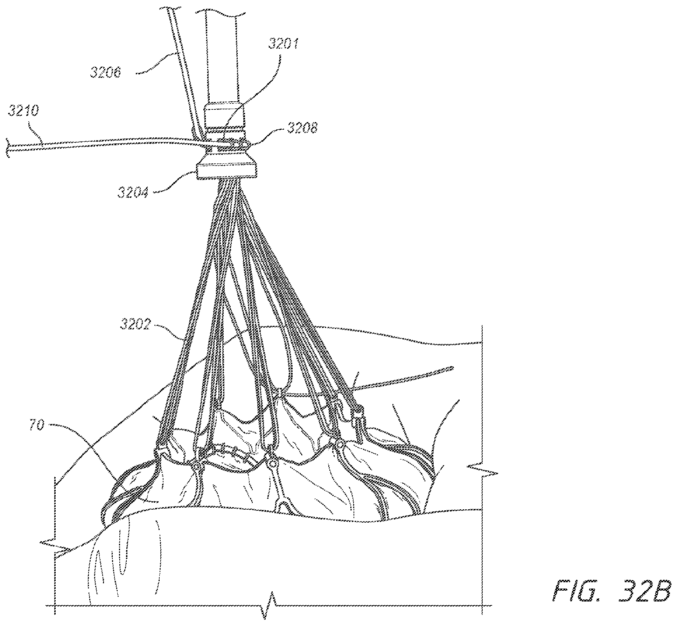

The release tether **3206** can be withdrawn so that a free end **3210** of the release tether **3206** can pass through the loops **3208,** thus releasing the prosthesis (e.g., valve) **70** from the tethered attachment to the manifold **3204.** as shown in **Figure 32B****.** Multiple release (or locking) tethers/sutures **3206** may be used in some embodiments (e.g., one for each loop **3208** or one for multiple loops **3208**).

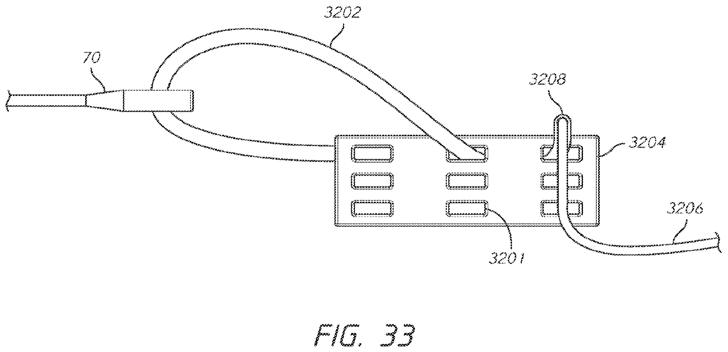

**Figure 33** illustrates an example of the manifold **3204** with apertures **3201.** The number of apertures **3201** and the pattern of arrangement of apertures **3201** may vary as desired and/or required.

Advantageously, natural tension that the prosthesis **70** exerts on the suture loops **3208** when the prosthesis **70** is loaded also cinches the release (or locking) tether **3206,** so there is no need for a catheter or user to keep a constant tension on the release (or locking) tether **3206.** At a time when the prosthesis **70** is ready for release, a user may pull one end of the release (or locking) tether **3206** until it is unthreaded from all of the loops **3208,** thereby enabling the loops **3208** to detach from the prosthesis **70** and the prosthesis **70** to thereby separate from the manifold **3204** of the delivery system.

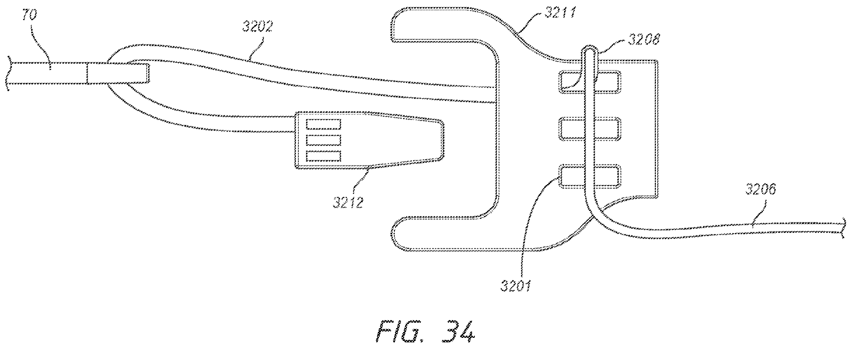

**Figure 34** illustrates an alternate embodiment of a tethered release system. As shown, a manifold combination, or assembly, can be composed of a modified outer retention member **3211** as well as modified inner retention member **3212.** The tethers **3202** can have one end attached to the inner retention member **3212.** The tethers **3202** can then pass through a portion of the prosthesis **70** (e.g., an eyelet extending from a proximal end of the prosthesis **70**) and return back to the outer retention member **3211,** where a second end of the tethers **3202** extends through the apertures **3201** and contains a loop **3208** for the release/locking tether **3206** to pass through. Members **3212/3211** can be moved relative to one another in order to tighten or loosen the tethers **3202.** The tethers **3202** may comprise a single continuous strand or multiple separate strands.

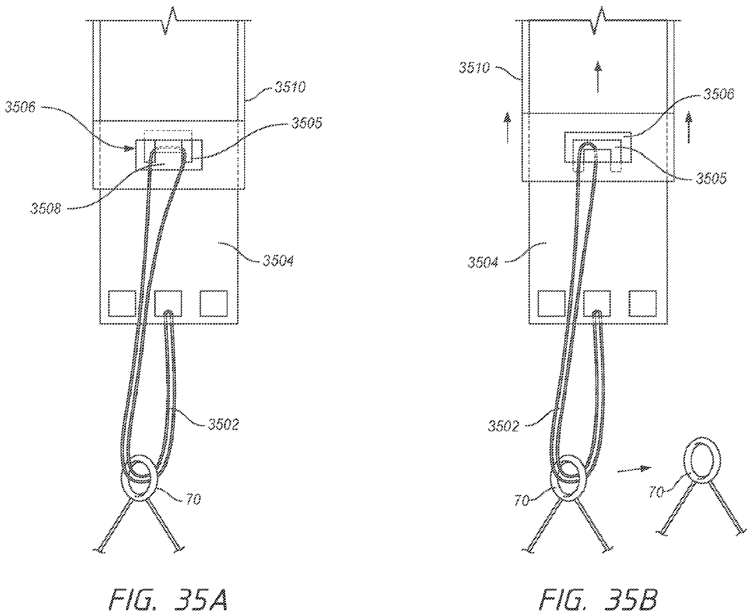

**Figures 35A** and **35B** illustrate another embodiment of a suture attachment mechanism in locked (**Figure 35A**) and unlocked (**Figure 35B**) positions, or configurations. The suture attachment mechanism facilitates tethered attachment and release between a prosthesis (e.g., replacement heart valve) and a delivery system (e.g., delivery device or catheter). As shown, a tether **3502** (e.g., tether or suture loop) can be attached (e.g., non-removably attached or permanently attached via glue or other adhesive coupling) to a distal end of an inner shaft manifold **3504** and then can extend through a portion (e.g., an eyelet or other retention member) of a prosthesis (e.g., prosthesis **70**) and then looped back to an outer shaft member **3510** (e.g., sleeve, shaft or lumen). The outer shaft member **3510** can include at least one radial aperture **3506.** The inner manifold **3504** can include at least one aperture **3505** adapted to be aligned with the at least one radial aperture **3506** of the outer shaft member **3510,** or vice-versa. The number of apertures **3505, 3506** may be the same or different. For example, the outer shaft member **3510** can have a single radial aperture **3506** extending around all or a portion of a circumference of the distal end portion of the outer shaft member **3510** and the inner manifold 3504 can have multiple circumferentially-spaced apart radial apertures **3505.** As another example, the outer shaft member **3510** can include the same number of apertures as the inner manifold **3504** and corresponding apertures may be configured to align with each other circumferentially. The at least one aperture **3505** of the inner manifold **3504** includes a proximally- and/or radially outward- facing tooth **3508** extending from a distal edge of the aperture **3505** toward a proximal edge of the aperture **3505.** The tether **3502** (e.g., one loop end of the tether **3502**) can wrap around the tooth **3508.** The outer shaft member **3510** of the manifold assembly can cover at least the proximal portion of the aperture **3505** in the locked position, or configuration, thereby preventing the tether **3502** (e.g., looped end of the tether **3502**) from releasing (e.g., from being able to come off or be released from the tooth **3508**) when in the locked position of **Figure 35A****.** The outer shaft member **3510** and/or the inner shaft manifold **3504** can be translated axially relative to each other. When the outer shaft member **3510** is proximally withdrawn or the inner manifold **3504** is distally advanced, to an unlocked configuration, the proximal end of tooth **3508** is exposed and the tether **3502** (e.g., looped end of the tether **3502**) can be released from the tooth **3508,** thereby facilitating decoupling of the prosthesis **70** from the manifold assembly of the delivery system while the tether **3502** remains attached to the inner manifold **3504.** As the delivery system is withdrawn or removed from a desired implantation location (e.g., mitral valve annulus such as shown in **Figures 19** or **20****,** tricuspid valve annulus, atrioventricular valve annulus), the tether **3502** is pulled through the eyelet or other retention member of the prosthesis **70** such that the prosthesis **70** is no longer tethered, as shown schematically in **Figure 35B** by the eyelet of the prosthesis **70** without a tether **3502.** The suture attachment mechanism may include multiple tethers **3502** even though a single tether is shown for simplicity.

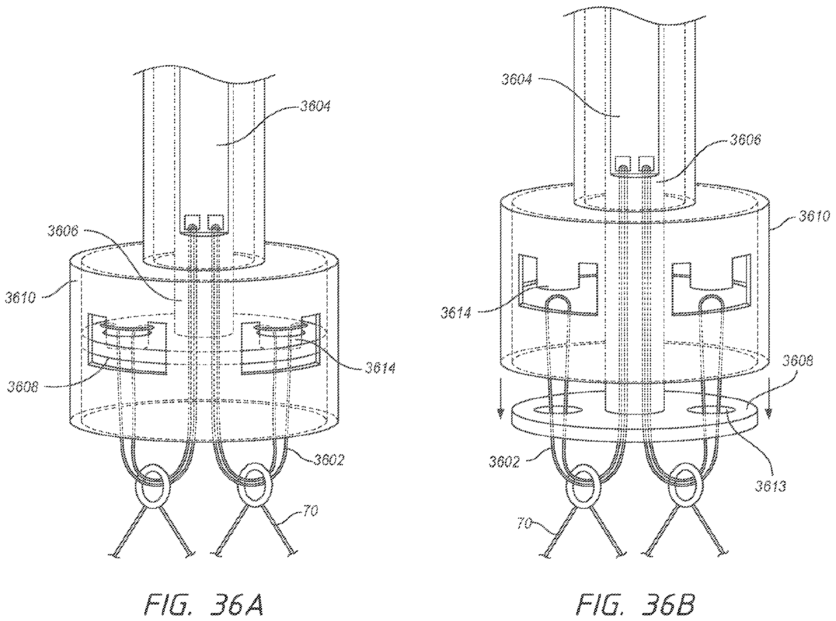

**Figures 36A and 36B** illustrate an alternative locking and release tether/suture mechanism with multiple lumens. As shown, sutures **3602** (e.g., suture or tether loops) can extend from an attachment point of an inner shaft **3604.** The sutures **3602** can extend through the lumen of a middle shaft **3606** and out a distal end of the middle shaft **3606.** The middle shaft **3606** can have a release disc **3608** on or within a distal end of the middle shaft **3606.** The sutures **3602** can loop proximally (e.g., from a distal side of the release disc **3608**) through apertures **3613** in the release disc **3608** and extend proximally to wrap around teeth **3614** of an outer ring **3610.**

In the locked position, or configuration, shown in **Figure 36A****,** the disc **3608** can be proximally withdrawn to abut the teeth **3614,** thereby preventing release of the sutures **3602.** The disc **3608** can be advanced distally to allow for release of the tethers, or sutures **3602.**

The tether/suture attachment mechanisms described herein can be used in conjunction with any prosthesis (e.g., replacement heart valve) that includes retention components (e.g., eyelets) through which one or more tethers can be looped. For example, the prosthesis could be a modified version of the prosthesis **70** illustrated in **Figure 3A** that includes eyelets as shown in one or more of **Figures 32A-36B** instead of or in addition to the mushroom-shaped tabs **74.** The prosthesis may be a replacement mitral valve, a replacement tricuspid valve, or other replacement heart valve. The tether/suture attachment mechanisms described herein (e.g., the manifold assemblies) may be incorporated into the delivery systems described herein (e.g., delivery system **10** or other delivery systems (e.g., transcatheter, transfemoral, transseptal delivery systems in conjunction with mitral valve replacement, tricuspid valve replacement, or other native heart valve replacement procedures).

### High Compression And Flexible Implant Housing

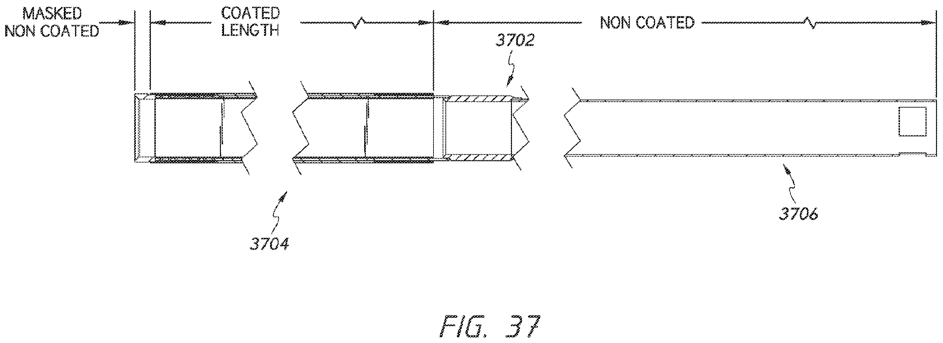

In some embodiments, the capsule **106** may have an alternate construction than discussed above, which are shown in **Figures 37-39B****.** Valves and their location in the human anatomy are challenging to navigate in order to reposition or retrieve a replacement device (e.g., prosthesis or replacement valve **70**), such as disclosed herein.

Advantageously, this alternative construction may allow for repositioning or retrieval of the implant (e.g., replacement heart valve). To do so, the capsule **3702** may be flexible, be good in compression, and good in tension. Thus, embodiments of the capsule **3702** may be universally flexible, able to deliver high compressive and tensile forces, and a low profile.

As shown in **Figure 37****,** the capsule **3702** can have a similar configuration to that of capsule **106** as disclosed above. The capsule **3702** can be formed of a distal section **3704** and a proximal section **3706.** In some embodiments, the distal section **3704** has a larger diameter than the proximal section **3706.** In some embodiments, the diameters may be the same.

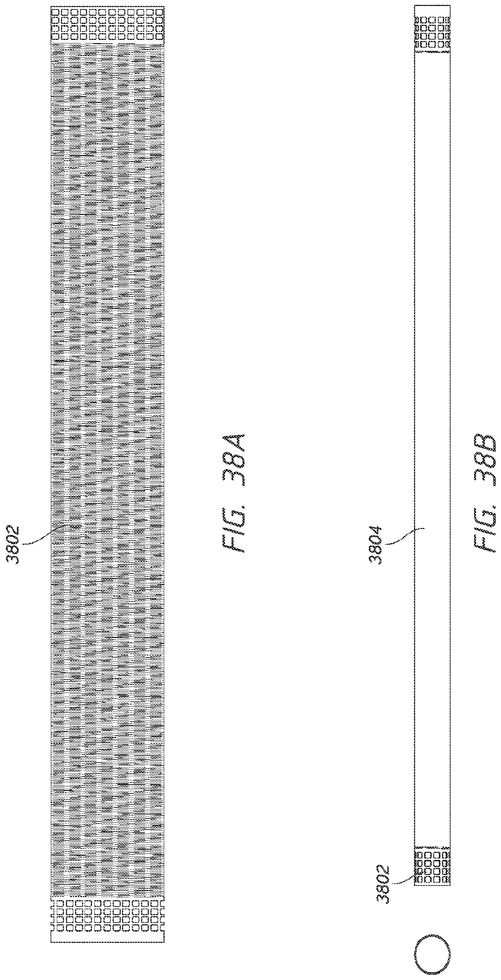

**Figure 38A** illustrates a flat pattern of the distal section **3704.** The distal section **3704** can be formed from a laser cut hypotube **3802.** As discussed above, the metal component can be a laser cut hypotube with an interrupted spiral. The hypotube **3802** can include a number of cuts and window sizes, dictating the flexibility of the metal structure.

Further, the distal section **3704** can include an outer jacket **3804** which can partially or fully surround the hypotube **3802,** as shown in **Figure 38B****.** For example, the jacket **3804** can cover greater than 70, 80, 90, or 95% of a length of the hypotube **3802.** In some embodiments, thermoplastics may be used on the outer diameter of the hypotube **3802** to form the outer jacket **3804.** An optional porous fluoropolymer can also be added to the outside, which is the "coated length" of **Figure 37****.** Advantageously, the metal structure of the hypotube **3802** can provide compression resistance, whereas the liner and/or jacket material **3804** can provide tension resistance.

Further, the hypotube **3802** can have an inner liner. In some embodiments, the inner liner is a fluoropolymer. In certain embodiments, the inner liner may be porous. The inner liner can then be bonded to a metal structure of the hypotube **3802,** such as using a reflow process. A polymer can be used which bonds the inner liner to the metal. For example, a low durometer thermoplastic elastomer or a thermoplastic polyurethane can be used.

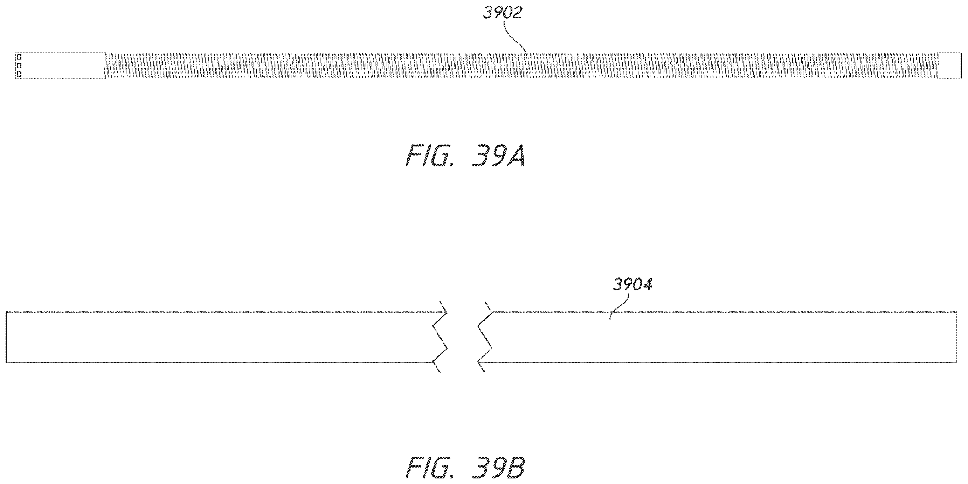

The proximal section **3706** can further include a lasercut hypotube **3902,** shown in **Figure 39A** which can be surrounded by an outer polymer jacket **3904,** shown in **Figure 39B****.**

### Fail-Safe Knobs or Load Indication Knobs

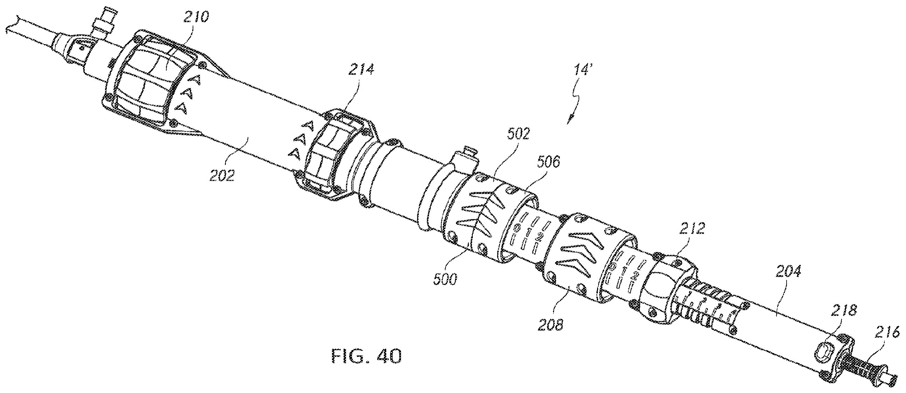

Another embodiment of the handle **14'** is shown in **Figure 40****.** The handle **14'** is similar to the handle **14** shown in Figures **16-17** except as described differently below. The handle **14'** can include one or more failsafe knobs **500.** The failsafe knobs **500** may advantageously prevent the delivery system **10** from reaching a certain unacceptable or undesired level of force or load (e.g., shear force or bending force) that could result in device failure. In accordance with several embodiments, the failsafe knob **500** can act like a backup "fuse" that permanently or temporarily disables continued advancement of one or more lumens or shafts of the delivery system **10** once a threshold level of force or load is reached. As one example, the failsafe knob **500** can be integrated with the distal pull wire knob **206,** the proximal pull wire knob **208,** or each of the knobs **206, 208** such that the failsafe knob **500** can include the same functionality as the distal pull wire knob **206** or the proximal pull wire knob **208** described above in relation to Figures **16-17****.** In some embodiments, the failsafe knob **500** can be integrated with any knob on the handle **14'** (e.g., distal pull wire knob **206,** proximal pull wire knob **208,** outer sheath knob **210,** depth knob **212,** mid shaft knob **214,** knob **216,** or other knobs) or be a separate, standalone knob.

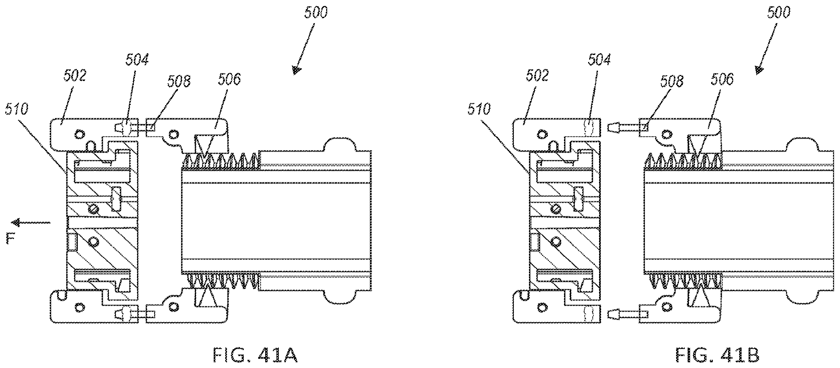

The failsafe knob **500** can be configured to decouple a lumen or shaft of the delivery system **10** from the handle **14'** when a threshold force is applied to the shaft assembly **12** (e.g., to one or more lumens or shafts) of the delivery system **10.** For example, the failsafe knob **500** can include a first portion **502** and a second portion **506.** In some embodiments, the first portion **502** can be positioned distally of the second portion **506** on the handle **14'.** In a first configuration, as shown in Figure **41A****,** the first and second portions **502, 506** can be coupled via, for example, a releasable coupling mechanism. In some configurations, the coupling mechanism can include a spring **504** and a projection **508.** For example, the first portion **502** can include the spring **504** that can be configured to receive the projection **508** of the second portion **506.** In some embodiments, the projection **508** can include one or more grooves configured to engage with the spring **504** when the first and second portions **502, 506** are coupled. In some configurations, the first portion **502** can include the projection **508** and the second portion **506** can include the spring **504.** The first portion **502** can be configured to be detachable from the second portion **506.** For example, in a second configuration, as shown in Figure **41B****,** the first and second portions **502, 506** are decoupled. The spring **504** can comprise a nitinol spring or finger, a tab that deflects at a certain load, a custom spring, or other mechanisms. In some configurations, the coupling mechanism can be a quick connect coupler set or a quick disconnect coupler set. For example, the quick disconnect coupler set can be a quick disconnect hydraulic coupler set. The spring **504** can be a female component of the coupler set and the projection **508** can be a male component of the coupler set. The female component can be configured to releasably receive the male component. For example, the two components can disconnect when a threshold force exerted on the system **10** is reached, as further described below.

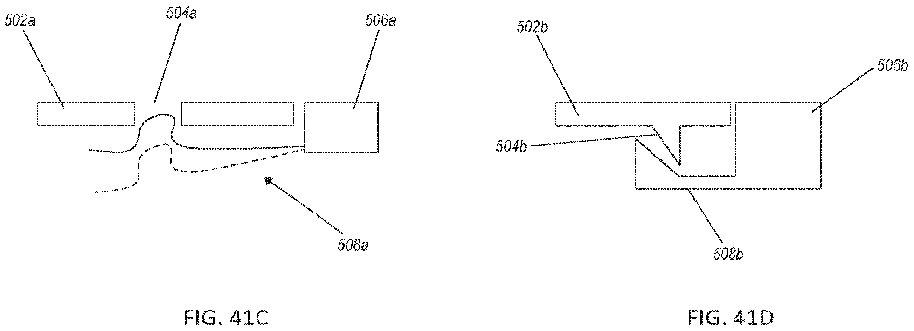

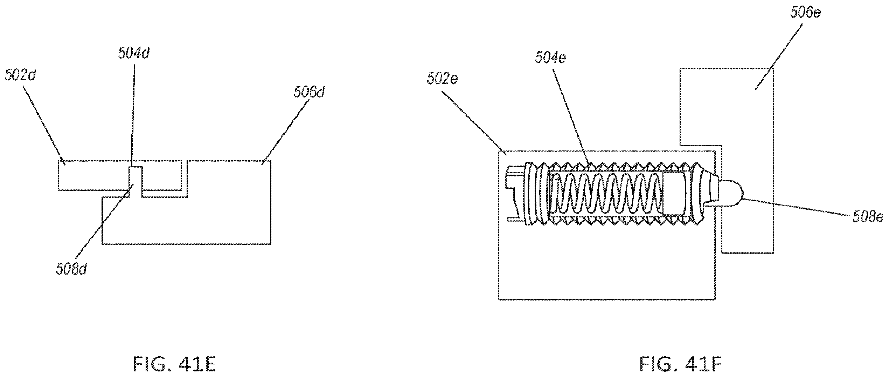

Figures **41C-41F** are schematic illustrations for alternative coupling mechanisms. In some configurations, the coupling mechanism can include a spring tab, as shown in Figure **41C****.** For example, one of the first portion **502a** or the second portion **506a** can include a spring tab **508a** configured to engage with a hole or recess **504a** of the other one of the first portion **502a** or the second portion **506a** when the first and second portions **502a, 506a** are coupled. When a threshold force is exerted on the delivery system **10,** as further described below, the spring tab **508a** can deflect such that the first and second portions **502a, 506a** decouple. The solid line in Figure **41C** illustrates the spring tab **508a** in a first configuration being received in the hole **504a** when the first and second portions **502a, 506a** are coupled. The dashed lines in Figure **41C** illustrate the spring tab **508a** in a second configurations when the first and second portions **502a, 506a** decouple and the spring tab **508a** deflects from the first configuration. In some configurations, the coupling mechanism can include a ratchet coupling, as shown in Figure **41D****.** For example, one of the first or second portions **502b, 506b** can include a ramped recess **508b** and the other one of the first or second portions **502b, 506b** can include a ramped protrusion **504b** configured to be received by the ramped recess **508b** when the first and second portions **502b, 506b** are coupled. When the threshold force exerted on the system **10** is reached, the ramped protrusion **504b** can deflect such that the first and second portions **502b, 506b** decouple. In some configurations, the coupling mechanism can include a shear coupling, as shown in Figure **41E****.** For example, one of the first or second portion **502d, 506d** can include a shearing pin or other protrusion **508d** configured to be received by a recess **504d** in the other one of the first or second portion **502d, 506d.** When the threshold force is exerted on the system **10,** the shearing pin or other protrusion **508d** can shear off or break such that the first and second portions **502d, 506d** can be permanently decoupled. In some configurations, the coupling mechanism can include a spring plunger, as shown in Figure **41F****.** For example, one of the first or second portions **502e, 506e** can include the spring plunger and the other one of the first or second portion **502e, 506e** can include a recess **508e** configured to receive the pin or nose of the spring plunger **504e.** When the threshold force is exerted on the system **10,** the spring plunger **504e** can decouple from the recess **508e** such that the first and second portions **502e, 506e** are decoupled.

The first portion **502** of the failsafe knob **500** can be coupled to an adapter **510** of or within the handle **14'.** The adapter **510** can be positioned radially inward of the failsafe knob **500** and engage with the delivery housing **204.** The second portion **506** of the failsafe knob **506** can include an engagement portion configured to engage with the delivery housing **204.** For example, the delivery housing **204** can be threaded and the engagement portion can be a projection (e.g., a v-shaped projection or tab) configured to engage the threading in the delivery housing **204.**

In use, a force **F** can be applied to the adapter **510** that can be correlated to a force exerted on the shaft assembly **12** (e.g., one or more lumens or shafts) of the delivery system **10** when the user navigates the shaft assembly **12** using the failsafe knob **500.** When the force **F** reaches a threshold force, the first portion **502** may move distally until the projection **508** of the second portion **506** detaches from the spring **504** of the first portion **502.** When the first portion **502** and the second portion **506** decouple, the user may no longer be able to rotate the failsafe knob **500** (either permanently or temporarily until the user reduces the force exerted on the shaft assembly **12** such that the force **F** is less than the threshold force). If temporary, once the force **F** is reduced, the user may couple the first portion **502** with the second portion **506** and resume the procedure. Advantageously, the failsafe knob **500** may prevent the delivery system **10** from failing due to excess forces or loads (e.g., (e.g., shear force or load) being exerted on the delivery system **10** (e.g., one or more shafts or lumens of the delivery system **10**). The threshold force may be a level of force or load substantially below a calculated failure level during testing so that risk of failure is reduced. For example, if the one or more lumens or shafts of the shaft assembly **12** have an ultimate tensile strength of 20 lbs., the threshold force can be 10 lbs. for a safety factor of 2 or can be 15 lbs. for a safety factor of 1.5. In some configurations, the threshold force can be between about 1 lb. and about 50 lbs., about 10 lbs. and about 40 lbs., or about 20 lbs. and about 30 lbs. In some configurations, the threshold force can be configured for a safety factor of between about 1.0 and about 5.0, about 1.5 and about 4.5, about 2.0 and about 4.0, or about 2.5 and about 3.5.

In some embodiments, the second portion **506** can be positioned distally of the first portion **502** on the handle **14'.** When the force **F** is applied to the adapter **510,** the first portion **502** may move distally toward the second portion **506** such that the spring **504** is increasingly compressed. When the force **F** reaches the threshold force, the compressive forces may cause the first and second portions **502, 506** to decouple. Any of the releasable coupling mechanisms described herein can be configured to decouple the first and second portions **502, 506** based on the compressive forces applied to the first and second portions **502, 506.**

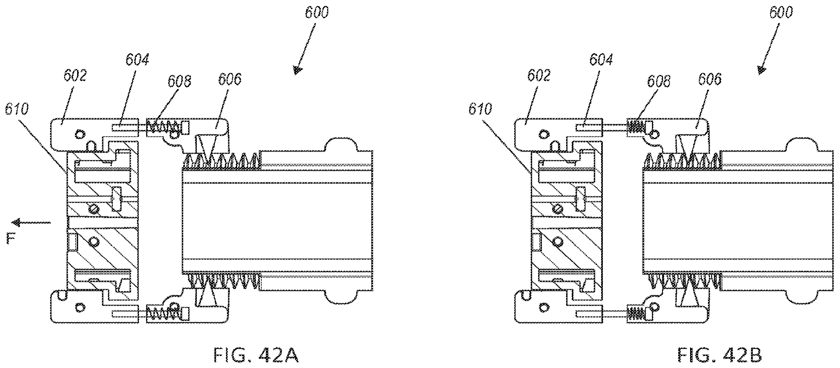

In some embodiments, a knob may not mechanically separate or decouple into two components but may instead provide a visual indicator of an amount of force being exerted on or by one or more shafts or lumens controlled by the knob. An embodiment of an indicator knob **600** is shown in Figures **42A-42B****.** The handle **14'** can include one or more indicator knobs **600** in addition to or instead of the failsafe knob **500.** For example, the indicator knob **600** can be integrated with the distal pull wire knob **206,** the proximal pull wire knob **208,** or each of the knobs **206, 208** such that the indicator knob **600** can include the same functionality as the distal pull wire knob **206** or the proximal pull wire knob **208** described above in relation to Figures **16-17****.** In some embodiments, the indicator knob **600** can be integrated with any knob on the handle **14'** (e.g., distal pull wire knob **206,** proximal pull wire knob **208,** outer sheath knob **210,** depth knob **212,** mid shaft knob **214,** knob **216,** or other knobs) or be a separate, standalone knob. In some embodiments, the indicator knob **600** can be integrated with the failsafe knob **500.**

The indicator knob **600** can be configured to indicate (e.g., visually) when a force being applied to the shaft assembly **12** (e.g., one or more lumens or shafts controlled by, or operatively coupled to, the indicator knob 600) of the delivery system **10** increases and/or decreases, a "real-time" amount of force being applied, or when a threshold amount of force has been reached. For example, the indicator knob **600** can include a first portion **602** and a second portion **606.** The first portion **602** can be positioned distally from the second portion **606.** The first portion **602** can be connected to the second portion **606** via a connector **604.** A first or distal end of the connector **604** can be moveably secured in the first portion **602** and a second or proximal end of the connector **604** can be moveably secured in the second portion **606.** For example, the first and second portions **602, 606** can have a recess (e.g., groove, notch, slot) that extends from an opening of the first portion **602** or the second portion **606.** The recess of the first portion **602** can be configured to receive the first end and/or a distal portion of the connector **604.** The recess of the second portion **606** can be configured to receive the second end and/or a proximal portion of the connector **604.** The second portion **606** can include a spring **608** that surrounds the connector **604** within the second portion **606.**

As shown in Figure **42A****,** when the first and second portions **602, 606** are in a first configuration, the connector **604** can be substantially or entirely encompassed by the first and second portions **602, 606.** Moreover, the second end of the connector **604** can be enlarged such that the second end has a greater diameter than the adjacent portions of the connector **604.** For example, the enlarged second end can have a larger diameter than the opening of the recess of the second portion **606** such that the connector **604** may not be removed from the second portion **606.** Moreover, the spring **608** can be positioned between the enlarged second end of the connector **604** and the opening of the second portion **606** to exert a force on the second portion **606** toward the first portion **602.** When the first and second portions **602, 606** are in a second configuration, the connector **604** may be at least partially or substantially exposed such that a distance between the first and second portions **602, 606** is greater in the second configuration than in the first configuration. Moreover, the spring **608** can be compressed in the second configuration.

The first portion **602** of the indicator knob **600** can be coupled to an adapter **610** of or within the handle 14'. The adapter **610** can be positioned radially inward of the indicator knob **600** and engage with the delivery housing **204.** The second portion **606** of the indicator knob **606** can include an engagement portion configured to engage with the delivery housing **204.** For example, the delivery housing **204** can be threaded and the engagement portion can be a projection (e.g., a v-shaped projection) that can be configured to engage the threading in the delivery housing **204.**

In use, a force **F** can be applied to the adapter **610** that is correlated to a force exerted on the shaft assembly **12** of the delivery system **10** when the user navigates the shaft assembly **12** (e.g., one or more shafts or lumens of the shaft assembly **12**) using the indicator knob **600.** When a force is exerted on the shaft assembly **12** (e.g., one or more shafts or lumens of the shaft assembly **12**), the force **F** applied to the adapter **610** may cause the first portion **602** to move distally while the position of the second portion **604** can remain unchanged. In some embodiments, both portions **602, 606** can be configured to move in response to the applied force **F.** As the force exerted on the shaft assembly **12** (e.g., one or more shafts or lumens of the shaft assembly **12** operatively coupled to the indicator knob **600**) increases, the increasing force **F** applied to the adapter **610** may cause the distance between the first and second portions **602, 606** to increase. The increasing force **F** may cause an increasing length of the connector **604** to be uncovered. The distance between the first and second portions **602, 606** and the length of the connector **604** that is uncovered can correlate (e.g., be proportional) with the amount of force **F** that is being exerted on the adapter **610.** For example, the shorter the distance between the first and second portions **602, 606** and the greater length of the connector **604** that is covered, the less force **F** that may be applied to the adapter **610.** Moreover, the greater the distance between the first and second portions **602, 606** and the greater length of the connector **604** is uncovered, the greater the force **F** that may be applied to the adapter **610.** Accordingly, as the force **F** is reduced, the distance between the first and second portions **602, 606** and the length of the connector **604** that is uncovered may decrease. In some embodiments, the distance between the first and second portions **602, 606** may be inversely related to the force **F** being applied.

The connector **604** may include indicators along the length of the connector **604** that can correlate with the force **F** being applied to the delivery system **10.** For example, the indicators can include colors, bands, stripes, numbers, shapes, symbols, or the like. The connector **604** can include any number of indicator(s). For example, the connector **604** can include one, two, three, four, five, six, or more than six indicator(s). In some embodiments, the connector **604** may include three indicators. The three indicators can include a band of different colors. For example, the three indicators can include one or more red bands, one or more yellow bands, and one or more green bands. The one or more green bands can include a single green band at or near the middle of the connector **604.** The one or more red bands can include two red bands that can be positioned adjacent the first and second portions **602, 606.** The one or more yellow bands can include two yellow bands that can be positioned between the green band and the red bands. The green band can indicate that the amount of force being applied to the delivery system **10** is within an acceptable range or a preferred range. The red bands can indicate that the amount of force being exerted on the delivery system **10** is in an unacceptable range and the user should reduce the amount of force. The yellow bands can indicate the amount of force being applied to the delivery system **10** is within an acceptable range but the amount of force is increasing to the unacceptable range or the amount of force is decreasing to the preferred range.

In some embodiments, the second portion **606** can be positioned distally of the first portion **602** on the handle **14'.** The distance between the first and second portions **602, 606** may be inversely related to the force **F** being exerted on the delivery system **10.** For example, the greater the distance between the first and second portions **602, 606** and the greater length of the connector **604** is uncovered, the less the force **F** that may be applied to the adapter **610.** Accordingly, as the force **F** is increased, the distance between the first and second portions **602, 606** and the length of the connector **604** that is uncovered may decrease.

In some embodiments, the indicator knob **600** can include the same or similar functionality as the failsafe knob **500.** For example, a distance between the first and second portions **602, 606** of the indicator knob **600** may increase as the force **F** being exerted on the delivery system **10** increases, as described above. Once the force **F** reaches a threshold force, the first and second portions **602, 606** may decouple.

From the foregoing description, it will be appreciated that an inventive product and approaches for implant delivery systems are disclosed. While several components, techniques and aspects have been described with a certain degree of particularity, it is manifest that many changes can be made in the specific designs, constructions and methodology herein above described without departing from the spirit and scope of this disclosure.

Certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as any subcombination or variation of any subcombination.

Moreover, while methods may be depicted in the drawings or described in the specification in a particular order, such methods need not be performed in the particular order shown or in sequential order, and that all methods need not be performed, to achieve desirable results. Other methods that are not depicted or described can be incorporated in the example methods and processes. For example, one or more additional methods can be performed before, after, simultaneously, or between any of the described methods. Further, the methods may be rearranged or reordered in other implementations. Also, the separation of various system components in the implementations described above should not be understood as requiring such separation in all implementations, and it should be understood that the described components and systems can generally be integrated together in a single product or packaged into multiple products. Additionally, other implementations are within the scope of this disclosure.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Conjunctive language such as the phrase "at least one of X. Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. For example, within less than or equal to 10 wt./vol. % of, within less than or equal to 5 wt./vol. % of, within less than or equal to 1 wt./vol. % of, within less than or equal to 0.1 wt./vol. % of, and within less than or equal to 0.01 wt./vol. % of the stated amount.

Some embodiments have been described in connection with the accompanying drawings. The figures are drawn to scale, but such scale should not be limiting, since dimensions and proportions other than what are shown are contemplated and are within the scope of the disclosed inventions. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

While a number of embodiments and variations thereof have been described in detail, other modifications and methods of using the same will be apparent to those of skill in the art. Accordingly, it should be understood that various applications, modifications, materials, and substitutions can be made of equivalents without departing from the unique and inventive disclosure herein or the scope of the claims.
Examples are set out in the following list of numbered clauses:
1. A delivery system for a replacement heart valve, the delivery system comprising:
   an inner shaft having a proximal end and a distal end,
   wherein the distal end of the inner shaft comprises a manifold,
   wherein the manifold comprises a plurality of circumferentially-spaced apart apertures, each of the apertures having a tooth extending from a distal edge of the aperture toward a proximal edge of the aperture;
   an outer shaft member configured to cover the apertures of the manifold when in a distal position to prevent release of the looped end of the attachment tether.
   wherein the outer shaft member comprises at least one aperture configured to be aligned with the plurality of circumferentially-spaced apart apertures of the manifold;
   at least one attachment tether configured to releasably connect to at least one eyelet of the replacement heart valve,
   wherein a first looped end of the at least one attachment tether is configured to be attached to the distal end of the inner shaft,
   wherein a second looped end of the at least one attachment tether is configured to extend through the at least one eyelet of the replacement heart valve, through the at least one aperture of the outer sleeve into a respective one of the apertures of the manifold, and then looped over a free proximal end of the tooth,
   wherein at least one of the outer shaft member and the inner manifold is configured to translate axially relative to the other to uncover the second looped end of the at least one attachment tether such that the second looped end can be removed from the tooth, thereby facilitating release of the replacement heart valve from the delivery system.
2. The delivery system of clause 1, wherein the at least one aperture of the outer shaft member comprises a plurality of circumferentially-spaced apart apertures.
3. The delivery system of clause 1, wherein the outer shaft member is configured to be moved proximally relative to the inner shaft to uncover the second looped end of the at least one attachment tether.
4. The delivery system of clause 1, wherein the inner shaft is configured to be moved distally relative to the outer shaft member to uncover the second looped end of the at least one attachment tether.
5. The delivery system of clause 1, wherein the outer shaft member comprises a sleeve.
6. The delivery system of clause 1, wherein the at least one attachment member comprises a plurality of attachment tethers, wherein a first looped end of each of the plurality of attachment tethers is configured to be attached to the distal end of the inner shaft, and wherein a second looped end of each of the plurality of attachment tethers is configured to configured to extend through the at least one eyelet of the replacement heart valve, through the at least one aperture of the outer sleeve into a respective one of the apertures of the manifold, and then looped over a free proximal end of the tooth of the respective one of the apertures of the manifold.
7. A method of facilitating delivery of a replacement heart valve within a body of a patient, the method comprising:
   advancing a distal portion of a delivery system to a desired implantation location within a heart of the patient,
   wherein the delivery system comprises an inner shaft and an outer shaft member,
   wherein the distal end of the inner shaft comprises at least one radial aperture, the at least one radial aperture having a tooth extending from a distal edge of the at least one radial aperture toward a proximal edge of the at least one radial aperture,
   wherein the outer shaft member comprises at least one radial aperture configured to be aligned with the at least one radial aperture of the inner shaft,
   wherein a first looped end of an attachment tether is attached to a distal end of the inner shaft,
   wherein a second looped end of the attachment tether is removably coupled to the tooth of the at least one radial aperture of the inner shaft after having been inserted through an eyelet of the replacement heart valve;
   causing the outer shaft member and the inner shaft to transition from a locked configuration in which the second looped end of the attachment member cannot be removed from the tooth to an unlocked configuration in which the second looped end of the attachment member can be removed from the tooth, thereby decoupling the replacement heart valve from the delivery system and allowing the replacement heart valve to remain in the desired implantation location; and
   removing the delivery system from the patient.
8. The method of clause 7, wherein the desired implantation location is a native mitral valve.
9. The method of clause 7, wherein the desired implantation location is a native tricuspid valve.
10. The method of clause 7, wherein causing the outer shaft member and the inner shaft to transition from the locked configuration to the unlocked configuration comprises moving the inner shaft in a distal direction.
11. The method of clause 7, wherein causing the outer shaft member and the inner shaft to transition from the locked configuration to the unlocked configuration comprises moving the outer shaft member in a proximal direction.
12. A delivery system for a replacement heart valve, the delivery system comprising:
   a shaft having a proximal end and a distal end;
   a manifold on a distal end of the shaft, wherein the manifold comprises a plurality of radially extending apertures, each aperture having a tooth;
   at least one attachment tether configured to releasably connect to the replacement heart valve, wherein looped portions of the at least one attachment tether extend through the radially extending apertures of the manifold to surround the tooth; and
   a sleeve configured to cover the radially extending apertures in a distal position to prevent release of the looped portions, wherein the sleeve is configured to be proximally translated to a proximal position to uncover the looped portions so that the replacement heart valve is released from the at least one attachment tether.
13. A delivery system for a replacement heart valve, the delivery system comprising:
   a shaft having a proximal end and a distal end;
   a manifold on a distal end of the shaft, wherein the manifold comprises a plurality of radially extending apertures;
   at least one attachment tether configured to releasably connect to the replacement heart valve, wherein looped portions of the at least one attachment tether extend through the radially extending apertures of the manifold; and
   a release tether configured to extend through the looped portions of the at least one attachment tether;
   wherein when the release tether is withdrawn from the looped portions, the replacement heart valve is released from the at least one attachment tether.
14. The delivery system of clause 13, wherein the manifold comprises an inner manifold and an outer manifold.
15. The delivery system of clause 14, wherein the outer manifold comprises the radially extending apertures for receiving the looped portions of a distal end of the at least one attachment tether.
16. The delivery system of clause 15, wherein a proximal end of the at least one attachment tether is attached to the inner manifold.
17. The delivery system of clause 13, wherein the at least one attachment tether comprises one and only one attachment tether.
18. The delivery system of clause 13, wherein the at least one attachment tether comprises a plurality of attachment tethers.
19. A handle for a replacement heart valve delivery system, the handle comprising:
   a housing;
   at least one knob located on the housing;
   at least one ring gear in communication with the at least one knob, wherein the at least one knob is configured to rotate the at least one ring gear;
   at least one planet gear located within the at least one ring gear and in communication with the at least one ring gear, wherein the at least one planet gear remains in the same circumferential position with relation to the at least one ring gear during rotation of the at least one ring gear; and
   a linear travel screw in communication with the at least one planet gear, wherein the linear travel screw is configured to move in an axial direction upon rotation of the at least one knob.
20. The handle of clause 19, wherein the at least one planet gear comprises a plurality of planet gears, each planet gear of the plurality of planet gears in communication with one of a plurality of linear travel screws.
21. A delivery system for use with a replacement heart valve, the system comprising:
   a bendable nose cone shaft having a proximal end and a distal end;
   a nose cone attached to a distal end of the nose cone shaft; and
   a rigid shaft at least partially covering the bendable nose cone shaft, wherein the rigid shaft is configured to axially translate with respect to the bendable nose cone shaft to cover or uncover the bendable nose cone shaft;
   wherein, when the bendable nose cone shaft is uncovered by the rigid shaft, the bendable nose cone shaft is configured to allow the replacement heart valve attached to the system to conform to a native anatomy of a native heart valve.
22. The delivery system of clause 21, wherein the bendable nose cone shaft comprises a bendable polymer.
23. A delivery system for use with a replacement heart valve, the system comprising:
   an inner retention member configured to releasably retain a replacement heart valve; and
   an outer retention member configured to at least partially cover a portion of the replacement heart valve and the inner retention member;
   wherein the outer retention member and the inner retention member are configured to have a locked and an unlocked configuration, wherein when in the unlocked configuration the outer retention member is configured to move axially with respect to the inner retention member, and when in the locked configuration the outer retention member is prevented from moving axially with respect to the inner retention member.
24. The delivery system of clause 23, wherein the inner retention member comprises an outer threading and the outer retention member comprises an inner threading, and wherein the locked position occurs when the outer threading is threaded onto the inner threading.
25. The delivery system of clause 23, wherein the inner retention member and outer retention member comprise locking features that an operator can unlock when the operator wants to move the outer retention member axially with respect to the inner retention member.
26. A delivery system for use with a replacement heart valve, the system comprising:
   an inner shaft having a proximal end and a distal end, the inner shaft having a cut pattern to allow bending of the inner shaft, and a spine;
   an outer shaft surrounding the inner shaft and having a proximal end and a distal end, the outer shaft having a cut pattern to allow bending of the outer shaft, and a spine; and
   at least one pull wire configured to bend one or more of the inner shaft and the outer shaft;
   wherein one of the inner shaft and the outer shaft are configured to rotate with respect to one another between a flexing configuration and an unflexing configuration;
   wherein, in the flexing configuration, the cut pattern of the inner shaft is aligned with the cut pattern of the outer shaft; and
   wherein, in the unflexing configuration, the spine of the inner shaft is aligned with the cut pattern of the outer shaft.
27. The delivery system of clause 26, wherein the cut pattern of the inner shaft is the same as the cut pattern of the outer shaft.
28. The delivery system of clause 26, wherein the cut pattern of the inner shaft is different than the cut pattern of the outer shaft.
29. The delivery system of clause 26, wherein the at least one pull wire comprises a plurality of pull wires, wherein a first pull wire is configured to cause bending of the inner shaft and wherein a second pull wire is configured to cause bending of the outer shaft.
30. A method of facilitating controlled bending of bendable coaxial shafts of a delivery system, the method comprising:
   providing a delivery system comprising:
      an inner shaft having a proximal end and a distal end, the inner shaft having a cut pattern to allow bending of the inner shaft, and a spine;
      an outer shaft surrounding the inner shaft and having a proximal end and a distal end, the outer shaft having a cut pattern to allow bending of the outer shaft, and a spine; and
      at least one pull wire configured to bend one or more of the inner shaft and the outer shaft;
   rotating one of the inner shaft and the outer shaft with respect to the other between a flexing configuration and an unflexing configuration;
   wherein, in the flexing configuration, the cut pattern of the inner shaft is aligned with the cut pattern of the outer shaft to allow bending of the outer shaft upon actuation of the at least one pull wire; and
   wherein, in the unflexing configuration, the spine of the inner shaft is aligned with the cut pattern of the outer shaft to prevent bending of the outer shaft upon actuation of the at least one pull wire.
31. The method of clause 30, wherein the cut pattern of the inner shaft is the same as the cut pattern of the outer shaft.
32. The method of clause 30, wherein the cut pattern of the inner shaft is different than the cut pattern of the outer shaft.
33. The method of clause 30, wherein the at least one pull wire comprises a plurality of pull wires, wherein a first pull wire is configured to cause bending of the inner shaft and wherein a second pull wire is configured to cause bending of the outer shaft.
34. A delivery system for use with a replacement heart valve, the system comprising:
   an inner shaft having a proximal end and a distal end; and
   an outer shaft surrounding the inner shaft and having a proximal end and a distal end;
   wherein the inner shaft and the outer shaft are keyed together at the distal end of the outer shaft and the distal end of the inner shaft to prevent rotation of the inner shaft with respect to the outer shaft.
35. The delivery system of clause 34, wherein the inner shaft and the outer shaft each have an ovaloid cross-section.
36. The delivery system of clause 34, wherein at least the distal end of the inner shaft comprises a locking tab and at least the distal end of the outer shaft comprises a notch or slot configured to receive the locking tab so as to prevent rotation of the inner shaft with respect to the outer shaft.
37. The delivery system of clause 34, wherein at least the distal end of the outer shaft comprises a locking tab and at least the distal end of the inner shaft comprises a notch or slot configured to receive the locking tab so as to prevent rotation of the inner shaft with respect to the outer shaft.
38. The delivery system of clause 34, wherein the outer shaft comprises an outer pull wire lumen and wherein the inner shaft comprises an inner pull wire lumen.
39. A delivery system for a replacement heart valve, the delivery system comprising:
   an inner shaft having a proximal end and a distal end;
   a mid shaft surrounding the inner shaft and having a proximal end and a distal end and a lumen, the mid shaft having a disc on the distal end, the diameter of the disc being greater than a diameter of the inner shaft, wherein the disc comprises a longitudinally extending aperture radially outward of the lumen; and
   an outer shaft surrounding the mid shaft, the outer shaft comprising a radially extending aperture having a tooth; and
   an attachment tether configured to releasably connect to the replacement heart valve, wherein the attachment tether has a first end connected to the inner shaft, wherein the attachment tether extends through the lumen of the mid shaft and out the distal end of the mid shaft, and wherein the attachment tether extends proximally through the longitudinally extending aperture and attaches to the tooth of the outer shaft;
   wherein, when the disc is proximally translated it prevents release of the attachment tether from the tooth; and
   wherein, when the disc is distally translated it releases the tether from the tooth so that the replacement heart valve is released.
40. A delivery system for a replacement heart valve, the delivery system comprising:
   a capsule configured to surround the replacement heart valve and configured to radially compress the replacement heart valve, wherein the capsule comprises:
   a distal portion, wherein the distal portion comprises a hypotube with an interrupted spiral cut pattern, an outer polymer jacket configured to at least partially cover a radially outwards surface of the hypotube, a fluoropolymer liner on a radially inner surface of the hypotube, and a porous fluoropolymer outer coating on one of the hypotube or the outer polymer jacket; and
   a proximal portion having a smaller diameter than the distal portion, wherein the proximal portion comprises a hypotube with a cut pattern, the hypotube surrounded by an outer polymer jacket.
41. The delivery system of clause 40, wherein the outer polymer jacket of the distal portion covers greater than 90% of a length of the hypotube of the distal portion.
42. The delivery system of clause 40, wherein the hypotube of the distal portion is configured to provide compression resistance and the liner and/or outer polymer jacket of the distal portion is configured to provide tension resistance.
43. The delivery system of clause 40, wherein the liner of the distal portion is porous.
44. The delivery system of clause 43, wherein the liner of the distal portion is bonded to a metal structure of the hypotube of the distal portion using a reflow process.
45. A handle for a replacement heart valve delivery system, the handle comprising:
   a housing comprising a threaded portion;
   at least one adapter; and
   at least one knob located on the housing, the at least one knob comprising:
      a first portion configured to be coupled to the at least one adapter, and
      a second portion configured to be detachably coupled to the first portion, wherein the first and second portions are configured to detach when a threshold force is exerted on the at least one adapter.
46. The handle of clause 45, wherein the first portion comprises a recess and an internal spring within the recess.
47. The handle of clause 46, wherein the second portion comprises a projection configured to be received by the recess of the first portion.
48. The handle of clause 47, wherein the internal spring of the first portion releasably retains the projection of the second portion when the first and second portions are coupled.
49. The handle of clause 45, wherein the first portion comprises a hole and the second portion comprises a spring tab, the hole of the first portion being configured to receive the spring tab of the second portion when the first and second portions are coupled.
50. The handle of clause 49, wherein the spring tab of the second portion is configured to deflect from the hole of the first portion when the threshold force is exerted on the at least one adapter to decouple the first and second portions.
51. The handle of clause 45, wherein the first portion comprises a ramped recess and the second portion comprises a ramped projection, the ramped recess of the first portion being configured to receive the ramped projection of the second portion when the first and second portions are coupled.
52. The handle of clause 51, wherein the ramped projection of the second portion is configured to deflect from the ramped recess of the first portion when the threshold force is exerted on the at least one adapter to decouple the first and second portions.
53. The handle of clause 45, wherein the first portion comprises a recess and the second portion comprises a pin, the recess of the first portion being configured to receive the pin of the second portion when the first and second portions are coupled.
54. The handle of clause 53, wherein the pin of the second portion is configured to break from the second portion when the threshold force is exerted on the at least one adapter to decouple the first and second portions.
55. The handle of clause 45, wherein the first portion comprises a recess and the second portion comprises a spring plunger, the recess of the first portion being configured to receive a portion of the spring plunger of the second portion when the first and second portions are coupled.
56. The handle of clause 55, wherein the portion of the spring plunger of the second portion is configured to decouple from the ramped recess of the first portion when the threshold force is exerted on the at least one adapter to decouple the first and second portions.
57. The handle of clause 45, wherein the second portion is configured to engage with the threaded portion.
58. A handle for a replacement heart valve delivery system, the handle comprising:
   a housing comprising a threaded portion;
   at least one adapter; and
   at least one knob located on the housing, the at least one knob comprising:
      a first portion configured to be coupled to the at least one adapter, and
      a second portion coupled to the first portion, wherein a distance between the first portion and the second portion is correlated with a force exerted on the at least one adapter.
59. The handle of clause 58, wherein the at least one knob further comprises a connector configured to extend between and couple the first and second portions, the connector comprising a distal portion and a proximal portion.
60. The handle of clause 59, wherein the first portion is configured to receive the distal portion of the connector and the second portion is configured to receive the proximal portion of the connector.
61. The handle of clause 59, wherein the connector comprises one or more indicators.
62. The handle of clause 61, wherein the one or more indicators indicate the force exerted on the adapter.
63. The handle of clause 58, wherein the distance between the first and second portions increases as the force exerted on the at least one adapter increases.
64. The handle of clause 58, wherein the distance between the first and second portions decreases as the force exerted on the at least one adapter decreases.
65. The handle of clause 58, wherein the first and second portions are configured to detach when the force exerted on the adapter reaches a threshold force.
66. The handle of clause 58, wherein the second portion is configured to engage with the threaded portion.

## Claims

1. A delivery system (10) for a replacement heart valve (70), the delivery system comprising:
a capsule (3702) configured to surround the replacement heart valve and configured to radially compress the replacement heart valve, wherein the capsule comprises:
a distal portion (3704), wherein the distal portion comprises a hypotube (3802) with an interrupted spiral cut pattern, an outer polymer jacket (3804) configured to at least partially cover a radially outwards surface of the hypotube, a fluoropolymer liner on a radially inner surface of the hypotube, and a porous fluoropolymer outer coating on one of the hypotube or the outer polymer jacket; and
a proximal portion (3706) having a smaller diameter than the distal portion, wherein the proximal portion comprises a hypotube (3902) with a cut pattern, the hypotube surrounded by an outer polymer jacket (3904).

2. The delivery system of claim 1, wherein the outer polymer jacket (3804) of the distal portion (3704) covers greater than 90% of a length of the hypotube (3802) of the distal portion (3704).

3. The delivery system of claim 1 or claim 2, wherein the hypotube (3802) of the distal portion (3704) is configured to provide compression resistance and the liner and/or outer polymer jacket (3804) of the distal portion (3704) is configured to provide tension resistance.

4. The delivery system of any preceding claim, wherein the liner of the distal portion (3704) is porous.

5. The delivery system of any preceding claim, wherein the liner of the distal portion (3704) is bonded to a metal structure of the hypotube (3802) of the distal portion (3704) using a reflow process.

6. The delivery system of claim 5, wherein a polymer bonds the liner to the metal structure of the hypotube (3802) of the distal portion (3704).

7. The delivery system of claim 6, wherein the polymer comprises a low durometer thermoplastic elastomer.

8. The delivery system of claim 6, wherein the polymer comprises a thermoplastic polyurethane.

9. The delivery system of any preceding claim, wherein the outer polymer jacket (3804) of the distal portion (3704) comprises thermoplastics.
